# EUROPÄISCHE PATENTSCHRIFT

(11) **EP 1 524 940 B1**
(45) Veröffentlichungstag und Bekanntmachung des Hinweises auf die Patenterteilung: **24.08.2011**
(21) Anmeldenummer: 03711931.0
(22) Anmeldetag: 05.03.2003
(51) Int. Cl.: A61B 10/00

(54) **BIOPSIEVORRICHTUNG SOWIE EIN IN DIE BIOPSIEVORRICHTUNG EINSETZBARES BIOPSIENADELMODUL**
BIOPSY DEVICE AND BIOPSY NEEDLE MODULE THAT CAN BE INSERTED INTO THE BIOPSY DEVICE
DISPOSITIF DE BIOPSIE ET MODULE D'AIGUILLE BIOPSIQUE INSERABLE DANS LE DISPOSITIF DE BIOPSIE

(30) Priorität: 19.03.2002 DE 10212154; 02.08.2002 DE 10235480; 17.10.2002 DE 10248425; 19.03.2002 DE 10212139; 19.03.2002 DE 10212156; 19.03.2002 DE 20204362 U; 19.03.2002 DE 20204361 U; 19.03.2002 DE 10212155; 19.06.2002 DE 20209530 U; 19.06.2002 DE 10227352
(43) Veröffentlichungstag der Anmeldung: 27.04.2005
(73) Patentinhaber: Bard Dublin ITC Limited, Crawley, West Sussex RH11 9BP (GB)
(72) Erfinder: HESKE, Norbert, 82299 Türkenfeld (DE); HESKE, Thomas, 82284 Grafrath (DE)
(74) Vertreter: Tomlinson, Edward James
(86) Internationale Anmeldenummer: PCT/EP2003/002285
(87) Internationale Veröffentlichungsnummer: WO 2003/077768

(56) Entgegenhaltungen:
- DE-A- 10 026 303
- US-A- 6 142 955

## Beschreibung

### Technisches Gebiet

Die Erfindung bezieht sich auf eine Biopsievorrichtung zur Gewebeentnahme in Art eines Handstückes mit zumindest einem federkraftbeaufschlagbaren Spannschlitten für eine Biopsienadeleinheit, die eine äußere Hohlnadel mit einer distalseits angeschärften Schneidklinge sowie eine im Inneren der Hohlnadel gelagerte, hohle Biopsienadel mit einem an ihrem distalen Endbereich vorgesehenen Gewebeprobeentnahmeraum aufweist, wobei die äußere Hohlnadel relativ zur hohlen Biopsienadel gleitend gelagert ist, sowie mit einer Druckquelle, die mit der hohlen Biopsienadel verbindbar ist. Ferner wird ein zum Betrieb der Biopsievorrichtung geeignetes Biopsienadelmodul beschrieben.

### Stand der Technik

Aus der DE 40 41 614 C1 ist ein Sogbiopsiegerät zu entnehmen, das als Handgerät ausgebildet ist und über eine Unterdruckquelle sowie einen Biopsiekanülenanschluß verfügt, der über einen über eine biegsame Welle verbundenen Drehantrieb in Rotation versetzbar ist. An den Biopsiekanülenanschluß ist eine als Hohlkanüle ausgebildete Biopsiekanüle ansetzbar, die vorzugsweise über eine distalseits angeschärfte, umlaufende Schneidkante verfügt, längs deren Hohlkanal ein mittels der Unterdruckquelle, die als Kolben-Zylindereinheit ausgebildet ist, Unterdruck applizierbar ist, sobald die Hohlkanüle an eine bestimmte intrakorporale Gewebestelle positioniert worden ist.

Ein ähnlich unterdruckunterstützes Biopsiegerät ist der WO 96/28097 zu entnehmen, das zwar keine in Rotation versetzbare Hohlkanüle vorsieht, doch über eine innerhalb eines Handgerätes angeordnete Spritzenkolbenanordnung zur Unterdruckerzeugung verfügt.

Die DE 100 34 297 A1 beschreibt im Unterschied zu den vorstehenden Sogbiopsieanordnungen mit nur einer einzigen Hohlnadel ein Gewebeentnahme-Endoskopieinstrument, das eine Biopsienadelanordnung aufweist, die eine an ihrem distalen Ende umlaufend angeschärfte Hohlnadel und eine innerhalb der Hohlnadel geführte hohle Biopsienadel vorsieht, wobei die innengeführte Biopsienadel an ihrem distalen Ende eine Ausnehmung zur Gewebeprobeentnahme aufweist. Proximalseitig zur hohlen Biopsienadel ist ein Ansauginstrument zur Unterdruckerzeugung vorgesehen. Eine Gewebeentnahme erfolgt derart, dass die Biopsienadelanordnung in einer gemeinsamen Stellung in einen zu untersuchenden Gewebebereich vorgeschoben wird, wobei die Biopsienadel eine distale Spitze vorsieht, die distalseits aus der Hohlnadel ein Stück emporragt, um einerseits den Eindringvorgang der Biopsienadelanordnung in das Gewebe zu begünstigen und andererseits das Eindringen von Gewebe in das Innere der Hohlnadel zu verhindern. Bei geeigneter Positionierung der Biopsienadelanordnung innerhalb des Gewebes wird die Hohlnadel ein definiertes Stück proximalwärts gezogen, wobei die innenliegende Biopsiekanüle in Ihrer Position verbleibt und die Ausnehmung freigegeben wird. Der längs der Biopsienadel angelegte Unterdruck bewirkt ein aktives Absenken bzw. Einziehen von umliegenden Gewebeteilen in die Ausnehmung. Durch kontrolliertes distalseitiges Vorschieben der Hohlnadel mit ihrem angeschärften distalen Ende über die Biopsienadel wird ein Teil des Gewebes abgetrennt und innerhalb der Ausnehmung der Biopsienadel eingeschlossen. Durch gemeinsames Zurückziehen der Biopsienadelanordnung wird sodann die abgetrennte Gewebeprobe exkorporal für Untersuchungszwecke entnommen. Der gesamte vorbeschriebene Gewebeentnahmevorgang erfolgt derart, dass die Nadelbewegungen sowie die Unterdruckapplizierung einzeln und getrennt voneinander manuell vorgenommen werden.

Demgegenüber ermöglicht die Biopsienadelanordnung, die in der WO 98/25522 beschrieben ist, eine Federkraft beaufschlagte Relativbewegung zwischen innenliegender hohlen Biopsienadel und der die Biopsienadel umgebenden äußeren Hohlnadel. Auch in diesem Fall wird die Biopsienadel zur Gewebeaufnahme distalseits zur angeschärften distalen Spitze der Hohlnadel positioniert, wobei eine Unterdruckquelle zur gezielten Unterdruckversorgung durch die hohle Biopsienadel hindurch in den Bereich ihrer Ausnehmung vorgesehen ist und den Gewebeeinbringvorgang unterstützt. Der Positioniervorgang der Biopsienadel relativ und letztlich innerhalb des zu untersuchenden Gewebebereiches erfolgt ausschließlich manuell. Eine derartige Positionierung führt insbesondere bei der Untersuchung harter Gewebebereiche nur zu unbefriedigenden Biopsieergebnissen.

Ähnliche vakuumunterstützte Gewebeentnahmevorrichtungen sind überdies der GB 2 018 601 A sowie EP 0 890 399 A1 zu entnehmen, doch sind in diesen Fällen die Unterdruckquellen sowie weitere zur gesteuerten Biopsienadelführung erforderliche Regeleinheiten als externe, an die Biopsienadelanordnung anzuschließende Zusatzeinheiten ausgebildet und vorgesehen.

Auch ist in der US 2001/0011156 A1 eine vakuumunterstützte Biopsievorrichtung beschrieben, die ein kompakt gestaltetes Handgerät vorsieht, in dessen Gehäuse alle für den Nadelantrieb der Biopsienadelanordnung notwendigen Antriebselemente vorgesehen sind. Eine Unterdruckquelle ist jedoch getrennt zum Handgerät vorgesehen, die über eine entsprechende Versorgungsleitung mit der Nadelanordnung innerhalb des Handgerätes an einer geeigneten Verbindungsstelle anschließbar ist.

Der stand der Technik nach DE-A-10026303 ist im Oberbegriff gewürdigt.

US-A-6142955 hat eine längsversdrieblich und drehbar angeordliche Aussennadel.

### Darstellung der Erfindung

Ausgehend von der als nächstkommenden Stand der Technik angesehenen Biopsienadelanordnung nach der WO 98/25522, liegt die Aufgabe zugrunde eine Biopsievorrichtung zur Gewebeentnahme, die in Art eines Handstückes ausgebildet ist und zumindest eine federkraftbeaufschlagbare Spann- und Abschussvorrichtung in Form eines Spannschlittens für eine Biopsienadeleinheit, die eine äußere Hohlnadel mit einer distalseits angeschärften Schneidklinge sowie eine im Inneren der Hohlnadel gelagerte, hohle Biopsienadel mit einem an ihrem distalen Endbereich vorgesehenen Gewebeprobeentnahmeraum aufweist, wobei die äußere Hohlnadel relativ zur hohlen Biopsienadel gleitend gelagert ist,sowie eine Druckquelle vorsieht, die mit der hohlen Biopsienadel verbindbar ist, derart weiterzubilden, dass der Bedienkomfort der Biopsievorrichtung dahingehend optimiert ist, dass eine verbesserte Untersuchung von Tumoren gewährleistet werden kann, indem die mit der Biopsievorrichtung entnehmbare Gewebeprobe eine Größe und Struktur aufweist, die einem Pathologen für eine weitere histologische Untersuchungen hervorragende Grundlagen bieten. Zudem gilt es den Gewebeentnahmevorgang an sich zu verbessern. Dies bedeutet im Einzelnen, dass die für den Gewebeabtrennvorgang erforderlichen Nadelbewegungen der Biopsienadeleinheit sowie die Druckerzeugung im Sinne eines gezielten Aufbaus eines Unterdruckes exakt aufeinander abgestimmt werden sollen. Eine weitgehend manuelle derartige Abstimmung zwischen Nadelbewegungen und Unterdruckerzeugung, wie im Falle der WO 98/25522 soll vermieden werden.

Im Lichte eines verbesserten Bedienkomfort soll die Biopsievorrichtung überdies eine möglichst kompakte Bauform aufweisen und als Handgerät bei bedarf freibeweglich die Einhandbedienung ermöglichen, so dass eine einzige Bedienperson den Gewebeentnahmevorgang einhändig durchführen kann. Im gleichen Sinne soll die Biopsievorrichtung als ein autonom arbeitendes Handinstrument ausgebildet sein, das zum Betrieb keine externen Steuer- oder Versorgungseinheiten benötigt, zu denen vom Handgerät verlaufende Verbindungsleitungen vorzusehen wären, insbesondere betrifft dies die Vermeidung einer Verbindungsleitung zu einer externen Unterdruckquelle bzw. ; Stromversorgung.

Zudem soll die Druckquelle, mit der vorzugsweise die Erzeugung eines Unterdruckes ermöglicht werden soll, möglichst einfach aufgebaut sein und zuverlässig arbeiten. Die Gewebeprobeentnahme soll möglichst derart erfolgen, dass dem Nutzer, in den meisten Fällen ist dies ein Pathologe, eine nicht verdrillte und nicht beschädigte Gewebeprobe zur Beurteilung zur Verfügung gestellt werden soll.

Die Biopsievorrichtung soll schließlich nur einen geringen Kostenaufwand erfordern und eine preisgünstige Lösung in Bezug auf auswechselbare Biopsienadeln ermöglichen, die als Verbrauchsmaterial anzusehen sind.

Die Lösung der der Erfindung zugrunde liegenden Aufgabe ist im Anspruch 1 angegeben, der eine erfindungsgemäß ausgebildete Biopsievorrichtung beschreibt. Gegenstand des nebengeordneten Patentanspruchs 33 ist ein Biopsienadelmodul, das zur funktionellen Anwendung der erfindungsgemäßen Biopsievorrichtung in diese implementierbar ist. Ferner geht aus Anspruch 50 ein Verfahren zur Gewebeentnahme mit Hilfe der erfindungsgemäßen Biopsievorrichtung hervor. Den Erfindungsgedanken vorteilhaft weiterbildende Merkmale sind Gegenstand der Unteransprüche sowie dem Beschreibungstext, insbesondere unter Bezugnahme auf die Ausführungsbeispiele zu entnehmen.

Die erfindungsgemäße Biopsievorrichtung zur Gewebeentnahme in Art eines Handstückes mit zumindest einer federkraftbeaufschlagbaren Spann- und Abschussvorrichtung in Form eines Spannschlittens für eine Biopsienadeleinheit, die eine äußere Hohlnadel mit einer distalseits angeschärften Schneidklinge sowie eine im Inneren der Hohlnadel gelagerte, hohle Biopsienadel mit einem an ihrem distalen Endbereich vorgesehenen Gewebeprobe-Entnahmeraum aufweist, wobei die äußere Hohlnadel relativ zur hohlen Biopsienadel gleitend gelagert ist,sowie mit einer Druckquelle, die mit der hohlen Biopsienadel verbindbar ist, zeichnet sich erfindungsgemäß dadurch aus, dass das Handstück ein Gehäuse aufweist, in dem wenigstens zwei Antriebseinheiten sowie die als Spannschlitten ausgebildete Spannund Abschussvorrichtung lösbar fest integriert sind. Die beiden Antriebseinheiten sowie der Spannschlitten sind derart ausgebildet und innerhalb des Gehäuses angeordnet, dass in einer geöffneten Gehäusedeckeistellung die in einem Biopsienadelträger gefasste Biopsienadeleinheit sowie eine mit der Biopsienadeleinheit verbundene Druckquelle in das Gehäuseinnere implementierbar sind und mit den darin befindlichen, vorstehend genannten Komponenten in geeigneter Weise in Wirkverbindung treten. Die Biopsienadeleinheit ist dabei mit ihrer hohlen Biopsienadel über eine Verbindungsleitung gasdicht mit der Druckquelle verbunden und stellt für sich ein einheitliches Biopsienadelmodul dar, das aus Sterilitätsgründen als Einwegartikel anzusehen ist.

Der Biopsienadelträger dient einerseits als mechanische Aufnahmestruktur für die Biopsienadeln, von denen zumindest die äußere Hohlnadel, wie im weiteren im einzelnen ausgeführt wird, über einen Spindelmechanismus durch Rotation um die Nadellängsachse längs zur hohlen Biopsienadel bewegbar ist. Andererseits werden die Biopsienadeln gemeinsam über den Biopsienadelträger mit dem Spannschlitten lösbar fest verbunden, durch den der Einschußvorgang beider Biopsienadeln gemeinsam in einen zu untersuchenden Gewebebereich vorgenommen wird. Hierzu weist der Biopsienadelträger eine geeignete Kupplungsstruktur auf, die in eine entsprechende am Spannschlitten vorgesehene Gegenkupplungsstruktur einsetzbar ist.

Der Biopsienadelträger weist in einer bevorzugten Ausführungsform ein einseitig offenes Gehäusemodul auf, über dessen offene Seite die Biopsienadeln lösbar fest in den Biopsienadelträger integrierbar sind. Zudem bietet die einseitig offene Ausgestaltung des Biopsienadelträgers die Möglichkeit für einen mechanisch Eingriff eines am Aussenumfang der äußeren Hohlnadel angebrachten Antriebsmittels in eine Getriebekomponente, die an der Abtriebswelle eines der Antriebseinheiten angebracht ist. Durch diese zwischen der äußeren Hohlnadel und der Antriebseinheit bestehenden kinematischen Wirkverbindung gelingt es die äußere Hohlnadel in Rotation zu versetzen, wodurch sich diese relativ zur Nadellängsachse der hohlen Biopsienadel, die in Nadellängsrichtung zum Biopsienadelträger feststehend angeordnet ist, verschiebt. Eben über denselben kinematischen Wirkmechanismus wird zugleich auch der Spannvorgang des Spannschlittens bewerkstelligt, indem bei Erreichen eines mechanischen Anschlages der gegenüber der hohlen Biopsienadel längsbeweglichen äußeren Hohlnadel der Biopsienadelträger samt der Biopsienadelanordnung in Spannrichtung zusammen mit dem Spannschlitten bis zum Erreichen der Spannstellung verschoben wird.

Somit ist es möglich mit Hilfe nur eines einzigen Antriebsmittels zwei Funktionen zu bedienen, nämlich das Spannen des Spannschlittens und zugleich die Bewegungsansteuerung der Biopsienadeln, die sich nicht nur auf eine relative Längsverschieblichkeit beider Biopsienadeln beschränkt, sondern, wie die weiteren Ausführungen zeigen werden, optional auch zusätzliche rotatorische Bewegungsabläufe um die Nadellängsachse mit umfassen.

Neben der vorstehend erwähnten Antriebseinheit dient die zweite Antriebseinheit ausschließlich der gezielten Erzeugung eines Druckniveaus innerhalb der hohlen Biopsienadel und dem damit verbundenen Gewebeprobenentnahmeraum. Das Druckniveau stellt in Abhängigkeit des jeweiligen mit der Biopsievorrichtung durchgeführten Arbeitsschrittes einen Über- oder Unterdruck dar, der mit der Druckquelle gezielt erzeugbar und einstellbar ist.

Mit Hilfe der erfindungsgemäß ausgebildeten Biopsievorrichtung ist eine vollständig autonom durchführbare Gewebeprobeentnahme möglich, die überdies von einem Arzt im Rahmen einer Einhandbedienung durchgeführt werden kann. Alle für die Gewebeprobeentnahme erforderlichen Vorgänge erfolgen automatisch,d.h ohne zusätzliche manuelle Unterstützung und können jeweils über einzelne Tastenbetätigungen an der Biopsievorrichtung selbst ausgelöst werden.

Die einzelnen für eine vollständige Gewebeentnahme erforderlichen Schritte werden von der Biopsievorrichtung in der folgenden Weise realisiert:
- Überführen der Biopsienadeleinheit sowie des Spannschlittens in eine Ausgangsstellung. Dieser erste Schritt entspricht einer Art Resetfunktion.
- Überführen des Spannschlittens in einen gespannten Zustand,
- Schussauslösung, mit der die Biopsienadeleinheit distalwärts in einen zu untersuchenden Geweberaum geschossen wird,
- Automatischer Aufbau eines Unterdruckes, der von der Druckquelle über die Verbindungsleitung längs der hohlen Biopsienadel in den Gewebeentnahmeraum applizierbar ist,
- Gewebeabtrennvorgang, bei dem die äußere Hohlnadel proximalwärts verschoben und zugleich der Gewebeentnahmeraum bei Unterdruckbedingungen freigegeben wird, wodurch umliegendes Gewebematerial in den Gewebeentnahmeraum gesaugt und vom übrigen Gewebe durch die Schneidwirkung längs der den Gewebeentnahmeraum seitlich begrenzenden als Schneiden ausgebildete Längskanten abgetrennt wird, wobei der Abtrennvorgang zusätzlich durch einen periodischen distalund proximalseits gerichteten Bewegungswechsel der hohlen Biopsiekanüle unterstützt wird, so dass letztlich die teilweise abgetrennte und in den Gewebeprobeentnahmeraum eingesaugte Gewebeprobe durch distalseitiges Vorschieben der äußeren Hohlnadel vollkommen abgetrennt wird, und
- Gewebeprobeentnahmevorgang, der extrakorporal erfolgt, bei dem die äußere Hohlnadel proximalseits zumindest teilweise den Gewebeprobeentnahmeraum freigibt und durch Applizieren eines Überdruckes über die hohle Biopsienadel insbesondere in den unteren Bereich des Gewebeprobeentnahmeraumes ein Ablösen der Gewebeprobe bewirkt wird, wodurch die Gewebeprobe leicht aus dem Gewebeprobeentnahmeraum entnommen werden kann.

Die vorstehend beschriebenen Arbeitsschritte für eine sorgfältige

Gewebeprobeentnahme ist mit Hilfe der erfindungsgemäßen Biopsievorrichtung zuverlässig und sicher durchführbar. Von besondere Bedeutung ist die völlige Unabhängigkeit der Biospievorrichtung von den Gewebeentnahmevorgang unterstützenden exterenen Geräten bei gleichzeitigem hohem Bedienkomfort, der eine Einhandbedienung probelmlos ermöglicht. Die Biopsievorrichtung wird unter Bezugnahme auf die im weiteren dargestellten Ausführungsbeispiele im einzelnen erläutert.

Besonders vorteilhaft zeichnet sich die Biopsievorrichtung durch das von einem zu behandelnden Arzt zu betätigende Bedienpanel aus, das an einer Seitenaussenwand des Gehäuses der Biopsievorrichtung vorgesehen ist und vorzugsweise jeweils nur drei Bedientastenfelder aufweist, die besonders übersichtlich angebracht und vollkommen fehlerfrei bedienbar sind. So sind bspw. jedem Bedientasenfeld Lichtsignalfelder zugeordnet, die den Arzt über die aktuelle Bedienbarkeit der einzelnen Bedientastenfelder informieren und darüberhinaus einen fest vorgegeben Funktionsablauf gemäß dem vorstehend beschriebenen Verfahrensablauf gewährleisten. Besonders mit Bedacht durchzuführende Funktionen, wie das Spannen des Spannschlittens oder die Funktion des Gewebeprobenentnahmevorgangs sind mit einem Zeitdelay ausgestattet, so dass deren Betätigung nicht aus Versehen erfolgen können. Durch diese und eine große Vielzahl weiterer Besonderheiten zeichnet sich die Biopsievorrichtung vorteilhaft aus, wie im weiteren unter Bezugnahme auf die nachstehenden Ausführungsbeispiele entnommen werden kann.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Biopsievorichtung mit geöffnetem Gehäusedeckel (perspektivisch)
- Fig. 2: Handstück mit den darin angeordneten Teilen der Biopsievorrichtung (ohne Gehäuseboden und Deckel) und austauschbare Biopsieeinheit; (perspektivisch abgehoben)
- Fig. 3: Längsschnitt A - A durch die Biopsienadel in Fig.1
- Fig. 3a: Längsschnitt A - A durch die Biopsienadel in Fig. 1 (wie Fig. 3) proximaler Teil (vergrößert)
- Fig. 3b: Vergrößerung des Ausschnitts A in Fig. 3a
- Fig. 3c: Vergrößerung des Ausschnitts B in Fig. 3a
- Fig. 4: Querschnitt A - A in Fig. 3 (linker Gehäuseteil)
- Fig. 5: Querschnitt B - B in Fig. 3 (rechter Gehäuseteil)
- Fig. 6: rechter Gehäuseenddeckel (Innenseite) mit integriertem Mikroschalter
- Fig. 7: Vorderseite des Bedienungspanels
- Fig. 8a: Basisblock in X-Achse gesehen von vorne (perspektivisch)
- Fig. 8b: Basisblock in X-Achse gesehen von hinten (perspektivisch)
- Fig. 9a: Handstück mit den gehäusefesten Einheiten der Biopsievorrichtung ohne Gehäusedeckel und Boden im ungespannten Zustand
- Fig. 9b: Verrasteinrichtung im ungespannten Zustand (Schnitt A - A)
- Fig. 10a: Wie Fig. 9, jedoch Spannschlitten in gespannter Position
- Fig. 10b: Wie Fig. 9a, jedoch in gespannter Position und in verriegeltem Zustand
- Fig. 11a: Biopsienadelspitze Seitenansicht
- Fig. 11b: Längsschnitt durch Fig. 11a (Probeentnahmeraum geöffnet)
- Fig. 11c: Wie Fig. 11b, jedoch (Probeentnahmeraum halb geöffnet)
- Fig. 11d: Wie Fig. 11b (Probeentnahmeraum mittels Schneidhülse verschlossen)
- Fig. 11e: Schnitt A - A in Fig. 11a
- Fig. 11f: Schnitt B-B in Fig. 11a
- Fig. 11g: Vergrößerung der Schnittkante bei A
- Fig. 12: Biopsienadelträger mit eingepresster Biopsienadel/Schneidhülse und Kunststoffteil (von unten, um ca. 90° gedreht, perspektivisch)
- Fig. 12a: Schnitt durch die Längsachse des proximalen Teils der Biopsienadel (vergrößert)
- Fig. 12b: Schnitt B - B durch den Vielkant der Biopsieträger im Verdrehzustand linksseitiger Anschlag
- Fig. 12c: Schnitt B - B wie Fig. 12b, jedoch Mittelstellung des Vielkants
- Fig. 12d: Schnitt B - B wie Fig. 12b, jedoch verschwenkt, rechtsseitiger Anschlag
- Fig. 12e: Schnitt A-A durch Nadelverformzone 0 der Biopsienadel und der Schneidhülse
- Fig. 12f: Biopsienadel distalseitig mit Probeentnahmeraum und Schneidhülse Grundstellung, entsprechend der Stellung des Vielkants in Fig. 12c
- Fig. 12g: Biopsienadel wie Fig. 12f mit Verschwenkung des Probeentnahmeraums nach rechts und vorgefahrener Schneidhülse, entsprechend der Stellung des Vielkants nach Fig. 12d
- Fig. 12h: Biopsienadel wie Fig. 12f mit Verschwenkung des Probeentnahmeraums und zurückgefahrener Schneidhülse entsprechend der Verdrehung des Vielkants nach Fig. 12b
- Fig. 13: Vakuum-/Druckvorrichtung, Einbau und Antrieb (von hinten gesehen, perspektivisch)
- Fig. 14a: Vakuum-/Druckvorrichtung mit auf den Spritzenboden aufgesetzten Kolben (Ausgangsstellung für Vakuumerzeugung und Endstellung für Druckerzeugung, teilweise geschnitten)
- Fig. 14b: Vakuum-/Druckvorrichtung mit zurückgezogenem Kolben; Endstellung des Vakuumhubs (teilweise geschnitten)
- Fig. 14c: Stellung des Kolbens nach Freigabe der Belüftungsbohrung; Druckausgleichsstellung; teilweise geschnitten)
- Fig. 14d: Schnitt A - A durch die Gewindespindel in Fig. 14c
- Fig. 15: Basisblock und Biopsienadel/Schneidhülse, vorbereitet für die Bestückung mit Fotozellen und Mikroschalter für die Ist Wert-Erfassung

### Wege zur Ausführung der Erfindung, gewerbliche Verwendbarkeit

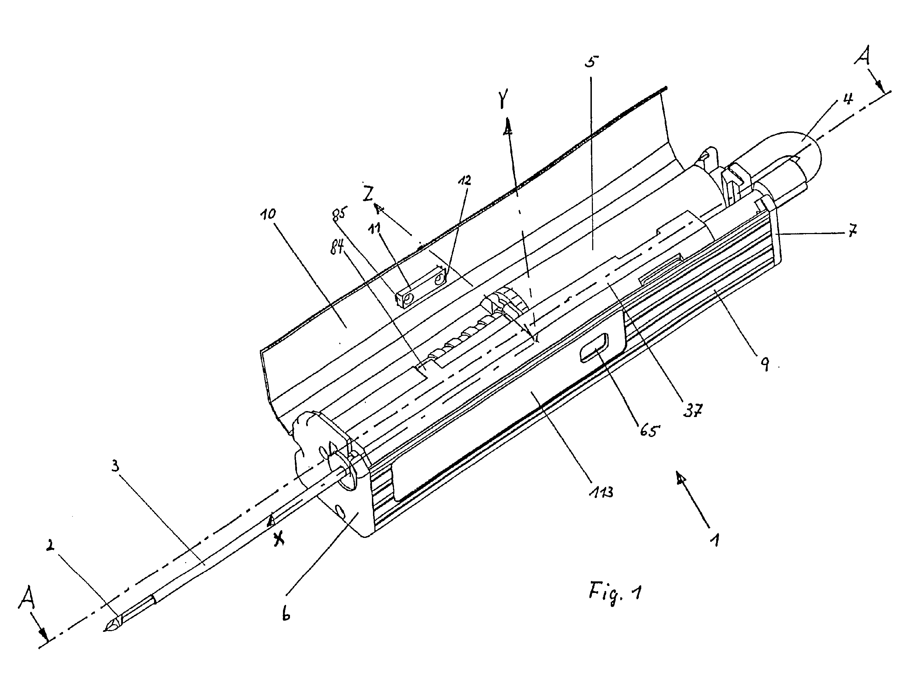

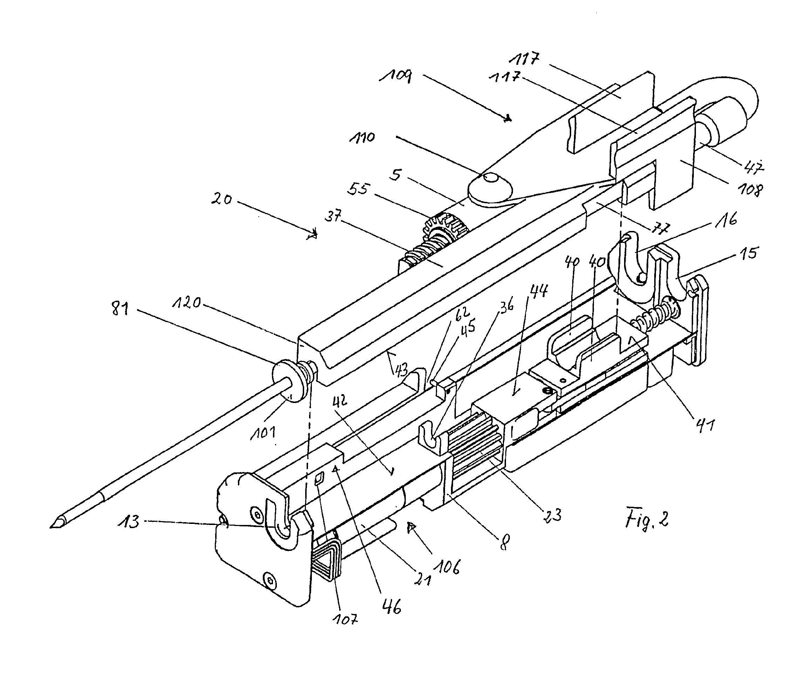

Im Ausführungsbeispiel gemäß Fig. 1 sind alle für die Durchführung einer Vakuumbiopsie erforderlichen Komponenten im Gehäuseinnenraum eines Handstückes 1 integriert, so dass keine Kabel oder Leitungen vom Gehäuse des Handstücks zu weiteren externen Versorgungsvorrichtungen erforderlich sind. Das Handstück 1 stellt somit eine komplette Vakuumbiopsievorrichtung dar, die nach allen Richtungen frei beweglich ist. Aus dem distalen Gehäuseenddeckel 6 ragt der distale Teil der hohlen Biopsienadel 2 mit der sie koaxial umgebenden äußeren Hohlnadel 3 heraus, die im weiteren als Schneidhülse bezeichnet wird; sie dient zur Entnahme bzw. zur vollständigen Abtrennung der Gewebeprobe. Meist wird zu Beginn der Biopsie eine nicht dargestellte Koaxialkanüle in das Gewebe gesetzt, in die dieser Teil der Biopsienadel 2 mit Schneidhülse 3 eingebracht wird und so vor dem zu untersuchenden Gewebe positioniert wird. Außerhalb des rechten proximalen Gehäuseenddeckels 7 ist ein Verbindungselement 4 angeordnet, z.B. ein durchsichtiger, flexibler Schlauch, der die parallel zur Biopsienadel angeordnete Druckquelle oder Vakuum-/Druckerzeugungsvorrichtung 5 mit dem Innenhohlraum der Biopsienadel 2 gasdicht verbindet. Das hohle Verbindungselement 4 liegt in unmittelbarer Nähe des Gehäuseenddeckels 7. Die in einem Biopsienadelträger 37 angeordnete Biopsienadel 2 mit Schneidhülse 3 und weiteren Elementen bildet mit dem Verbindungselement 4 und der Vakuum-/Druckerzeugungsvorrichtung 5 ein leicht nach oben herausnehmbares sowie einlegbares Biopsienadelmodul 20, im weiteren als herausnehmbares Element bezeichnet, das nach Bedarf gewechselt wird (Fig. 2). Hierzu wird der Gehäusedeckel 10 geöffnet. Wie insbesondere Fig. 2 zeigt, lässt sich die Biopsievorrichtung in Teile, die fest mit dem Gehäuse verbunden sind (desinfizierte Teile), und in ein herausnehmbares Element 20 (steriler Teil)) gliedern. Während die mit dem Gehäuse fest verbundenen Teile lediglich desinfiziert werden, wird das herausnehmbare Element 20 steril verpackt angeliefert und je nach Bedarf , vor allem bei jedem neuen Patienten, erneuert. Wie später noch im Einzelnen ausgeführt wird, ist darauf geachtet, dass der desinfizierte Teil bei Gebrauch nicht mit Gewebeflüssigkeit verunreinigt wird.

Im nachfolgend beschriebenen Ausführungsbeispiel ist die Vakuum-/Druckerzeugungsvorrichtung (5) parallel zur Biopsienadeleinheit angeordnet. Im Rahmen der Erfindung kann jedoch die Vakuum-/Druckerzeugungsvorrichtung auch in der Achse der Biopsienadel oder des Handstücks liegend angeordnet sein; auch bedarf es keines eigenen Verbindungselementes, wenn sie z.B. unmittelbar auf das Ende der Biopsienadel aufgesetzt ist, in diesem Fall ist das Verbindungselement als eine geeignte Fanschverbindung, bspw. in Form eines Luer-Lock-Verschlusses, anzusehen.

Zwischen dem linken und dem rechten Gehäuseenddeckel 6, 7 befindet sich ein Gehäuseunterteil 9 und ein in den Gehäuseenddeckeln schwenkbar gelagerter Gehäusedeckel 10 mit einem Verschlussriegel 11. Über Zuganker oder Schrauben, die teilweise in einen Basisblock 8 eingeschraubt sind, wird der Gehäuseunterteil 9 zwischen die Gehäuseenddeckel 6, 7 eingeklemmt, bzw. mit dem Basisblock 8 verbunden. Der Gehäusedeckel 10 ist über eine in den Gehäuseenddeckeln 6, 7 befestigte Achse schwenkbar verbunden. Der Gehäusedeckel 10 wird vor dem Betrieb der Biopsieeinrichtung geschlossen; die Innenkontur des Gehäusedeckels entspricht der Außenkontur des später genauer beschriebenen Biopsienadelträgers 37. Etwa im Zentrum des Gehäuseinnenraumes, ist der Basisblock 8 angeordnet, der mit dem Gehäuseunterteil z.B. über Fixierelemente und/oder über eine Schraubverbindung fest verbunden ist. Mit dem Basisblock 8, sind die Antriebselemente für die Vakuum-/Druckerzeugungsvorrichtung 5, die Schneidhülse 3 und die Spanneinrichtung für den Spannschlitten 28, auf dem der Biopsienadelträger 37 aufgesetzt ist, verbunden. Der Basisblock 8 erstreckt sich von der Mitte des Gehäuses nach links und eine an ihn angefügte Platte deckt die Antriebe ab und dient zur Lagerung der Steuerplatine, die innerhalb bzw. unterhalb der Abdeckung 46 geschützt angeordnet ist. Weiterhin weist der Basisblock 8 je eine nach oben offene Halterung 36 für die Biopsienadel/Schneidhülse und ein weiteres Einlegeelement 62 für die Vakuum-/Druckerzeugungsvorrichtung auf.

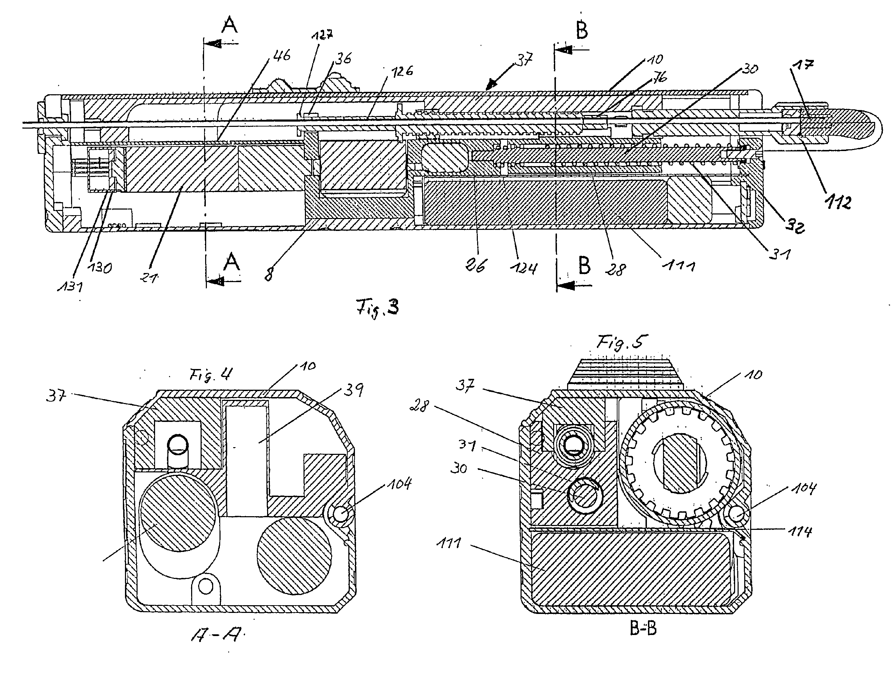

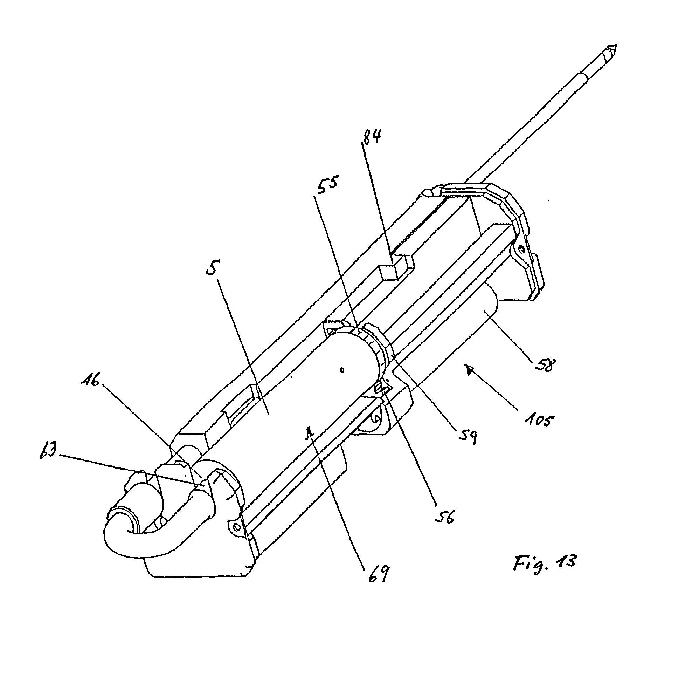

Zur Beschreibung der Lage der einzelnen Elemente, sowie der Lage der Einzelteile, insbesondere im Gehäuseinnenraum, wurde in Fig. 1 ein Koordinatenkreuz eingezeichnet, wobei der Koordinatenmittelpunkt des Koordinatensystems im Zentrum des Basisblocks 8 (Fig. 1) liegt. Danach gelten für die nachfolgende Beschreibung für die Richtungsangaben in Richtung der X-Achse gesehen als links (distal) und entgegengesetzt der X-Achse gesehen als rechts (proximal). Für die übrigen Koordinaten gilt in Richtung der Y-Achse als oben, entgegensetzt der Y-Achse als unten und in Richtung der Z-Achse als hinten und entgegengesetzt der Z-Achse als vorne (Fig. 1). Das Koordinatensystem teilt den Geäuseinnenraum und die übrigen Bezüge also in links und rechts, in vorn und hinten sowie oben und unten auf. Bei der Winkeldrehbewegung der Biopsienadel wurde abweichend von dieser Festlegung die Drehung um die gemeinsame Längsachse von Biopsienadel und Schneidhülse nach links (also nach vorne) und rechts (nach hinten) zum leichteren Verständnis gewählt. Unter Bezug auf diese Festlegungen befinden sich etwa im unteren, vorderen, linken Gehäuseteil des Gehäuseinnenraumes die gemeinsamen Antriebseinrichtungen 106 für die Spanneinrichtung und die Schneidhülse sowie im unteren, hinteren, linken Gehäuseteil die Antriebseinrichtung 105 (Fig. 13) für die Vakuum-/Druckerzeugungsvorrichtung 5. Im unteren, rechten Teil ist die Energieversorgung für die Antriebsmotoren und die sonstige Elektrik untergebracht, wie z.B. für die Steuer-und/oder Überwachungselemente; vorzugsweise werden hierfür Batterien oder ein Akku 111, z.B. eine 7,2V-Lithium-lonen-Batterie, 1 Ah verwendet. Der vordere, rechte, obere, über dem Batterieraum liegende Gehäuseinnenraum wird weitgehend für den Spannschlitten 28 mit Verriegelungsteil genutzt (Fig. 5); der mit einem Block 26, der Teil des Basisblocks 8 ist, verbunden ist. Der Batterieraum ist nach oben durch eine Trennplatte 114 abgedichtet.

Im obersten, vorderen Teil des Gehäuseinnenraums ist ein in die U-förmige, nach oben offene Einlegehalterung 36 des Basisblocks 8 und in die nach oben zeigenden, beidseitig des Spannschlittens 28 angeordnete Lasche 40 einlegbarer und herausnehmbarer Biopsienadelträger 37, in dem die Biopsienadel/Schneidhülseneinheit mit Antriebsteilen drehbar gelagert ist, der über nahezu die gesamte Länge des Handstücks reicht, angeordnet. Der Biopsienadelträger ist, wie später beschrieben, mittels des Spannschlittens längsverschieblich. Dies bedeutet, in nicht gespanntem Zustand liegt die linke Stirnfläche des Biopsienadelträgers 37 nahezu am linken Gehäuseenddeckel 6 an, in gespanntem Zustand die rechte Stirnfläche am rechten Gehäuseenddeckel 7. "Nahezu der gesamten Länge" bedeutet, dass der Biopsienadelträger mindestens um den Betrag kürzer ist, als der Gehäuseinnenraum für den Spannvorgang benötigt wird. Beträgt der Spannweg des Spannschlittens z.B. 20 mm so muss sich der Biopsienadelträger mindestens um diesen Betrag verschieben lassen. Im Allgemeinen liegt der Spannweg zwischen 15 und 25 mm, je nach verwendeter Biopsienadel. Daher ist es zweckmässig, den Innenraum auf den jeweils größtmöglichen Spannweg plus einige mm auszulegen.

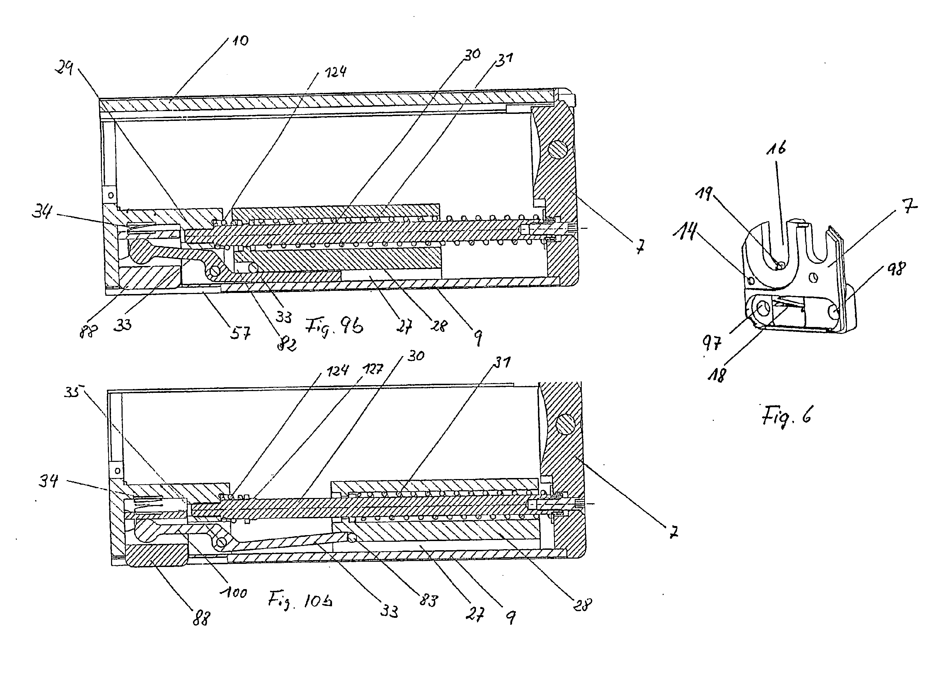

Die Spannvorrichtung (rechts, vorn liegend) selbst, besteht aus einem auf einem Bolzen 30 geführten Spannschlitten 28, wobei der Bolzen in den Block 26 des Basisblocks 8 eingeschraubt ist. Der Bolzen 30 wird proximalseitig von einer Spiralfeder 31 umgeben. Auf der distalen Seite des Spannschlittens ist eine weitere kurze Spiralfeder 124 auf dem Bolzen 30 angeordnet. Diese kurze Spiralfeder stützt sich einerseits am Block 26 und andererseits an einer Innenlippe 122 distalseitig im Spannschlitten ab. Auf der gegenüber liegenden Seite (proximalseitig) der Lippe des Spannschlittens stützt sich die Spiralfeder 31 ab. Die Verrastvorrichtung (sh. insbesondere Fig. 9b und 10b) des Spannschlittens ist an dem Block 26 befestigt. Im oberen, hinteren, rechten Gehäuseinnenraum ist die Vakuum-/Druckerzeugungsvorrichtung 5 mit Teilen des Antriebs untergebracht; der Antriebsmotor mit Reduktionsgetriebe für die Vakuum-/Druckerzeugungsvorrichtung befindet sich im linken, unteren, hinteren Bereich des Gehäuseinnenraumes.

Der Gehäusedeckel, das Gehäuseunterteil, die Gehäuseenddeckel sowie der Basisblock bestehen vorzugsweise aus Aluminium.

Das Handstück 1 besteht, wie bereits beschrieben, aus einem Gehäuse, das aus einem Gehäuseunterteil 9 mit seitlich unterschiedlich hochgezogenen Wänden, dem Gehäuseunterteil angepassten Gehäusedeckel 10 mit längsverschieblichem Verschlussriegel 11 und den beiden Gehäuseenddeckeln 6, und 7 gebildet wird. Das Gehäuseunterteil ist mit den beiden Gehäuseenddeckeln über Zuganker oder Schrauben, z.B. aus Eisen, verbunden, die teilweise unmittelbar in den Basisblock 8 eingeschraubt werden. Das Gehäuse ist ca. 200 mm lang, die Gehäuseenddeckel haben etwa quadratischen Querschnitt, ca. 40 x 40 mm, (Fig. 2). Der Gehäusedeckel 10 ist um eine Achse 104 verschwenkbar, die in den Gehäuseenddeckeln 6, 7 befestigt ist; hierzu dienen in den Gehäuseenddeckeln die Bohrungen 14. Die Nase 12 des Verschlussriegels 11 ist in die Ausnehmung 45 des Basisblocks 8 zum Verschließen des Gehäusedeckels einschiebbar. Der linke Gehäuseenddeckel 6 weist im oberen, vorderen Teil eine nach oben offene U-förmige Durchführung 13 für den nach vorne herausragenden Teil der Biopsienadel/Schneidhülse 2, 3 und der darauf angeordneten Führungsrolle 81 auf. Die Führungsrolle 81 die bei Verwendung einer-Koaxialkanüie auf diese aufgesetzt wird, verhindert auch, dass Gewebeflüssigkeit in das Gehäuse eindringen kann. Der hintere Gehäuseenddeckel 7 weist zwei nach oben offene, U-förmige Durchführungen 15, 16 auf. Die Durchführung 15 korrespondiert mit der Durchführung 13; sie nimmt das Ende des auf die hohle Biopsienadel aufgesetzten querschnittsrunden Kunststoffteils 47 auf. In die Durchführung 16 wird ein Stutzen 63 der Vakuum/Druckerzeugungsvorrichtung eingelegt (Fig. 2). Ein in das Kunststoffteil 47 eingesetztes, weiteres Kunststoffteil 112 weist einen Zapfen 17 auf, der zur Verbindung des Verbindungselements 4 mit dem Ausflussstutzen 64 der Vakuum-/Druckerzeugungsvorrichtung dient. Der Innenhohlraum der Biopsienadel ist über das ebenfalls hohle Verbindungselement 4 durchgängig mit dem Hohlraum der Kolben-/Zylinderanordnung und dem Hohlraum der Vakuum-/Druckerzeugungsvorrichtung verbunden. Die Verbindungen sind derart gestaltet, dass in das System weder Luft von außen eindringen kann, noch bei Überdruck Luft nach außen ausströmen kann; die Verbindungsstellen sind also luftdicht ausgebildet. Das so ausgebildete System bewirkt, dass das Dichtelement 76 bei Anlegen eines Unterdrucks im Inneren der Biopsienadel gegen die Biopsienadel 2 gezogen wird, was die Dichtwirkung zwar verstärkt, jedoch die Drehbewegung der Schneidhülse gegenüber der Biopsienadel nicht negativ beeinflusst, jedoch die Biopsienadel bei entsprechender Gestaltung mit verdreht, bis die Verdrehung durch eine Begrenzungsvorrichtung gestoppt wird. Wie insbesondere Fig. 6 zeigt, ist in die Durchführung 16 des Gehäuseenddeckels 7 auf der unteren Seite ein Mikroschalter 18 integriert, dessen Schaltstift 19 in die Durchführung hinein ragt.

Sobald der Stutzen 63 der Vakuum-/Druckerzeugungsvorrichtung in die Durchführung eingelegt wird und der Gehäusedeckel geschlossen ist, wird der Schaltstift 19 des Mikroschalters 18 nach unten gedrückt und der Mikroschalter 18 gibt die Stromzufuhr frei. In die Durchführungen 97, 98 des Gehäuseenddeckels können die Anschlüsse zum Anschluss eines Ladegerät eingebaut werden.

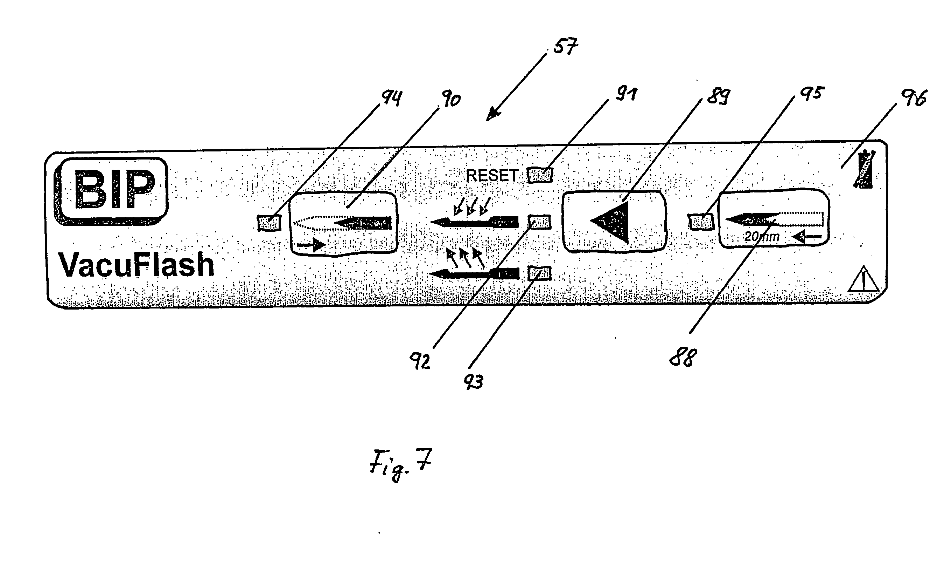

An der vorderen Seite des Gehäuseunterteils 9 ist eine Fläche 113 für das an dem Gehäuse befestigte Bedienungspanel (Fig. 7) mit den Bedien- und Überwachungselementen vorgesehen. Das am Gehäuse zu befestigende Bedienungspanel 57 ist als eigenes Bauteil ausgebildet, das beispielsweise auf die Fläche 113 des Gehäuseunterteils 9 aufgeklebt wird. Über Leitungen ist dieses Bedienungspanel 57 mit weiteren elektronischen Bauteilen, die im Gehäuse angeordnet sind, sowie mit der Stromversorgung verbunden. Von den mit dem Bedienungspanel verbundenen elektrischen/elektronischen Bauteilen ist insbesondere die in den Raum 39, der unterhalb der Abdeckung 46 liegt, angeordnete Platine zu erwähnen. Auf der Platine ist ein programmierbarer Mikroprozessor sowie weitere elektronische Bauteile angeordnet. Mittels des Mikroprozessors wird die später beschriebene halbautomatische Ablaufsteuerung gesteuert. Das Bedienungspanel beinhaltet vor allem Schalter zum Bedienen der Biopsievorrichtung und Dioden für die Kontrolle des Bedienablaufs. Aus einer Aussparung 65 im Gehäuseunterteil ragt die Betätigungstaste 88 für die mechanische Auslösung des gespannten Spannschlittens heraus und drückt das darüberliegende Bedienungspanel an dieser Stelle etwas heraus, so dass die Betätigungstaste durch die Folie des Bedienungspanels leicht erfühlbar ist.

Bei der Gestaltung der Bedien- und Überwachungselemente wurde darauf geachtet, dass zwischen dem Spannvorgang des Spannschlittens und dem Auslösen des Spannschlittens einerseits, und andererseits der Durchführung der Biopsie, wie dem Heraustrennen der Probe, sowie insbesondere der Probeentnahme durch Auswurf der Probe, unterschieden wird. Dementsprechend ist die Betätigungstaste 88 (Auslöser) für den Spannschlitten nach rechts und die das Spannen des Spannschlittens auslösende Spanntaste 90 nach links gesetzt worden. Die für das Durchführen der Biopsie vorgesehene Programmtaste 89 liegt mittig. Ebenfalls mittig liegen die Kontrollleuchten für Reset, Durchführung der Biopsie und Auswurf der Probe, nach Öffnen des Probeentnahmeraums. Durch Drücken der Programmtaste 89 werden nach Einlegen des herausnehmbaren Elements 20 und nach Schließen und Verriegeln des Gehäusedeckels sowie automatischer Einstellung der Grundstellung zwei Funktionen abgerufen, nämlich die Probeentnahme und der Probenauswurf. Nach Einlegen des herausnehmbaren Elements und Schließen des Deckels leuchtet kurzzeitig die Resetdiode 91 gelb auf und während der Einstellung der Grundstellung blinkt sie; nach der Einstellung der Ausgangsposition erlischt die Resetdiode. Die Probeentnahmediode 92 (grün) und die Spanndiode (gelb) leuchten auf und zeigen an, dass der Bediener entweder die eine oder die andere Funktion abrufen kann. Drückt er die Spanntaste 90, so wird der Spannschlitten 28 in Spannstellung gebracht und dort verriegelt. Um zu verhindern, dass die Spanntaste versehentlich gedrückt wird, ist sie mit einer Verzögerungsschaltung, ca. 1,2 Sekunden, ausgestattet. Während des Spannvorgangs blinkt die gelbe Spanndiode; nach Abschluss des Spannvorgangs leuchtet die Verriegelungsdiode (grün) auf ; das Gerät, d.h. die Biopsiendadel, ist bereit um in das zu untersuchende Gewebe durch Auslösen mittels der Betätigungstaste 88 eingeschossen zu werden. Nach dem Einschuss erlischt die Verriegelungsdiode und die Spanndiode (gelb) und die Probeentnahmediode (grün) leuchten auf. Beide Funktionen (Spannen oder Probeentnahme) können nun abgerufen werden. Bei Drücken der Programmtaste 89 wird der Biopsievorgang automatisch durchgeführt, wie später erläutert. Es könnte aber auch erneut der Spannvorgang abgerufen werden. Bei Aufruf des Biopsievorgangs (Probeentnahme) läuft dieser automatisch ab. Nach Abschluss des Vorgangs erlischt die grüne blinkende Probeentnahmediode und die gelbe Auswurfdiode leuchtet auf. Durch erneutes Drücken der Programmtaste wird der automatisierte Ablauf der Probeentnahme durchgeführt. Nach Abschluss des Vorgangs erlischt die blinkende Auswurfdiode und die gelbe Resetdiode leuchtet auf, was bedeutet, dass das herausnehmbare Element 20 entnommen werden kann, oder dass durch Drücken der Programmtaste das Gerät automatisch für die Entnahme einer weiteren Probe vorbereitet wird. Es folgt dann wieder der Vorgang wie bereits beschrieben, Spannen usw. oder Probeentnahme. Beim Drücken der Programmtaste 89 zur Probeentnahme (zum Auswurf der Probe) ist eine Verzögerungsschaltung vorgesehen, um zu verhindern, dass bei versehentlichem Berühren der Programmtaste der Auswurf erfolgt, ohne dass die Nadel vorher herausgezogen wurde.

Die Batterieladediode 96 zeigt den Ladungszustand der Batterie, bzw. der Akkus an. Die Dioden sind,wie bereits beschrieben, so geschaltet, dass bei Durchführung des jeweils aufgerufenen Vorgangs die Diode blinkt und nach Abschluss des Vorgangs die Diode des Folgevorgangs aufleuchtet. Sofern zwei Möglichkeiten zur Auswahl stehen, leuchten beide Folgedioden auf. Es ist dem Bediener dann freigestellt, welche Auswahl er trifft. Die Farben der Dioden sind so gewählt, dass Vorgänge im Gewebe grün angezeigt werden, während Vorgänge außerhalb durch die Farbe gelb angezeigt werden. Bei der Funktion Spannen und Probeentnahme sind Verzögerungsschaltungen (z.B. 1,2 - 1,5 Sekunden) vorgesehen um sicherzustellen, dass der Vorgang bewusst aufgerufen wurde. Auf die Wirkungsweise und Steuerungsmöglichkeit im Einzelnen wird bei der Beschreibung des Ablaufvorgangs näher eingegangen. Symbole (Pictogramme) auf dem Bord symbolisieren die einzelnen Vorgänge.

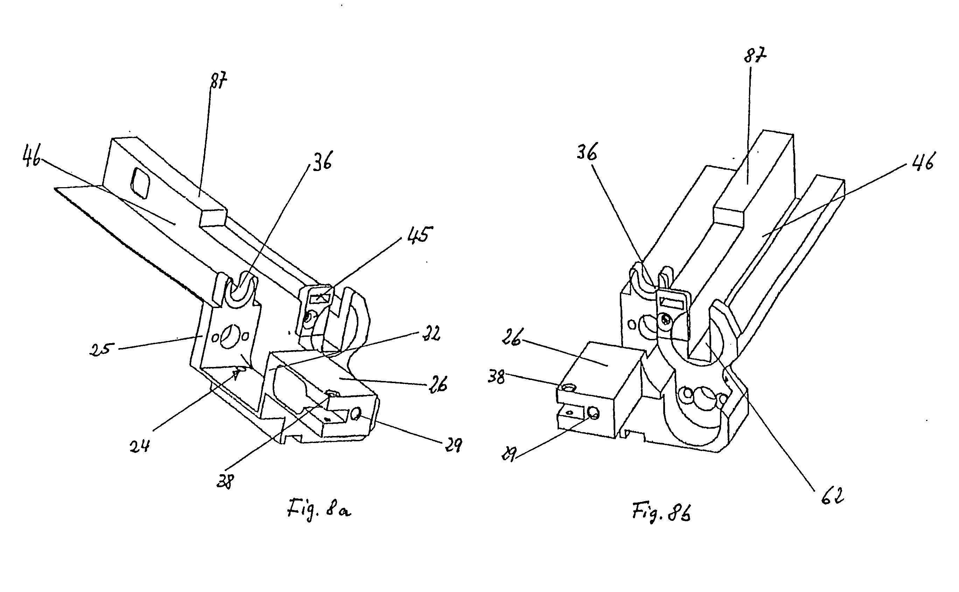

Eine perspektivische Darstellung des Basisblocks 8 (von vorne in Richtung der X-Achse gesehen) zeigt Fig. 8a; den Basisblock 8 von hinten in X-Achse zeigt Fig. 8b (beide Darstellungen perspektivisch). Der Basisblock 8 lässt sich in Längsrichtung gesehen in zwei Hälften gliedern; der vordere Teil dient der Befestigung des gemeinsamen Antriebs für Schneidhülse und Spannschlitten sowie in seinem oberen Teil der Lagerung des Biopsienadelträgers (Fig. 8a); der rückwärtige Teil dient der Befestigung des Antriebs für die Vakuum-/Druckerzeugungsvorrichtung sowie der Lagerung der distalen Seite der Vakuum-/Druckerzeugungsvorrichtung (Fig. 8b). Zwischen den beiden Antriebsmotoren 21, 58, unter der Mittelrippe 87 ist eine zentrale Elektronikplatine in dem darunter liegenden Raum 39 angeordnet. Der Basisblock 8 weist in seinem linken, vorderen Teil einen U-förmigen Raum 24 auf, in den eine Zahnwalze 23, die von dem Getriebemotor 21 angetrieben wird, eingebaut ist. Hierzu ist die Abtriebswelle des Getriebemotors in einer Öffnung in der Wand 25 des Basisblocks 8 gelagert bzw. eingesteckt. Die Zahnwalze 23 ist auf die Abtriebswelle aufgesteckt und auf ihr z.B. mittels einer Schraube dreh- und verschiebesicher befestigt. Auf der anderen Seite ist die Zahnwalze 23 in der Wand 22 des Basisblocks 8 gelagert. Als Antriebsmotor wird ein Gleichstrommotor mit einer Drehzahl von ca. 11000 U/min verwendet. Dem Gleichstrommotor ist ein Planetengetriebe mit hoher Untersetzung nachgeschaltet, auf dessen Abtriebswelle ist die Zahnwalze 23 aufgesetzt.

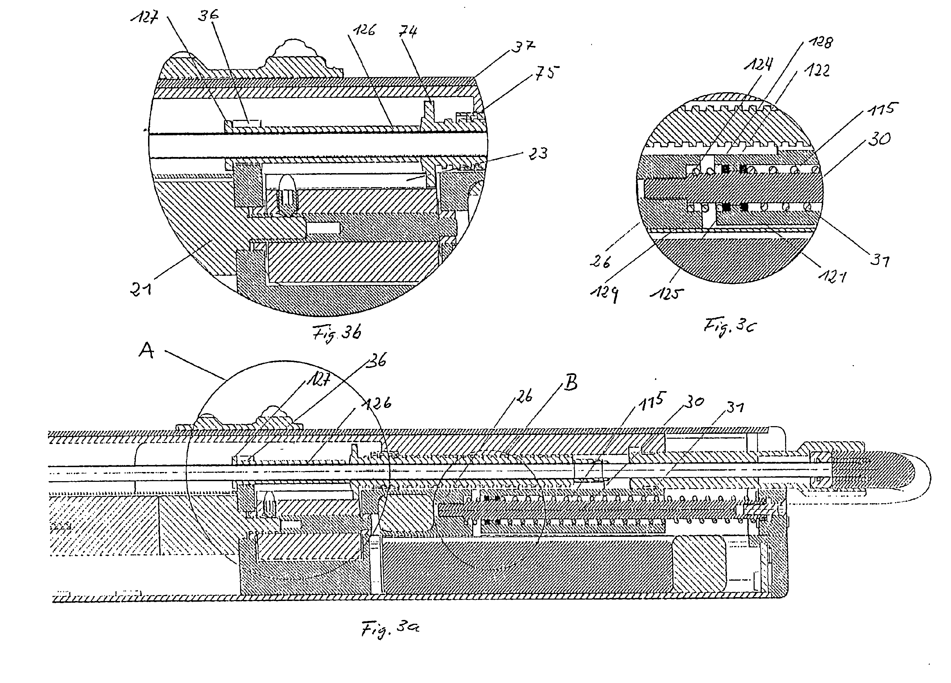

An der Wand 22 ist nach rechts zeigend ein weiterer Block 26 angeformt, der sowohl den verschwenkbaren Doppelhebel 33 für die Verriegelung aufnimmt, als auch zur Befestigung des Bolzens 30 für die Führung des Spannschlittens 28 dient. Der Bolzen 30 wird in die Gewindebohrung 29 eingeschraubt. Der Spannschlitten 28 gleitet beim Spannvorgang auf der darunter angeordneten Trennplatte 114 nach rechts. Beim Spannvorgang wird die auf dem Gewindebolzen 30 angeordnete Spiralfeder 31 zusammengedrückt. Die Spiralfeder stützt sich mit einem Ende auf einem Endstück 32 des Gewindebolzens oder direkt am Gehäuseenddeckel 7 ab; das andere Spiralfederende, das in eine Sackbohrung des Spannschlittens hineinragt, stützt sich über eine Beilagscheibe an einer Lippe 122 der Führungsbohrung 115 ab. Der Gewindebolzen 30, der einerseits im Gehäuseenddeckel 7 und andererseits im Block 26 befestigt ist, trägt an seinem distalen Ende eine kurze Spiralfeder 124 , die sich ebenfalls auf ihrer proximalen Seite an der Lippe in einer der Bohrung 115 gegenüber liegenden koaxialen Sackbohrung 129 über eine weitere Beilagscheibe 125 an der umlaufenden Lippe 122 abstützt. Beide Spiralfedern haben den gleichen Durchmesser und die distale und proximale Bohrung 129, 115 im Spannschlitten und die distalseitige Bohrung 128 im Block 26 sind im Durchmesser so gestaltet, dass die Spiralfedern leicht eingeschoben werden können. Alle Bohrungen sind koaxial zum Bolzen 30 angeordnet. In gleicher axialer Entfernung zur umlaufenden Lippe in der Sackbohrung des Schlittens weist der Gewindebolzen 30 einen Bund 123 auf. Der Spannschlitten 28 wird in seiner Ausgangsstellung (Ruheposition) durch die gering vorgespannten Federn 31, 124 über die Beilagscheiben in Ruheposition gehalten, wie in Fig. 3a und 3c dargestellt. Die Beilagscheiben liegen dabei sowohl an der entsprechenden Seite des Bundes und der Lippe an und stehen senkrecht; wird also der Schlitten nach rechts oder links ausgelenkt, so wird die jeweilige Feder versuchen, den Spannschlitten in seine Ausgangslage zurückzuführen, der Spannschlitten ist gewissermaßen "schwimmend" gehalten.

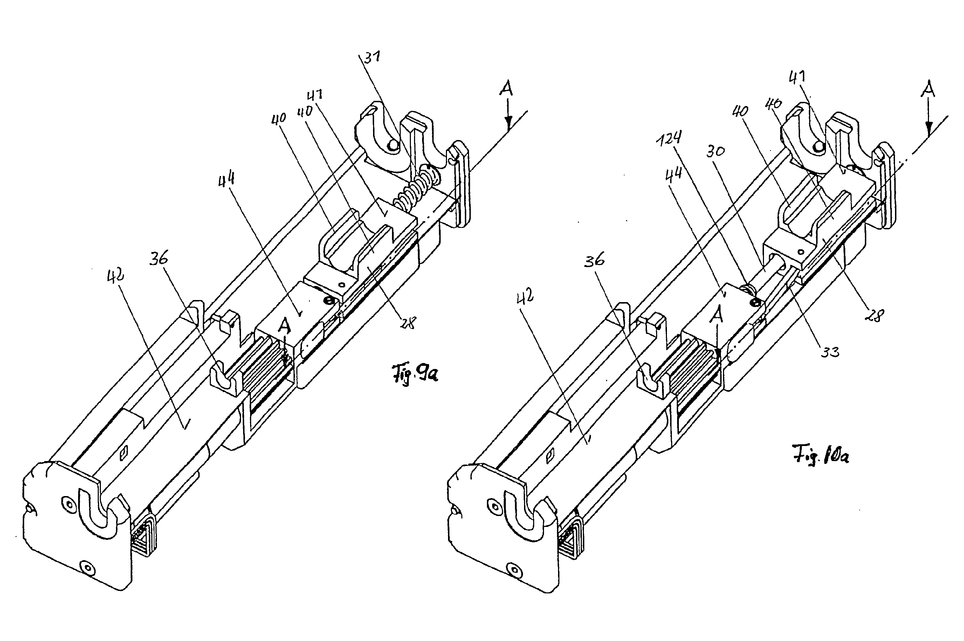

Der Spannschlitten 28 gleitet vor allem auf der Trennplatte 114 und ist durch diese und die Seitenwand verdrehgesichert. Ein Arm 99 des doppelarmigen Hebels 33 der Verrrastvorrichtung greift in eine Nut 27 des Spannschlittens 28 ein (Fig. 9a und 10a). Die in den Block 26 des Basisblocks 8 integrierte Verrastvorrichtung besteht aus dem doppelarmigen Hebel 33, der um eine senkrecht stehende Achse 35 (in Y-Achse gesehen) mittels einer Druckfeder 34 verschwenkbar ist. Die Achse 35 , ein senkrecht stehender Stift, ist in den Bohrungen 38 des Basisblocks befestigt. Im ungespannten Zustand liegt der Teil 99 des doppelarmigen Hebels in der Nut 27 des Spannschlittens; die zusammengedrückte Feder 34 wirkt auf den Teil 100 des Hebels ein um die Verrasttaste 88 nach außen (nach vorn) zu drücken. Die Verrasttaste ist im Bedienungspanel, das an dieser Stelle nach dem Spannen etwas nach außen gedrückt wird, leicht ertastbar. Sobald der Teil 99 des doppelarmigen Hebels in die Ausnehmung 82 des Spannschlittens einrasten kann, wird die Betätigungstaste 88 nach außen gedrückt. Der Spannschlitten wird durch Einrasten des Hebelteils 99 im gespannten Zustand verrastet und kann nun bei Bedarf mit der Betätigungstaste 88 ausgelöst werden. Da der Spannschlitten zweckmäßigerweise aus Kunststoff gefertigt ist, hat es sich als zweckdienlich erwiesen, in die Vertiefung ein Metallteil 83 einzusetzen um den Kunststoff nicht zu beschädigen, da ja der doppelarmige Hebel aus Metall gefertigt ist. Im Gegensatz zum herausnehmbaren Element 20 wird das Handstück mit ausgewechseltem Einlegeelement mehrfach verwendet. Der Spannweg entspricht der Eindringtiefe der Biopsienadel in das Gewebe. Daraus ergibt sich, dass die Länge des Hebels 99 ebenfalls dem Spannweg entspricht. Da die Eindringtiefen in der Regel zwischen 15 und 25 mm liegen, kann durch entsprechende Ausbildung der Länge des Hebels 99 und entsprechender Vorgabenänderung in der Steuerung, das gleiche Handstück für verschiedene Eindringtiefen verwendet werden.

Der sich an den Block 26 anschließende Spannschlitten 28 ist höhengleich zum Block 26 angeordnet und ist etwa querschnittgleich wie der Block 26. An seiner oberen Seite weist der Spannschlitten zwei Laschen 40 auf. Die nach oben zeigende Fläche 41 des Spannschlittens, sowie die nach oben zeigende Fläche 44 des Blocks 26, sowie die nach oben zeigende Fläche der Verlängerung 42 des Basisblocks 8, bilden zusammen eine plane Lagerfläche für die untere Gleitfläche 43 des aufzusetzenden Biopsienadelträgers 37 (sh. Fig. 2). Der Biopsienadelträger ist aus Kunststoff gefertigt. Bei der Verschiebung des Spannschlittens vom ungespannten Ausgangszustand (Fig. 9a) in den gespannten Zustand (Fig. 10a), also nach rechts, gleitet der von den Laschen 40 gehaltene Biopsienadelträger 37 über die Fläche 42 und 44. Es ist auch denkbar, dass die Gleitflächen nicht plan, wie beim Ausführungsbeispiel gestaltet sind, sondern eigens gestaltete Gleitflächen aufweisen; wichtig ist, dass der Biopsienadelträger 37 auf der Gleitfläche leichtgängig und geradlinig gleiten kann und dass nach dem Auslösen der Betätigungstaste 88 die Biopsienadel geradlinig in das Gewebe, den Tumor, eindringen kann. Deshalb ist auch die obere Außenkontur des Biopsienadelträgers der Innenkontur des Gehäusedeckels entsprechend ausgebildet und weist ein nur geringes Spiel zum Gehäusedeckel auf um ein Ausweichen der Biopsienadel nach oben zu verhindern, was auch von Vorteil beim Spannvorgang ist.

Oberhalb des U-förmigen Raumes 24 für die Zahnwalze 23, in der Höhe der Gleitfläche 42, weist der Basisblock 8 eine U-förmige, nach oben offene Halterung 36 u.a. für das Einlegen der Biopsienadel/Schneidhülse, auf. Diese Halterung dient vor allem als radiales Drucklager, also zur Abstützung für den mit der Schneidhülse verbundenen Antriebsteil, das Zahnrad 74, bzw. die Kunststoffscheibe 78, um den Spannschlitten mittels der Antriebsvorrichtung 106 in seine Spannposition zu bringen. Distalseitig dient die Halterung auch als Anschlag für den Bund 127 beim Erzeugen der Vorwärt-Rückwärtsbewegung und der damit verbundenen Windekldrehbewegung.

Im hinteren, oberen Teil des Basisblocks ist ein weiteres U-förmiges Einlegeelement 62 vorgesehen, in das das aus dem Spritzenkörper herausragende freie Ende 61 (distalseitige Ende) der Gewindespindel der Vakuum-/Druckerzeugungsvorrichtung eingelegt wird. Das Einlegeelement ist als Kanal ausgebildet, in dem die Gewindespindel 53 gleitet. In der Mitte, oben, des Basisblocks ist eine Befestigung für eine Platte, die die Ausnehmung 45 aufnimmt, vorgesehen, in die die Nase 12 des Verschlussriegels 11 des Gehäusedeckels eingeschoben wird. Eine am Basisblock 8 angeordnete Abdeckung 46, die nach links zeigt, trennt den Raum der Antriebsmotoren und der eingesetzten Platine, von dem oberen, linken Teil des Gehäuseinnenraums, der vor allem der Lagerung des austauschbaren Biopsienadelträgers 37, einschließlich Biopsienadel und Schneidhülse dient. Die Abdeckung 46 schützt die elektrischen Getriebemotoren und die Platine vor Verschmutzung. Die Platine für die Elektronik liegt zwischen den Antriebsmotoren und unterhalb der Mittelrippe im Raum 39.

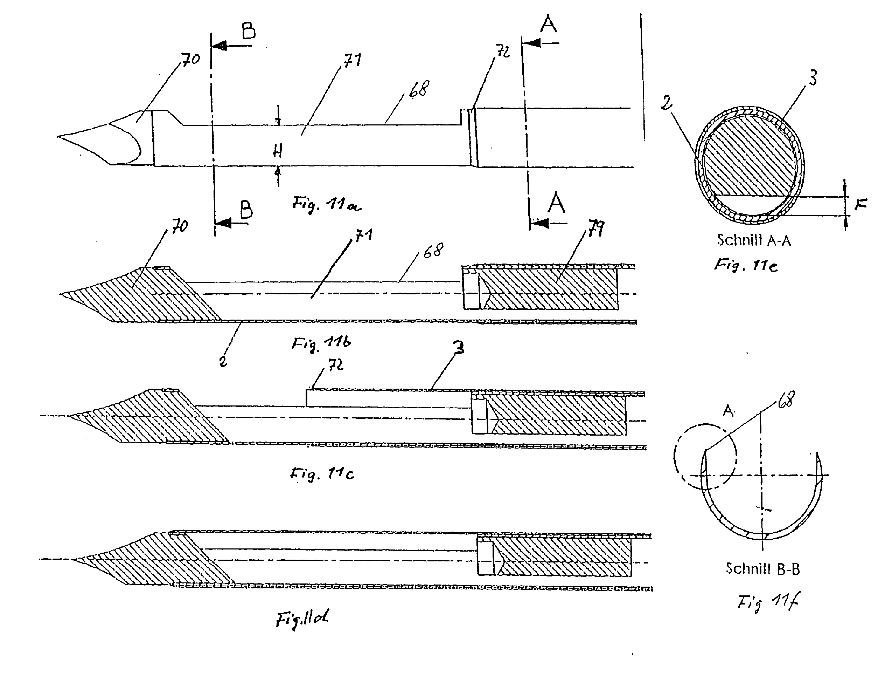

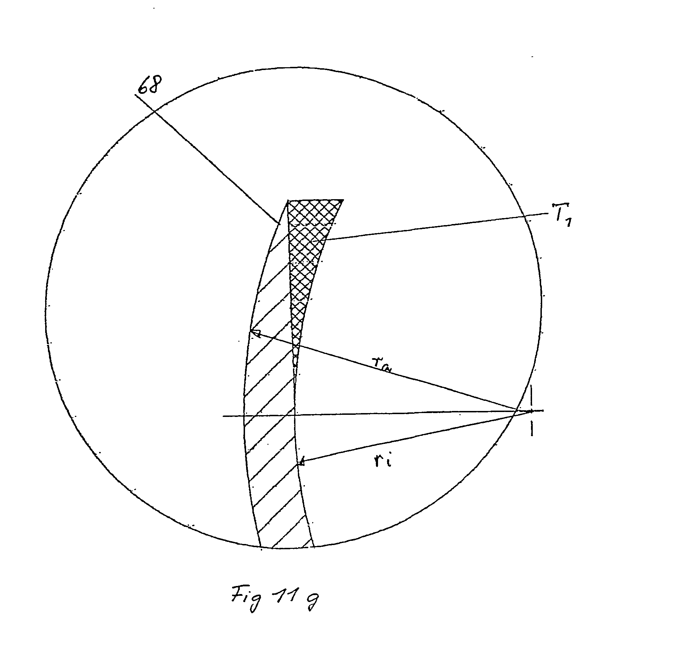

Den Biopsienadelträger 37, der in die Laschen 40 des Spannschlittens 28 mit Biopsienadel 2 und Schneidhülse 3 sowie weiteren Teilen einlegbar ist, zeigt Fig. 2. Die hohle, kreisrunde Biopsienadel 2 hat eine Nadelspitze 70, an die sich der Probeentnahmeraum 71 anschließt (Fig. 11a - 11f). Die querschnittsrunde Biopsienadel 2 wird von einer koaxial angeordneten, querschnittsrunden Schneidhülse 3 umgeben, die an ihrem linken, dem Probeentnahmeraum zugewandten Ende eine Schneide 72 aufweist, die dazu dient, nach Einführung der Biopsienadel (mit geschlossenem Probeentnahmeraum) und nach Öffnen des Probeentnahmeraums und Durchführung einer mehrmaligen Vorwärts- und Rückwärtsbewegung der Nadel, die in einer besonders bevorzugten Ausführungsform gleichzeitig von einer vorher festgelegten, begrenzten Winkeldrehbewegung der Biopsienadel um ihre Längsachese überlagert wird, die Probe herauszuschneiden und im geschlossenen Probeentnahmeraum zu halten, wie später näher beschrieben. Die distal angeordnete Schneide der Schneidhülse steht vorzugsweise senkrecht zur Längsachse von Biopsienadel und Schneidhülse. Der Abtrennvorgang geschieht vorzugsweise durch Rotation und gleichzeitiger Längsverschiebung der Schneidhülse mittels des Gewindespindelantriebs. Es ist auch denkbar, dass die Bewegung der Schneidhülse nicht kontinuierlich erfolgt, sondern schrittweise oder vibirerend, d.h. der Vorschub wird in kurzen Abständen vor und zurück bewegt. Wie insbesondere Fig. 11f im Querschnitt zeigt, liegen die Längskanten 68 des Probeentnahmeraums oberhalb des Mittelpunktes des Querschnitts, d.h. der Probeentnahmeraum ist über die Z-Achse hinaus um ca. 15 - 30° hochgezogen. Um nun das Eindringen von festem, hartem Gewebe in den Probeentnahmeraum zu verbessern, weisen die Längskanten eine Schneide auf. Diese Schneide an den Längskanten wird dadurch erzeugt, indem von oben her die Wandstärke so reduziert wird, dass die Breite b' an der Schneidkante der Breite b eines tiefer liegenden Schneidhülsenrohr-Innendurchmessers entspricht, d.h. die Wandstärke wird im oberen Teil reduziert und so zur Ausbildung der Schneidkante genutzt (sh. Fig. 11f und Vergrößerung in Fig. 11 g).

Auf dem der Schneide 72 abgewandten anderen, proximalen Ende der Schneidehülse ist eine Gewindespindelhülse 73 mit einem auf der Stirnseite der Gewindespindelhülse angeordneten Zahnrad 74 befestigt. Die Gewindespindelhülse mit Zahnrad ist auf der Schneidhülse dreh- und verschiebesicher angeordnet. Mit der Gewindespindel arbeitet eine Gewindespindelmutter 75 zusammen, die fest in den Biopsienadelträger 37 eingepresst ist. Das Zahnrad 74 liegt links ,also vor dem Beginn der Spindelhülse. Bei Verdrehen der Gewindespindelhülse mittels des Zahnrades 74 wird die Schneidhülse gedreht und in Längsrichtung über der Biopsienadel 2 verschoben.

Distalseitig vom Zahnrad 74 ist mit der Gewindespindel ein Rohrstück 126 mit dem Bund 127 fest verbunden. Das Rohrstück wird in die Halterung 36 eingelegt, wobei der Bund 127 hierbei distalseitig vor der Halterung liegt. Die Länge des Rohrstücks 126 entspricht in etwa dem Spannweg, wobei zusätzlich die Wandstärke der Halterung 36 zu berücksichtigen ist (s. Fig. 3a und 3b). Der Bund 127 wandert bei der Grundstellung des Gerätes (geschlossener Probeentnahmeraum) nach links, zur distalen Seite, während er nach Öffnen des Probeentnahmeraums an der Halterung 36 (distalseitig) zur Anlage kommt. Bei weiterem Drehen der Spindelhülse mit Schneideinrichtung, also bei dem Versuch den Probeentnahmeraum weiter zu öffnen, wird der Spannschlitten gegen die Wirkung der kurzen Spiralfeder zum Block 26 hingezogen, weil der Bund 127 distalseitig an der Halterung 36 anliegt. Dadurch kann, wie später beschrieben, die Biopsienadel in eine Vor- und Rückwärtsbewegung gebracht werden, die von einer begrenzten Winkeldrehbewegung der Biopsienadel nach beiden Seiten überlagert wird. Diese Winkeldrehbewegung erfolgt dadurch, dass die Schneidhülse versucht, die Biopsienadel mit zu verdrehen, die Biopsienadel jedoch andererseits gehindert wird, sich über eine vorgegebene Winkeldrehung hinaus zu verdrehen, wie insbesondere die Zeichnungen 12b - 12d zeigen.

Das Zahnrad 74 am linken Ende der Gewindespindel kämmt nach dem Einsetzen des Biopsienadelträgers in die Laschen 40 mit der Zahnwalze 23. Um den Biopsienadelträger 37 bei nicht gespanntem Spannschlitten (Fig. 2) in die Laschen des Spannschlittens einsetzen zu können, weist der Biopsienadelträger zwei plane, parallele Ausnehmungen 77 auf (Fig. 2). Beim Aufsetzen der Gleitfläche des Biopsienadelträgers 37 auf die Flächen 41, 42 und 44 wird gleichzeitig die Biopsienadel in die Halterung 36 des Basisblocks 8 eingesetzt. Auf der linken Seite des Zahnrades kann zur Verbesserung der Drehbarkeit des Spindelantriebs, insbesondere, wenn die Halterung 36 als Abstützung für das Spannen des Spannschlittens dient, eine Kunststoffscheibe 78 eingefügt sein, die mit einem leichten Konus versehen ist. Bei korrekt eingelegtem Biopsienadelträger gleitet beim Spannen des Spannschlittens der Biopsienadelträgers mit der Gleitfläche 43 über die Flächen 42 und 41 nach rechts. Da nach dem Einlegen des Biopsienadelträgers zunächst der Probeentnahmeraum geschlossen wird, liegt das Zahnrad 74 an der Halterung 36 an. Wird nun die Zahnwalze 23 weiter in gleicher. Richtung angetrieben, so schraubt der Gewindespindelantrieb über den Biopsienadelträger den Spannschlitten nach rechts, bis er verrastet ist; dabei wird die Biopsienadel nach innen gezogen, während die Schneidhülse in ihrer Position verbleibt. Die Schneidhülse ragt nach dem Verrasten über die Biopsienadelspitze hinaus. Es wird daher nach Verrastung des Spannschlittens die Schneidhülse in die Ausgangslage (entgegengesetzte Drehrichtung) zurückgedreht; das Zahnrad 74 verschiebt sich hierbei in der Zahnwalze von links nach rechts. Nach dem Entrasten des Spannschlittens gleitet mit dem Biopsienadelträger die Biopsienadel-/Schneidhülse mit Zahnrad wieder nach links. Nun kann die Schneidhülse wieder nach rechts verschoben werden um den Probeentnahmeraum zu öffnen bis der Bund 127 zur Anlage kommt.

Die Funktion des "schwimmend" gelagerten Spannschlittens in Verbindung mit dem steuerbaren Antriebsmotor und des mit der Schneidhülse verbundenen Rohrstücks 126 mit dem Bund 127 wird im Zusammenhang mit dem Ablauf der Biopsie näher beschrieben.

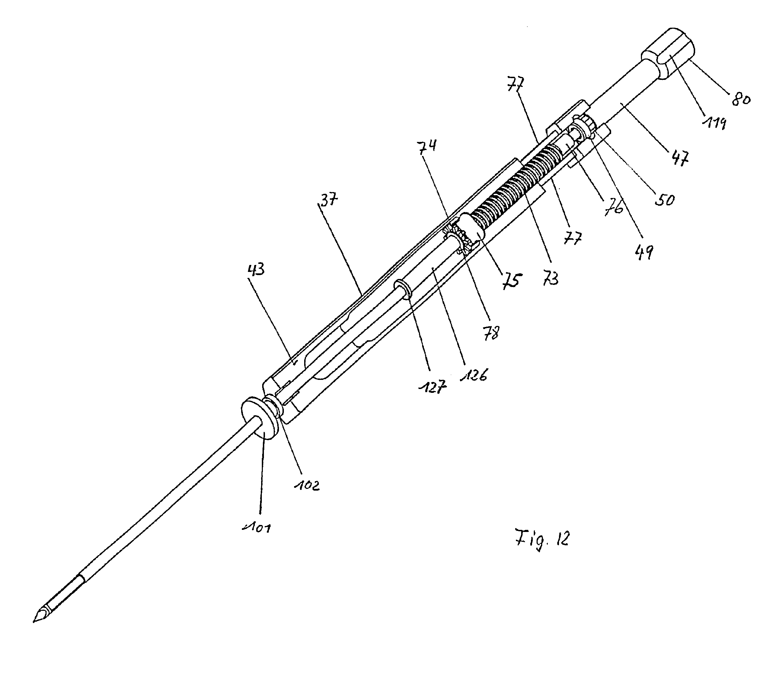

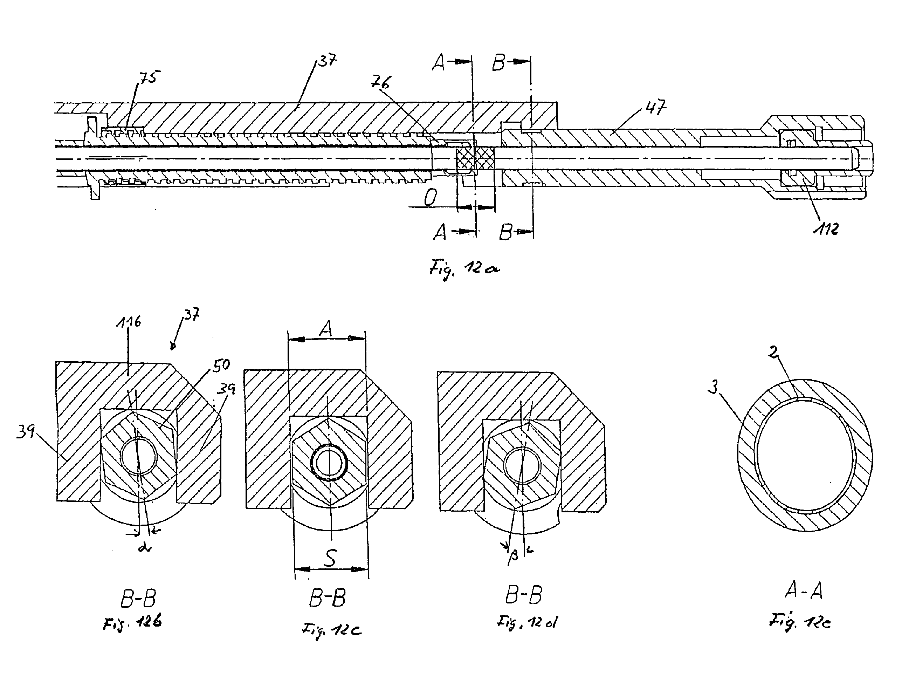

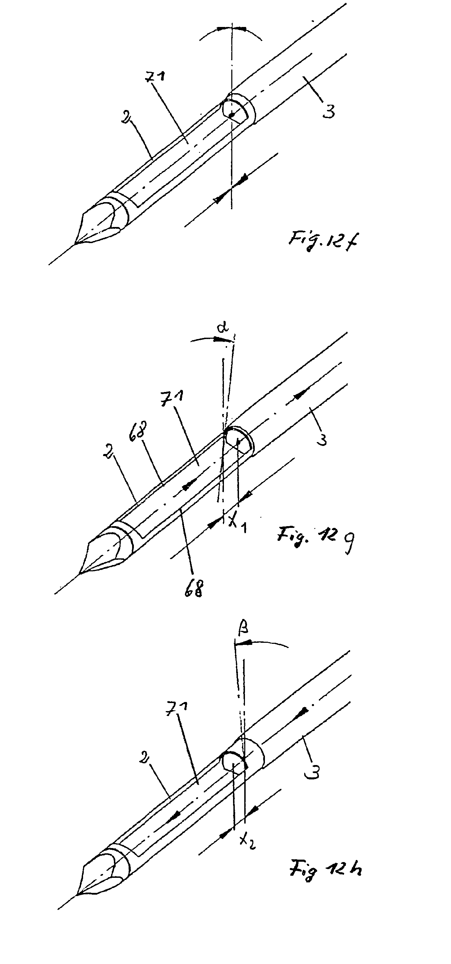

Das rechte Ende der Schneidhülse ist über ein Dichtelement 76 mit der hohlen Biopsienadel rotationsbeweglich, aber luftabschließend verbunden, damit weder Luft zwischen Biopsienadel und der sie koaxial umgebenden Schneidhülse eindringen, noch bei Überdruck Luft austreten kann. Das Dichtelement 76 besteht aus einem Kunststoffschlauch, der über das proximale Ende der Schneidhülse gezogen wird. Der Innendurchmesser ist so gewählt, dass er auf dem Außendurchmesser der Biopsienadel leicht anliegt. Bei Anlegen von Unterdruck im Innenraum der Biopsienadel und damit auch zwischen Biopsienadel (Außenseite) und Schneidhülse (Innenseite) wird der elastische Kunststoffschlauch gegen den Außendurchmesser der Biopsienadel gezogen. Der Schlauch kann dann, sofern die Biopsienadel gegenüber der Schneidhülse verdreht wird, als Rückstellelement (Rückstellfeder) dienen. Um die Biopsienadel mittels der Schneidhülse geringfügig zu verdrehen ist die Biopsienadel im Bereich des Dichtelements 76 leicht deformiert, so dass sie an der deformierten Stelle 0 oval ist, (sh. auch Fig 12f). Beim Verdrehen der Schneidhülse wird die Biopsienadel durch die Verformung 0 mit verdreht, und zwar soweit, bis das Verdrehen der Biopsienadel durch Anschlag begrenzt ist (Fig. 12b-12d).

Diese Drehwinkelbewegung der Biopsienadel hat zur Folge, dass gleichzeitig die geschärften Längskanten des Biopsienadelraumes um die Biopsienadellängsachse nach beiden Seiten verschwenkt werden. Da diese Drehwinkelbewegung durch den gleichen Antrieb und gleichzeitig mit der Vor-/Rückwärtsbewegung der Biopsienadel erfolgt, trennen die Schneidkanten des Probeentnahmeraums nach Art eines angetriebenen Messers sowohl longitudinal in der X-Achse als auch gleichzeitig winkelverdreht das Gewebe auf, so dass das unter Druck (Außen- und/oder Innendruck) stehende Gewebe in den geöffneten Probeentnahmeraum gesichert eindringt. Fig. 12f zeigt die Stellung des Probeentnahmeraums in der neutralen Grundstellung nach Öffnung; Fig. 12g zeigt die Stellung nach einer Winkeldrehung um den Winkel α nach rechts und gleichzeitigem Zurückfahren der Biopsienadel um den Betrag X₁ (ca. 2 mm) nach der Proximalseite; Fig. 12h zeigt die Lage der Biopsienadel bei Linksdrehung um den Winkel β und gleichzeitiger Bewegung der Biopsienadel nach der Distalseite um den Betrag X₂ (ca. 2 mm). Durch die Bewegung der Schneidkanten des Probeentnahmeraums, bzw. der Biopsienadel wird sichergestellt, dass das Gewebe in jedem Fall an den Längskanten aufgetrennt wird, unabhängig von der Gewebestruktur. Die beschriebene Bewegung der Biopsienadel und damit der geschärften Längskanten des Probeentnahmeraums führt auch dazu, dass der aufgetrennte Gewebeteil in den Probeentnahmeraum eingelegt wird, auch wenn der üblicherweise angelegte Druck nicht vorhanden ist.

Auf das rechte Ende der Biopsienadel 2 ist ein rundes, ebenfalls hohles Kunststoffteil 47 luftdicht aufgesetzt und mit der Biopsienadel kraftschlüssig verbunden. Das Kunststoffteil 47 hat an seinem linken Ende ein Lagerelement 49, das in den Biopsienadelträger eingepresst ist; an seinem aus dem Handstück herausragenden rechten Ende ist ein weiteres Kunststoffteil 112 eingesetzt, das gegenüber dem Kunststoffteil 47 und gegenüber der Biopsienadel 2 drehbeweglich ist. Zwischen Biopsienadel und Kunststoffteil 112 ist ein O-Ring zur Abdichtung eingesetzt. Das Kunststoffteil hat an seinem rechten Ende einen Zapfen 17, auf den das Verbindungselement 4 luftdicht aufgeschoben wird. Ebenfalls am rechten, aus dem Biopsienadelträger und dem Gehäuse herausragenden Teil, befindet sich eine Rändelscheibe 80, mit der durch Drehen die Lage des Probeentnahmeraums radial verstellt werden kann, ohne dass die Position der Schneidhülse verändert wird. Mit einer Verdrehung der Biopsienadel ist allein eine Verdrehung des Probeentnahmeraums verbunden. Das Kunststoffteil 47 mit Biopsienadel und Schneidhülse wird mit dem Lagerelement 49 und der Gewindespindelmutter 75 in den Biopsienadelträger eingepresst. Die Biopsienadel ist über das Lagerelement 49 und seine enge Führung in der Schneidhülse drehbeweglich im Biopsienadelträger und in der Schneidhülse gelagert und mit dem Biopsienadelträger in der Längsachse verschiebbar. Wie vorher beschrieben, ist die Schneidhülse gegenüber der Biopsienadel durch Verdrehen axial beweglich.

Rechts vom Lagerelement 49 ist ein Vielkant 50 auf dem Kunststoffteil 47 angeordnet, der mit dem Biopsienadelträger 37 durch Verspannen verrastbar ist, so kann der Probeentnahmeraum der Biopsienadel mittels der Rändelscheibe 80 in die für die Biopsieentnahme günstigste Position gebracht und gehalten werden. Beim Verdrehen werden die beiden Schenkel 39 des Biopsienadelträgers, der aus einem elastischen Kunststoff besteht, durch die Ecken des Vielkants aufgeweitet bis die Flächen des Vielkants wieder nahezu senkrecht zu den Schenkeln 39 stehen und somit der Vielkant wieder verrastet ist (sh. insbesondere Fig. 12c). Der Vielkant ist danach um eine vorgegebene Stufe verstellt. Handelt es sich bei dem Vielkant um ein Sechseck, so erfolgt das Verdrehen um jeweils 60°, will man mehr Verdrehstufen haben, so wählt man dementsprechend einen Vielkant mit 8, 10 usw. Stufen aus.

Wie insbesondere Fig. 12b - 12f zeigen, hat der Biopsienadelträger zwei Schenkel 39, die über einen Holm 116 miteinander verbunden sind. In dem Kunststoffträger ist der Vielkant 50 des Kunststoffteils verrastbar gelagert, in dem die mit dem elastischen Querholm verbundenen Schenkel 39 beim Verdrehen zunächst nach außen aufgeweitet werden um anschließend wieder durch die Elastizität in ihre Ausgangslage zurückzukehren. Wählt man nun eine Schlüsselweite S für den Vielkant, die geringer ist als der Abstand A (lichte Weite) der beiden Schenkel voneinander, so ist die Biopsienadel geringfügig nach beiden Seiten um ihre Achse um einen vorher bestimmten Winkel (α bzw. β) nach rechts und links um die Längsachse begrenzt verdrehbar (Fig. 12b und Fig. 12d, Fig. 12c zeigt die Mittelstellung). Die Schenkel 39 des Biopsienadelträgers werden hierbei nicht aufgeweitet; sie verhindern im Gegenteil, dass die Biopsienadel um einen größeren Winkel verdreht wird, da der Antrieb so gestaltet ist, dass zwar die Schneidhülse noch weiter verdreht werden kann, der Widerstand der Schenkelbegrenzung jedoch größer ist als das Antriebsmoment. Die Ecken des Vielkants schlagen also an die Schenkel 39 an und verhindern ein weiteres Verdrehen, weil das auf die Biopsienadel wirkende Drehmoment zu einer Aufweitung der beiden Schenkel nicht ausreicht. Da das Kunststoffteil 47 mit dem Vielkant fest mit der Nadel verbunden ist und die Schneidhülse bei geöffnetem Probeentnahmeraum auf den deformierten Bereich 0 der Biopsienadel aufgeschoben wurde, und letztlich auch das Dichtelement 76 auf die Außenseite der Biopsienadel bei geöffnetem Probeentnahmeraum gewissermaßen eine Reibverbindung eingeht, wird beim Antreiben der Schneidhülse, je nach Drehrichtung , die Biopsienadel durch diese Reibverbindung um ihre Achse verdreht, bis der Anschlag des Vielkants ein weiteres Verdrehen - mangels eines größeren Drehmoments - verhindert. Da das elastische Dichtelement in dieser Phase durch den herrschenden Unterdruck verstärkt gegen die Außenfläche der Biopsienadel gezogen wird, bewirkt dieses, dass beim Verdrehen der Schneidhülse gegenüber der Biopsienadel das Dichtelement einerseits das Verdrehen begünstigt; andererseits wirkt es als Rückstellelement, sofern es geringfügig touchiert wurde. Diese begrenzte Drehbewegung wird als Winkeldrehbewegung verstanden. Die begrenzt verdrehbare Biopsienadel kehrt aus der Winkeldrehbewegung durch Änderung der Drehrichtung und infolge des verdrillten Dichtelements in ihre Ausgangslage zurück um dann erneut gegen die Wirkung des elastischen Dichtelements in der anderen Richtung verdreht zu werden. Für die begrenzte Winkeldrehbewegung in Verbindung mit der Vor-/Rückwärtsbewegung, wie nachfolgend beschrieben, reicht im Allgemeinen etwa eine Umdrehung des Zahnrads 74 nach jeder Richtung (jeweils ca. eine Umdrehung von der Nullstellung). Bei einer Drehbewegung des Zahnrads wird die Biopsienadel ca. 2 mm nach links bzw. nach rechts aus der Nullstellung verschoben und gleichzeitig um den Winkel α bzw. β um die Längsachse bewegt. Im Allgemeinen wird dieser Bewegungsablauf nach jeder Richtung ca. 5 mal wiederholt .

Wie insbesonderes Fig. 12 zeigt, ist die koaxial die Biopsienadel umgebende Schneidhülse über die Gewindespindelmutter 75 mit dem Biopsienadelträger 37 verbunden. In der Gewindespindelmutter 75 ist die Gewindespindelhülse 73 verdrehbar gelagert. Ein Verdrehen des Zahnrades 74 durch den Antriebsmotor der Zahnwalze 23 bewirkt, dass der Biopsienadelträger und der Spannschlitten nach rechts bewegt wird sobald das Zahnrad 74 zur Anlage an der Halterung 36 kommt. Bei einer Stellung des Zahnrades innerhalb der Länge der Zahnwalze 74, also sofern das Zahnrad frei ist und weder an der Halterung noch an der Gewindespindelmutter 75 anliegt, kann die Schneidhülse alleine verstellt werden, so z.B. nach dem Spannen der Biopsienadel zum Ausgleich von Nadelspitze und Schneidhülse um die Schneidhülse in die Ausgangsstellung zurückzuführen oder zum Öffnen und Schließen des Probeentnahmeraums.

Beim Öffnen des Probeentnahmeraums wird die Schneidhülse über den leicht deformierten Bereich 0 der Biopsienadel geschoben. In dieser Stellung wird bei weiterer Drehung der Schneidhülse die Biopsienadel in Drehrichtung um einen vorgegebenen Winkel mitgenommen; da jedoch die Verbindung Schneidhülse/Biopsienadel nur die Übertragung eines vorbestimmten Drehmoments zulässt, kommt die Drehbewegung der Biopsienadel zum Stillstand, wenn die entsprechenden Ecken des Vielkants an den Schenkeln des Biopsienadelträgers zur Anlage kommen (s. Fig. 12b bzw. 12d).

Das auf der distalen Seite des Zahnrades angebrachte Rohrstück 126 mit Bund 127, das mit der Halterung 36 zusammenwirkt, dient dazu, im Zusammenwirken mit der Steuerung die Nadel in eine kurzzeitige Rüttelbewegung (Vor- und Rückwärtsbewegung) zu versetzen und gleichzeitig wie beschrieben die Biopsienadel in eine wechselnde Winkeldrehbewegung zu versetzen.

Da die Rüttelbewegung (Vor- Rückwärtsbewegung) über den Antrieb für die Schneidhülse 3 erfolgt, wird gleichzeitig über die Verbindung Schneidhülse mit der deformierten Stelle "0" der Biopsienadel und durch die Gestaltung des Vielkants 50 sowie des Biopsieträgerteils eine begrenzte Verdrehung der Biopsienadel nach beiden Drehrichtungen (je nach Drehrichtung) durchgeführt, die die Vor-/Rückwärtsbewegung der Biopsienadel überlagern. Durch diese beiden gemeinsamen Bewegungen wird jede Art von Gewebe durch die Schneidkanten des Probeentnahmeraums aufgetrennt. Das Einziehen , bzw. Einlagern des Gewebes in den Probeentnahmeraum wird auch bei zähem oder mit Einschlüssen versehenem Gewebe mit und/oder Druck zuverlässig erreicht. Die vorher erwähnte Vor-/Rüttelbewegung der Biopsienadel (Vor- und Rückwärtsbewegung), die auch die Winkeldrehbewegung erzeugt, wird nachfolgend beschrieben:

Beim Antrieb der Zahnwalze wird über das Zahnrad 74 der Probeentnahmeraum soweit geöffnet bis der Bund an der Halterung 36 distalseitig zur Anlage kommt. Wird nun die gleiche Drehrichtung beibehalten, und das Zahnrad liegt nicht an der Gewindespindelmutter an, so bewirkt das weitere Drehen, dass der Spannschlitten über den Biopsienadelträger zum Block 26 gegen die Wirkung der kurzen Spiralfeder gezogen wird, da ein weiteres Öffnen der Schneidhülse durch das Anliegen des Bundes 127 an der distalen Seit der Halterung 36 nicht möglich ist. Der Spannweg oder der Bewegungsweg (X₁ bzw. X₂) beträgt ca. 2mm oder entspricht ca. 1 Umdrehung der Schneidhülse. Mit dem Anschlag des Zahnrads 74 an der Gewindespindelmutter wird die Drehrichtung des Motors umgekehrt und mit Unterstützung der kurzen Spiralfeder wird der Spannschlitten in seine Ausgangsposition (Ruhelage) zurückgedrückt, die Biopsienadel wird in ihre Null-Position zurückgedreht. Da ein Messgeber die Motorumdrehungszahlen zählt, und die Ist-Werte in einem programmierbaren Mikroprozessor abgelegt sind, kann über entsprechende Vorgaben die Motordrehrichtung nach den Vorgaben umgesteuert werden, so dass der Spannschlitten erneut gegen den Block gezogen, bzw. nach Freigabe zurückgefahren wird. Durch die ständige Umdrehung der Drehrichtung des Motors, gemäß Vorgabe, in Zusammenwirken mit dem Spannen und Freigeben des Schlittens führt die Biopsienadel eine Vor-/Rückwärtsbewegung aus, die von einer begrenzten Winkeldrehbewegung der Biopsienadel nach beiden Seiten je nach Drehrichtung überlagert wird. Fünf Vor-Rückwärtsbewegungen sind im Allgemeinen ausreichend um auch bei hartem und/oder zähem Gewebe oder Gewebe mit Einschlüssen, z.B. Kalk, eine gute Probeentnahme zu gewährleisten. Durch die Vor- und Rückwärtsbewegung in Verbindung mit der begrenzten Winkeldrehbewegung der Biopsienadel und den geschärften Schneidkanten wird das Gewebe, das z.B. vom Vakuum an den Probeentnahmeraum angezogen wird, an den Seitenrändern durchgetrennt, um das Einlegen der Probe auch bei schwierigem Gewebe in den Probeentnahmeraum zu ermöglichen bzw. erheblich zu erleichtern.

Die beschriebene Bewegung der Biopsienadel und damit der geschärften Längskanten des Probeentnahmeraums ermöglicht ein ausgezeichnetes Auftrennen des Gewebes nach dem Öffnen, bzw. während des Öffnens des Probeentnahmeraums. Die gleiche gute Wirkung der Gewebeauftrennung wird auch erzielt, wenn bereits während des Öffnens, also beim Zurückfahren der Schneidhülse, diese Rüttelbewegung, bzw. Winkeldrehbewegung erzeugt wird.

In den Fig. 12g - 12f ist diese überlagerte Bewegung des Biopsienadelraumes nochmals im Einzelnen in den Phasen Links- und Rechtsdrehung sowie Nullstellung dargestellt. Die Fig. 12g u.a. zeigen die Ausgangssstellung; der Biopsienadelraum ist geöffnet, die Schneidhülse ist ca. 2mm über die proximale Kante des Probeentnahmeraums zurückgefahren, der Vielkant steht in Neutralstellung (Fig. 12c). Bei der Darstellung in Fig. 12h und Fig. 12g wird die Biopsienadel in der Schneidhülse zurückgefahren und gleichzeitig die Nadel um den Winkel α verschwenkt. Fig. 12f und Fig. 12d zeigen die andere Drehrichtung und die Verdrehung um den Winkel β. Die proximale Kante des Biopsienadelraums ist hierbei um ca. 2 mm zur distalen Seite gegenüber der Grundstellung verschoben und gleichzeitig um den Winkel β in die andere Richtung verschwenkt. Fig. 12g - 12f und Fig. 12b - 12d zeigen einen Zyklus, der je nach Steuerung durch den Mikroprozessor mehrmalig wiederholt wird, in der Regel sind es 5 Zyklen. Diese Vor-/Rückwärtsbewegung und begrenzte Winkeldrehbewegung kann über elektrische Elemente, die mit der Nadel oder dem Spannschlitten verbunden sind, ebenso erzeugt werden.

Einzelheiten zum Probeentnahmeraum sowie zu der Ausbildung der Biopsienadelspitze sind in den Fig. 11a -11g dargestellt. Der an die Nadelspitze 70 anschließende Probeentnahmeraum 71 ist etwa über 25% seines Querschnitts nach oben offen: So ist bei einem Biopsienadelaußendurchmesser von 3,3 mm die Höhe H des Probeentnahmeraumes ca. 2,3 mm. Der Probeentnahmeraum ist etwa zwischen 15 bis 25 mm lang. Daran schließt sich der Hohlraum der Biopsienadel an. Am Übergang, also am proximalen Ende des Probeentnahmeraumes, ist der Hohlraumquerschnitt der Biopsienadel zwischen 50% und 75% durch eine Einengung, z.B. einen Stopfen 79 verschlossen (Fig. 11b - 11e). Die Höhe des Stopfens ist so ausgelegt, dass er über die Ausnehmung für den Probeentnahmeraum nach unten reicht. Wie Fig. 11e zeigt, ist am Boden des Probeentnahmeraums eine Öffnung F von der Höhe von 0,6 mm bei einem Nadelinnendurchmesser von 3,0 mm. Das Vakuum soll dadurch vor allem die Gewebeprobe mit dem kontinuierlichen Öffnen des Probeentnahmeraums in den Probeentnahmeraum nach unten hineinziehen und an der Wand des Probeentnahmeraums zur Anlage bringen. Bei Überdruck im Biopsienadelhohlraum wirkt die Einengung, der Stopfen, druckerhöhend. Der Stopfen hat etwa die Länge von 10 mm und ist in den Hohlraum eingeklebt oder eingeschweißt. Beim Lasereinschweißen hat sich gezeigt, dass es vorteilhaft ist, die linke Seite des Stopfens durch Herausnahme von Material an der Stirnfläche auf eine kurze Länge, ca. 2 mm, dünn zu gestalten. Dadurch wird in diesem Bereich an der Stirnfläche das Rohr der Biopsienadel mit der Stirnseite des Stopfens durchgeschweißt und ist an der Stirnseite luftdicht. Der Stopfen kann auch von geringerer Länge sein, sofern die gleiche Wirkung erzielt wird. So kann der Stopfen auch durch eine Lippe oder Nase von etwa gleicher Höhe ersetzt werden. Wichtig ist, dass die Verengung so gestaltet ist, dass das Vakuum im Probeentnahmeraum vor allem vom Boden her zur Wirkung kommt, damit die Probe beim Schließen der Schneidhülse, also beim Schneidevorgang, an der Wand des Probeentnahmeraums anhaftet und ihre Lage nicht verändert. Es hat sich auch als vorteilhaft erwiesen, evtl. zusätzliche Fixiermittel an der Probeentnahmewand vorzusehen. Durch das Einsaugen der Probe von unten in den Probeentnahmeraum ergibt sich zum einen ein hoher Füllungsgrad des Probeentnahmeraums und zum anderen, vor allem durch seine Gestaltung, eine gute Fixierung der Probe an der Wand. Aus diesem Grund ist es wichtig, dass die seitliche Auftrennung des Gewebes durch die beschriebene Bewegung der geschärften Längskanten des Probeentnahmeraums ein Eindringen des Gewebes bis zum Boden sicherstellt. Da die Schneidhülse die Probe auf der Außenseite der Biopsienadel abtrennt, bleibt dieses Festsaugen der Probe in der Innenseite möglichst auch beim Trennvorgang erhalten. Durch die außen angeordnete Schneidhülse und das auf dem Innenboden des Probeentnahmeraums haftende Gewebe wird beim Schließvorgang durch die rotierende Längsbewegung der Schneidhülse die Probe selbst weder verdreht noch verdrillt. Die Qualität der Probe ist dadurch erheblich verbessert, als bei Anlagen mit Drilleffekt. Der Pathologe erhält ein Ausgangsmaterial, das im Querschnitt dem Schnitt im Gewebe entspricht und nicht eine verdrillte oder verformte Gewebemischung. Der Auswurf der Probe unter Druck ist eine sichere Form der Ablage. Durch den Stopfen 79 wird dies ermöglicht. Zusätzlich findet eine vollständige Reinigung des Probeentnahmeraums statt, so dass bei Wiederholung der Biopsie keine Vermischung von Gewebeproben (Restpartikel) stattfindet. Da die Vakuumerzeugungsvorrichtung gleichzeitig als Druckerzeugungsvorrichtung genutzt wird, wird der gesamte Hohlraum, insbesondere die Biopsienadel beim Auswurf gereinigt. Bei normalem Gewebe genügt es, die Wandstärke des Biopsienadelrohres von ca. 0,15 mm als Seitenschneidkante zu verwenden. Bei hartem und/oder festem Gewebe ist der Füllungsgrad im Probeentnahmeraum allein durch das Einsaugen mittels Vakuum nicht ausreichend, da das Gewebe an den Seitenkanten nicht genügend aufgetrennt wird. Durch die Ausbildung der Längsseiten des Probeentnahmeraums 71 zu Schneidkanten 68, wie insbes. Fig. 11g und Fig. 11f zeigen, wird durch Überlagerung einer mehrmaligen Vorwärts-/Rückwärtsbewegung und einer begrenzten mehrmaligen Winkeldrehbewegung der Biopsienadel mit dem Probeentnahmeraum nach links und rechts (wie beschrieben) sowie durch die Druckeinwirkung, z.B. durch Innenvakuum, das Gewebe der Probe längsseitig aufgetrennt, so dass die Probe mittels Vakuum oder Außendruck erheblich besser zum Boden des Probeentnahmeraums wandert. Durch Anlegen eines Außendrucks oder eines Innenvakuums, z.B. mittels des Ultraschallkopfes oder eines Vakuums im Biopsienadelhohlraum kann die Schneidwirkung der Längskanten verstärkt werden. Durch das Auftrennen der längsseitigen Schnittflächen der zu entnehmenden Gewebeprobe wird auch bei hartem und/oder zähem Gewebe bzw. Gewebe mit Einschlüssen durch die Bewegung der Nadel ein hervorragender Füllungsgrad erzielt. Auf diese Weise wird genügend Gewebematerial für die Untersuchung bereit gestellt. Die Schneidkante an der Längsseite der Probeentnahmeeinrichtung wird dadurch ausgebildet, dass das Teilstück (T1) von der Wandstärke abgefräst wird (Fig. 11 g). Der Durchmesser (ra) der Außenkontur des Biopsienadelrohres bleibt erhalten, während der Innendurchmesser (ri) der Innenkontur durch z.B. Ausfräsen des Teilstücks (T1) über eine senkrechte Wand in die Außenkontur übergeführt wird (sh. Fig. 11 g).

Fig. 11d zeigt den verschlossenen Probeentnahmeraum in dem die entnommene Probe liegt. Beim Abtrennvorgang hat sich als vorteilhaft erwiesen, die Schneidhülse 78 über ihre in Fig. 11d gezeigte Endlage in distaler Richtung ca. 2 mm hinaus zu bewegen und danach die Schneidhülse um diese 2 mm in ihre Endlage zurückzuführen. Durch diesen Schereneffekt werden eventuell noch nicht durchtrennte Fasern zuverlässig abgetrennt, was zu einer weiteren Verbesserung der Probenqualität führt.

Die hier beschriebene Biopsienadel arbeitet mit im Innenraum angelegtem Vakuum. Die kombinierte Vorwärts-/Rückwärtsbewegung mit der überlagerten, begrenzten Winkeldrehbewegung der Biopsienadel und damit der Schneiden des Probeentnahmeraums, führt auch bei Biopsienadeln ohne Vakuum oder bei Ausfall des Vakuums zu hervorragenden Ergebnissen, insbesondere dann, wenn z.B. anstatt des Innenvakuums in der Biopsiehohlnadel ein Außendruck auf das Gewebe z.B. mittels Ultraschall angelegt wird. Aber auch die alleinige Anwendung der Winkeldrehbewegung, überlagert mit der Vorwärts-/Rückwärtsbewegung der Nadel in Verbindung mit den als Schneiden ausgebildeten Längskanten des Probeentnahmeraums, verbessert erheblich den Gewebeauftrennvorgang und erleichtert das Eindringen der herauszuschneidenden Gewebeprobe in den Probeentnahmeraum.

Nachzutragen wäre auch, dass nicht in jedem Fall das elastische Dichtelement als Rückstellelement nötig ist, die Rückstellung kann auch allein durch die Umkehrung der Drehrichtung erfolgen.

Fig. 13 zeigt den Antrieb und den Einbau der Vakuum-/Druckerzeugungsvorrichtung 5 (Blick von hinten, also entgegen der Z-Achse, Gehäusedeckel und Gehäuseunterteil sind weggelassen).

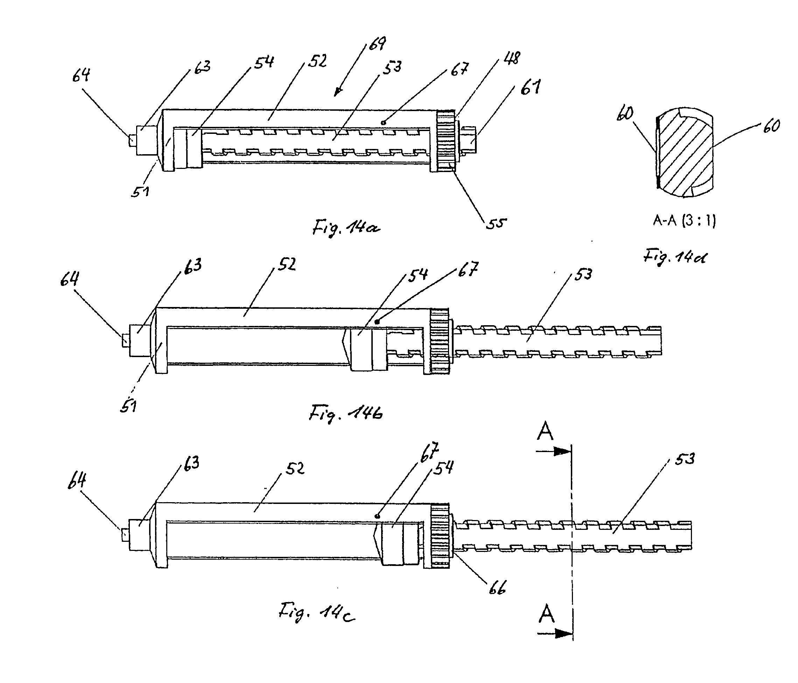

Im oberen, hinteren, rechten Bereich ist die Vakuum-/Druckerzeugungsvorrichtung 5 als Kolben-/Zylindereinheit 69 angeordnet. Sie besteht aus einem Spritzenkörper 52 mit darin angeordneter Gewindespindel 53, an deren dem Spritzenboden 51 zugewandten Ende ein Kolben 54 mit Dichtelementen - wie bei Spritzen allgemein bekannt - befestigt ist (Fig.14a - 14d).

An dem dem Basisblock 8 zugewandten Ende des Spritzenkörpers 52 ist auf der Gewindespindel eine Gewindespindelmutter 48 mit am Umfang ausgebildetem Zahnrad 55 angeordnet. Die Gewindespindelmutter hat eine oder mehrere Gewindegänge. Die Gewindespindel 53 wirkt mit der Gewindespindelmutter 48 zusammen. Die Spindel weist eine Steigung von ca. 5 mm pro Gang auf, so dass bei jeder Umdrehung der Kolben mittels des Spindelantriebs um einen genau definierten Betrag aus dem Spritzenkörper heraus, also vom Spritzenboden 51 weg, oder zum Spritzenboden hin, je nach Drehrichtung, bewegt wird. Der am Umfang der Gewindespindelmutter angeordnete Zahnkranz 55 kämmt mit dem Antriebsritzel 56, das auf der Abtriebswelle des Gleichstromgetriebemotors 58, befestigt ist. Die Abtriebswelle des Gleichstromgetriebemotors 58 ist im Basisblock 8 gelagert; hierfür ist die Abtriebswelle in die Querplatte 59 des Basisblocks eingesteckt. Wird der Gleichstromgetriebemotor 58 aktiviert, so wird der Kolben je nach Drehrichtung zum Spritzenboden oder in Richtung Basisblock 8 hin bewegt. Als Antriebsmotor wird ebenfalls ein Gleichstrommotor mit hoher Drehzahl verwendet, dem ein Planetengetriebe mit hoher Untersetzung nachgeschaltet ist. Er entspricht dem bereits beschriebenen Motor für die Spanneinrichtung. Auf dem Gleichstromgetriebemotor ist daher ebenfalls auf der distalen Seite eine Zähleinrichtung befestigt, die aus einem zweiarmigen Flügelrad 131 und einer motorseitig montierten Fotozelle besteht. Die Zähleinrichtung ist mit dem programmierbaren Mikroprozessor verbunden, so dass die Funktion der Vakuum/Druckeinrichtung über die Umdrehungszahl steuerbar ist, indem nach Ermittlung eines Ausgangswertes durch programmierbare oder programmierte Vorgaben die Funktionen abrufbar sind.

Der Kolben 54 ist in bekannter Weise als Spritzenkolben ausgebildet. Der aus Kunststoff gefertigte Spritzenkörper, ein Zylinder mit Boden, ist durchsichtig.

Um ein Verdrehen der Gewindespindel 53 beim Antrieb der Gewindespindelmutter zu verhindern, sind die beiden gegenüber liegenden Flächen 60 der Gewindespindel flächig ausgebildet (Fig. 14d). Die Gewindespindel wird mit dem freien Ende in das Einlegeelement eingelegt. Der Abstand der Flächen der Gewindespindel entspricht der Breite des U-förmigen Einlegeelements 62 des Basisblocks 8. Zwischen U-förmigem Querschnitt des Einlegeelements und den beidseitigen Spindelflächen besteht nur ein geringes Spiel. Die Gewindespindelmutter stützt sich am Basisblock ab.

Um ein Herausgleiten des Spritzenkörpers 52 beim Verdrehen der Gewindespindelmutter zu verhindern, ist die Anlagefläche am Basisblock 8 leicht konisch nach unten ausgebildet.

Der Stutzen 63 des Spritzenkörpers 52 ist in die Durchführung 16 des Gehäuseenddeckels 7 so eingelegt, dass der Spritzenkörper in etwa in waagrechter Lage gehalten wird.

Um das Verdrehen der Gewindespindel leichtgängig zu machen, weist die Gewindespindelmutter mit Zahnkranz auf der dem Basisblock zugewandten Seite eine etwa 1,5 mm starke Anphasung 66 auf. Da darüber hinaus die Fläche der Rippe 59 am Basisblock 8, die mit der Anphasung 66 der Gewindespindelmutter 48 zusammenwirkt, von oben nach unten geneigt ist, wird die Vakuum-/Druckerzeugungsvorrichtung bei Betrieb nach unten gezogen. Zur Erzeugung eines ausreichenden Vakuums von ca. 200 hph im Probeentnahmeraum wird z.B. bei einer Biopsienadellänge von ca. 250 mm und einem Innendurchmesser der Biopsiehohlnadel zwischen 3 und 5 mm ein Spritzenkörper für 20 ml mit einer Länge von ca. 90 mm verwendet. Um den Spritzenkörper auch als Druckerzeuger verwenden zu können ist nach etwa ¾ der Länge, entsprechend dem Hub für das Erzeugen des Vakuums (Stellung gemäß Fig. 11 b) eine Belüftungsbohrung 67 von ca. 1,5 mm Durchmesser vorgesehen. Die Belüftungsbohrung kann auch als Langloch ausgebildet sein. Wird der Spritzenkolben über die Belüftungsbohrung 67 hinaus bewegt (Fig. 14c), - wenn das Vakuum nicht mehr benötigt wird - wird durch Luftzufuhr (Atmosphärendruck) über die Belüftungsbohrung 67 das vorher aufgebaute Vakuum in der Biopsiehohinadel abgebaut. Wird danach die Drehrichtung des Getriebemotors umgekehrt, so wird durch Hineinfahren des Kolbens (zum Spritzenboden hin) die Vakuum-/Druckerzeugungsvorrichtung im System einen Überdruck aufbauen, was nach Öffnen des Probeentnahmeraums den Auswurf der Gewebeprobe bewirkt. Um zu verhindern, dass bei der kurzzeitigen Öffnung der Belüftungsbohrung 67 evtl. Gewebeflüssigkeit austritt, kann die Belüftungsbohrung bspw. mit einem luftdurchlässigen Schwämmchen (nicht gezeichnet) abgedeckt werden. Eine Verzögerungsschaltung, die in die Steuerung integriert ist, verhindert, dass der Auswurf der Gewebeprobe versehentlich durch Antippen der Programmtaste 89 erfolgt, da diese Verzögerungsschaltung erst nach etwa 1,2 - 1,5 sec. Drücken den Vorgang auslöst. Der Auswurf der Probe darf erst nach Entnahme der Biopsienadel aus dem Gewebe erfolgen, was so sichergestellt ist. Im Übrigen wird durch die Druckluft nicht nur der Probeentnahmeraum, sondern insbesondere auch der Innenraum der Biopsienadel gereinigt. Durch den den Nadelhohlraum einengenden Stopfen wird das Eindringen von Gewebeteilen in den Biopsienadelhohlraum erschwert, bzw. ganz verhindert. Durch die Einengung des Nadelhohlraums durch den Stopfen 79 wird der Druck am Probeentnahmeraum erhöht und dadurch der Auswurf der Probe gerade bei halb geöffnetem Probeentnahmeraum verbessert. Bei Einsatz des Vakuum-Biopsiegerätes empfiehlt es sich, eine speziell ausgebildete Koaxialkanüle zu verwenden, die auf die Belange und Bedürfnisse des Gerätes abgestimmt ist. Sie muss entsprechende Einrichtungen enthalten, die einen Lufteintritt verhindern und das Austreten von Gewebeflüssigkeit behindern, bzw. ausschließen; zum anderen soll sie leicht in das Gewebe einsetzbar sein.

Nachfolgend wird die Bedienung der Biopsieeinrichtung näher erläutert:

Es lassen sich folgende Abschnitte vom Ablauf her unterscheiden, die nach ihrer jeweiligen Einleitung weitgehend automatisch ablaufen:
a) Starten und Einstellen der Grundposition
b) Spannen der Biopsienadel und Einschießen in das Gewebe
c) Herausschneiden der Probe aus dem Gewebe (Probeentnahme)
d) Entnehmen der Probe nachdem die geschlossene Biopsienadel aus dem Gewebe entnommen wurde.

### a) Starten und Einstellen der Grundposition

Das herausnehmbare Einlegeelement 20, bestehend aus Vakuum-/Druckerzeugungsvorrichtung, elastischem Verbindungselement sowie Biopsienadelträger mit Nadel und Schneidhülse und weiteren damit verbundenen Elementen, sowie eine auf die Nadel aufgesetzte Führungsrolle 81, wird steril verpackt angeliefert. Die herausnehmbaren Elemente (s. Fig. 2) werden von einer Einlegehilfe gehalten, die nach dem Einlegen in das Handstück entfernt wird. Diese Einlegehilfe 104 weist zwei Haltestücke zum Anfassen an der Oberseite sowie Laschen 108 zur Halterung des Biopsienadelträgers 37 und der Vakuum-/Druckerzeugungsvorrichtung auf. Zur Fixierung der Lage der Vakuum-/Druckerzeugungsvorrichtung (parallel zum Biopsienadelträger) ist zu der Laschenhalterung ein Stift 110 vorgesehen, der in die Belüftungsbohrung eingesteckt ist.

Der Kolben 54 im Spritzenkörper 52 ist bei Lieferung geringfügig (1-2 mm) vom Spritzenboden abgehoben, der Probeentnahmeraum 71 der Biopsienadel 2 ist geöffnet um so vor dem Einlegen eine visuelle Überprüfung des Probeentnahmeraums vornehmen zu können. Nach dem Öffnen des Gehäusedeckels 10 wird der Biopsienadelträger, einschließlich Biopsienadel 2, Schneideinrichtung 3 und anderer damit verbundener Teile, wie die am Verbindungselement 4 angeschlossene Vakuum-/Druckerzeugungsvorrichtung 5 in die am Handstück vorgesehenen Verbindungselemente eingelegt (s. Fig. 2). Beim Einlegevorgang ist darauf zu achten, dass das Zahnrad 74 in die Zähne der Zahnwalze 23 eingreift; die Schneidhülse wird von oben in die U-förmige Halterung 36 eingelegt, gleichzeitig werden die Laschen 40 des Spannschlittens in die Ausnehmungen 77 des Trägerelements eingeführt; die Führungsrolle 81 wird in die Durchführung 13 eingelegt, so dass sie die Flanken 101 und 102 den Gehäuseenddeckel 6 umfassen. Die Schneidhülse ist in der Führungsrolle längs verschieblich und frei drehbar gelagert; die Führungsrolle selbst ist jedoch gegenüber der Schneidhülse nach dem Einlegen in den Gehäuseenddeckel nicht mehr verschieblich. Die Vakuum-/Druckerzeugungsvorrichtung wird anschließend einerseits in das nach oben offene Einlegeelement 62 des Basisblocks 8 mit dem freien Ende 61 und andererseits in die U-förmige, nach oben offene Durchführung 16 mit dem Stutzen 63 eingelegt. Der Stutzen 63 liegt oberhalb des Schaltstifts 19. Da das basisblockseitige Einlegeelement eine lichte Breite aufweist, die gerade das Einlegen der beidseitig mit Flächen 60 versehenen Gewindespindel zulässt, ist die Gewindespindel im Einlegeelement drehsicher gehalten. Der Zahnkranz 55 der Gewindespindelmutter 48 greift nach dem Einlegen in das Abtriebsritzel 56 des Getriebemotors ein. Der Abstand zwischen dem Basisblock einerseits und dem Gehäuseenddeckel 7 andererseits ist so gehalten, dass der Spritzenkörper 52 mit auf dem Spritzenkörper aufgesetzter Gewindespindelmutter 48 gerade Platz findet. Die Einheit Spritzenkörper und aufgesetztes Zahnrad ist dadurch so gehalten, dass sie nicht axial verschiebbar ist. Nach dem Einlegen liegt die Vakuum-/Druckerzeugungsvorrichtung parallel zum Biopsienadelträger; das Verbindungselement 4 beschreibt einen Bogen von ca. 180° Nachzutragen wäre noch, dass das Einlegen bei nicht gespanntem Spannschlitten erfolgt; dies bedeutet, dass das Zahnrad 74 bei geöffnetem Probeentnahmeraum am rechten Ende der Zahnwalze eingreift (Fig. 3). Nach dem korrekten Einlegen kann der Gehäusedeckel geschlossen werden.

Um den Einlegevorgang zu erleichtern, kann die beschriebene Einlegehilfe benutzt werden. Das Einlegen kann jedoch auch ohne Einlegehilfe erfolgen. Beim Schließen des Gehäusedeckels wird der Stutzen 63 nach unten gedrückt und dabei über den im Gehäuseenddeckel eingebauten Schaltstift 19, der Mikroschalter betätigt. Dadurch wird das elektrische System aktiviert, was durch Blinken der Resetdiode (gelb) 91 an der Vorderfront des Handstückes angezeigt wird. Die Resetdiode blinkt gelb, was bedeutet, dass die Positionierung der einzelnen Elemente, d.h. der Einlegevorgang, noch nicht beendet ist; der Gleichstromgetriebemotor 21 muss zunächst den Probeentnahmeraum 71 mit der Schneidhülse 3 verschließen (der Probeentnahmeraum war beim Einlegen teilweise geöffnet). Dies geschieht, durch Verdrehen der mit der Schneidhülse verbundenen Gewindehülse. Die Schneidhülse wandert nach links bis das Zahnrad 74 nahe der Innenseite der Halterung 36 zur Anlage kommt. Die Kunststoffscheibe 78 liegt nach dem Schließen des Probeentnahmeraums an der Halterung 36 (Innenseite) an. Der Gleichstromgetriebemotor 58 bringt während dieses Vorgangs oder vorher oder danach den Spritzenkolben 54 in Anlage mit dem Spritzenboden 51. In dieser Phase werden auch die Zähler des Mikroprozessors für die Bewegung der Biopsienadel/Schneidhülseeinheit und der Vakuum-/Druckerzeugungsvorrichtung auf Null gestellt. Von dieser Grundeinstellung aus erfolgen über die an den beiden Motoren angeordnete Zähleinrichtung die programmierten Bewegungsabläufe. Nachdem die Ausgangspositionen für die Vakuum-/Druckerzeugungsvorrichtung und die Biopsienadel/Schneidhülseeinheit, erreicht sind, leuchten die Spanndiode 94 (gelb) und die Probeentnahmediode 92 (grün) auf, die Resetdiode erlischt.

### b) das Spannen der Biopsienadel und das Einschießen der Biopsienadel in das Gewebe

Der Bediener muss sich in dieser Phase entscheiden, ob er das Spannen des Spannschlittens einleiten, oder eine weitere Probe entnehmen möchte, z.B. nachdem bereits eine Gewebeprobe entnommen wurde. Im Falle der Entnahme einer ersten Gewebeprobe wird der Bediener die Spanntaste 90 drücken. Damit wird das Spannen des Spannschlittens eingeleitet; die Spanndiode blinkt gelb, die Probeentnahmediode (grün) 92 erlischt. Durch Drücken der Spanntaste (wegen der Verzögerungsschaltung ist die Taste ca. 1,2 - 1,5 Sekunden zu drücken) erhält der elektrische Gleichstromgetriebemotor 21 Strom und der Gleichstromgetriebemotor treibt die Zahnwalze 23 an. Das mit der Zahnwalze 23 kämmende Zahnrad 74 dreht die Spindelwelle und gleichzeitig die damit verbundene Schneidhülse 3. Da die Spindelmutter 75 im Biopsienadelträger 37 eingepresst ist und das Zahnrad 74 sich über die Kunststoffscheibe 78 an der Halterung 36 abstützt, die über den Basisblock 8 mit dem Gehäuse fest verbunden ist, bewirkt das Drehen der Gewindespindelhülse 73, dass der Biopsienadelträger nach rechts bewegt wird. Gleichzeitig wird die mit dem Biopsienadelträger über das Lagerelement 49 verbundene Biopsienadel 2 mitgenommen, was dazu führt, dass die Biopsienadelspitze in die Schneidhülse hinein wandert. Der Biopsienadelträger 37 wird über die Ausnehmung/Laschenverbindung des Spannschlittens gegen die Wirkung der Spiralfeder 31 nach rechts verschoben bis der Hebel 33 des Verrastelementes in die Ausnehmung 82 des Spannschlittens durch die Feder 34 eingedrückt wird. Der Spannschlitten ist in dieser Position verriegelt. Der Getriebemotor erhält den Steuerbefehl dass die Verriegelungsposition erreicht ist, z.B. über eine in die Gleitfläche der Abdeckplatte eingelassene Fotozelle, die mit dem zurückgefahrenen Biopsienadelträger zusammenwirkt; oder über den Mikroprozessor der einerseits die tatsächliche Umdrehungszahl mit der eingegebenen Sollzahl vergleicht, die vorher einprogrammiert wurde; die Drehrichtung des Motors wird nach Erreichen des Sollwerts umgekehrt und die Schneidhülse wird um den Betrag nach rechts zurückgedreht, um den die Schneidhülse durch Verschieben des Spannschlittens und die Biopsienadel über die Biopsienadelspitze hinaus gewandert war. Am Ende dieses Schrittes verschließt die Schneidhülse den Probeentnahmeraum vollständig (Fig. 11 d), wie zu Beginn des Spannvorgangs. Die Verriegelungsdiode 95 leuchtet grün; das Blinken der Spanndiode 94 erlischt. Damit beim Spannvorgang die Reibkraft zwischen Zahnrad und Abstützelement verringert wird, ist z.B. eine zusätzliche Kunststoffscheibe 78 zwischen Zahnrad 74 und Halterung 36 angeordnet.

Nun wird die Biopsienadel der Biopsieeinrichtung z.B. in eine vorher gesetzte Koaxialkanüle eingesetzt. Das proximale Ende der gesetzten Koaxialkanüle enthält eine Dichtung, die so bemessen ist, dass sie den Raum zwischen Schneidhülse und Kanüle einerseits abdichtet, andererseits ein leichtes Einschieben der Biopsienadel mit Schneidhülse zuläßt. Durch den Dichtring wird verhindert, dass Luft von außen über den Raum zwischen Kanüle und Schneidhülse eingesaugt wird. Ebenso verhindert der Dichtring ein Austreten von Flüssigkeit (zytologischem Material) nach Einführung bzw. Einschießen der Biopsienadel. So wird die Möglichkeit einer Verschmutzung des desinfizierten Handstücks nahezu vermieden; andererseits verhindert die Flanke 101 der sterilen Führungsrolle 81, dass eine Verschmutzung des Handstücks von der Kanüle aus stattfindet. Die Biopsienadelspitze wird durch Entnahme des Doms in der Koaxialkanüle an die Geschwulst herangeführt und nach korrekter Positionierung in die Geschwulst eingeschossen.

Der Schuss wird durch Drücken der Betätigungstaste 88 ausgelöst. Das Drücken hat zur Folge, dass durch Verschwenken des doppelarmigen Hebels 33 um die Achse 35 der Spannschlitten freigegeben wird. Der Spannschlitten wird durch Federwirkung nach links geschleudert. Dem Mikroprozessor wird die Auslösung des Schusses und die neue Position der Nadel durch z.B. eine integrierte Fotozelle mitgeteilt. Die Probeentnahmediode leuchtet grün auf, die Spanndiode leuchtet gelb.

### c) Das Herausschneiden der Probe aus dem Gewebe

Durch erneute Betätigung der Programmtaste 89 wird der Ablauf für die Probeentnahme freigegeben; die Probeentnahmediode 92 blinkt grün. Zunächst wird der Gleichstromgetriebemotor 58 der Vakuum-/Druckerzeugungsvorrichtung aktiviert. Der Kolben der Vakuum-/Druckerzeugungsvorrichtung wird in Richtung Basisblock , also vom Spritzenboden weg bewegt, bis er eine Stellung kurz vor Freigabe der Belüftungsbohrung 67 erreicht (Fig. 14b). Das Vakuum im System ist erzeugt. Nach Erreichen seiner Endstellung aktiviert das System den Motor 21, die Schneidehülse, die den Probeentnahmeraum verschließt, wird über den Zahnrad-/Spindelantrieb geöffnet. Während des Öffnungsvorganges soll durch den im System herrschenden Unterdruck oder einen von außen aufgebrachten Druck das Gewebe und eventuelle zytologische Flüssigkeit (zytologisches Material) in den Probeentnahmeraum eingesaugt oder gedrückt oder gedrückt werden. Zytologische Flüssigkeit wird u.a. durch das Vakuum in den Biopsienadelhohlraum und in die Vakuum-/Druckerzeugungsvorrichtung fließen. Es hat sich als vorteilhaft erwiesen, dass durch den Stopfen (79) der Unterdruck vor allem auf den unteren Bereich, die untere Seite, des Probeentnahmeraums gelenkt wird und durch den Stopfen 79 ein Eindringen von Gewebe in die Biopsiehohlnadel erschwert, bzw. verhindert wird. Nach vollständigem Öffnen des Probeentnahmeraums oder während der Öffnung, wird die Biopsienadel kurzzeitig, etwa 5 mal, im Bereich von ca. 2 mm, vor- und zurück bewegt. Mit diesem Bewegungsvorgang, also gleichzeitig, ist in in einer bevorzugten Ausführungsform eine Winkeldrehbewegung der Biopsienadel und damit des Probeentnahmeraums um die Längsachse verbunden. Dies erfolgt dadurch, dass über den Antriebsmotor 21, bei vollständig geöffnetem Probeentnahmeraum der Antriebsmotor vom Mikroprozessor den Befehl erhält, den Probeentnahmeraum weiter zu öffnen; dies ist jedoch nicht möglich, da der Bund 127 ein weiteres Verschieben der Schneidhülse nach rechts verhindert.. Über die Verbindung

Gewindespindel/Gewindespindelmutter und das Biopsienadel-trägerelement wird der Spannschlitten zur distalen Seite um ca. 2 mm verschoben und dabei die kurze Spiralfeder zusammengedrückt. Durch die Mikroprozessorsteuerung wird nach einer vorherbestimmten Umdrehungszahl, die dem Weg von 2 mm entspricht, die Drehrichtung des Antriebsmotors umgesteuert. Der Spannschlitten wird von der Spiralfeder und dem Motor in seine Ausgangslage zurückgebracht. Danach wird erneut der Antriebsmotor umgesteuert und der Spannschlitten erneut gegen die Wirkung der kurzen Spiralfeder gezogen; nach Erreichen des Spannweges erfolgt die Umsteuerung und so weiter. Diese Vorwärts-/Rückwärtsbewegung und die damit verbundene Winkeldrehung der Biopsienadel führen dazu, dass das Gewebe durch die Längskanten des Probeentnahmeraums aufgetrennt werden und somit das Einlegen der Probe in den Probeentnahmeraum auch bei schwierigem Gewebe gesichert ist. Der Vorgang kann beliebig oft wiederholt werden, je nach Programmierung. Im Allgemeinen genügen 5 Zyklen um durch diese Bewegungssteuerung die geschärften Längsseiten des Probeentnahmeraums dazu zu benutzen, auch bei harten Gewebeproben oder Gewebe mit Einschlüssen durch Auftrennen des seitlich liegenden Gewebes die Gewebeprobe in den Probeentnahmeraum leicht und vollständig, z.B. mittels Vakuum einzulegen ist.

An dieser Stelle sei nochmals betont, dass der Gewebeabtrennvorgang im einfachsten Fall auch durch ausschließliches Vor- und Zurückbewegen der Biopsienadel durchgeführt werden kann, insbesondere wenn ein Aussendruck bspw. mittels Ultraschall aufgebracht wird. Die zusätzliche Rotationsbewegung um die Nadellängsachse kann optional durch entsprechende Vorsehung der hierfür erforderlichen, vorstehend erläuterten Massnahmen vorgenommen werden, die den Gewebeabtrennvorgang vorteilhaft unterstützt.

Nach der vorstehend als vorteilhaft beschriebenen kombinierten Bewegung der Biopsienadel wird der Getriebemotor 21 umgesteuert und der Probeentnahmeraum 39 wird durch Drehen der Schneidhülse geschlossen, wobei die Schneidkante 72 der Schneidhülse 3 beim Schließvorgang das Gewebe abtrennt. Es ist selbstverständlich, dass durch entsprechende konstruktive Abänderung oder entsprechende Steuerung sowie zusätzliche Elemente die Vorwärts-/Rückwärtsbewegung, bzw. Winkeldrehbewegung der Biopsienadel zum Auftrennen der seitlichen Probekanten auch während des Öffnens der Schneidhülse erfolgen kann. Beim Schließvorgang wird die Schneidhülse über ihre Verschließposition hinaus, ca. 2 mm, in Richtung zur Nadelspitze hin zusätzlich vorgefahren. Dadurch werden die Gewebefasern mit Sicherheit durchtrennt. Danach wird die Schneidhülse um 2 mm in die Schließposition zurückgefahren.

Die Steuerung der Vorgänge erfolgt durch den Mikroprozessor, in dem die Sollwerte abgelegt sind und die der Mikroprozessor mit den Messdaten (Zähldaten) vergleicht und so die Vorgänge steuert. Durch die spezielle Gestaltung des Probeentnahmeraums und auch infolge eines evtl. angelegten Vakuums ist die Gewebeprobe drehgesichert im Probeentnahmeraum gehalten, so dass durch die die Biopsienadel außen umgebende, drehend längsverschiebliche Schneidhülse 3 die Gewebeprobe, wie beschrieben, nicht verdreht oder verdrillt wird. Nachdem der Probeentnahmeraum geschlossen ist, wird der Gleichstromgetriebemotor für die Vakuumerzeugungseinheit 5 aktiviert. Der Kolben 54 wird zunächst soweit zurückgefahren bis der Kolben die Belüftungsbohrung freigibt (Fig. 11 c). Nach Abbau des Vakuums im System fährt der Kolben soweit zum Spritzenboden vor, dass die Belüftungsbohrung wieder verschlossen wird, um den Ausfluss von Körperflüssigkeit (zytologische Flüssigkeit) zu verhindern. Diese kurzzeitige Öffnung der Belüftungsbohrung liegt im Bereich von Bruchteilen einer Sekunde um möglichst zu vermeiden, dass Flüssigkeit in das Handstück austreten kann. Um zu verhindern, dass Flüssigkeit über die Belüftungsbohrung oder die Belüftungsbohrungen in das Handstück gelangt, können aus Sicherheitsgründen die Belüftungsbohrungen zusätzlich mit luftdurchlässigem Material abgedeckt sein, so dass der Innenraum des Handstücks nicht verschmutzt wird. Das Blinken der Probeentnahmediode 92 erlischt. Die Auswurfdiode 93 leuchtet gelb. Die Biopsienadel mit geschlossenem Proberaum wird aus der Kanüle gezogen.

### d) Das Entnehmen der Probe nachdem die Biopsienadel aus dem Gewebe entnommen wurde

Nach der Entnahme der Biopsienadel aus dem Gewebe und der Bereitstellung eines Gefäßes für die Aufnahme der Gewebeprobe und Gewebeflüssigkeit, wird die Programmtaste 89 erneut betätigt und die Auswurfdiode 93 beginnt zu blinken. Wegen der Verzögerungsschaltung, aus Sicherheitsgründen, muss die Programmtaste ca. 1,2 - 1,5 Sekunden gedrückt werden bevor der Ablauf eingeleitet wird. Zunächst wird der Getriebemotor 21 der Schneidhülse betätigt um den Probeentnahmeraum etwa bis zur Hälfte zu öffnen. Danach wird der Gleichstromgetriebemotor 58 der Vakuum-/Druckerzeugungsvorrichtung aktiviert. Die Drehrichtung des Gleichstromgetriebemotors 58 bleibt und die Gewindespindel 53 mit Kolben bewegt sich in Richtung Spritzenboden, so dass im System nun ein Überdruck entsteht. Der Kolben wird bis zum Kolbenboden vorgefahren, der Antriebsmotor 58 wird deaktiviert. Der Getriebemotor 21 fährt die Schneidhülse über dem Probeentnahmeraum weiter zurück, nachdem der Kolben den Kolbenboden erreicht hat. Infolge des im System aufgebauten Überdrucks wird die Probe unter Druck schon bei halb geöffnetem Probeentnahmeraum in ein bereitstehendes Laborgefäß herausgedrückt, gleichzeitig wird der Hohlraum Vakuum-/Druckerzeugungsvorrichtung, der Biopsienadel und der Probeentnahmeraum von Gewebepartikeln und Flüssigkeit befreit. Der Auswurf der Probe bei etwa halb geöffnetem Probeentnahmeraum erfolgt deshalb, weil dadurch der Auswurf der Gewebeprobe sichergestellt wird und nicht durch vorzeitigen Abbau des Überdrucks die Gewebeprobe in den Probeentnahmeraum zurückfällt. Die Einengung des Biopsienadelhohlraums durch den Stopfen 79, der ein Eindringen von Gewebe in den Biopsienadelhohlraum erschwert, bzw. verhindert hat, erweist sich bei der Probeentnahme als besonders vorteilhaft, da der verengte Querschnitt den Auswurfdruck erhöht. Die besten Auswurfergebnisse wurden bei halb geöffnetem Probeentnahmeraum erzielt; d.h. die Schneidhülse gibt die Hälfte der axialen Länge des Probeentnahmeraums frei. Durch den Überdruck wird auch die Gewebeflüssigkeit aus dem Probeentnahmeraum gedrückt und dieser gereinigt.

Nachdem der Probeentnahmeraum vollständig geöffnet ist, ist die Entnahme und Reinigung abgeschlossen, die Auswurfdiode erlischt. Die Resetdiode 91 leuchtet gelb. Sofern nun keine weitere Probe entnommen werden soll, wird der Gehäusedeckel geöffnet und das herausnehmbare Element 20 entfernt. Beim Öffnen des Gehäusedeckels 10 wird das System über den Mikroschalter 18 deaktiviert. Soll jedoch eine weitere Probe aus der gleichen Gewebeumgebung entnommen werden, so drückt der Bediener die Programmtaste 89 und die Resetdiode 91 beginnt zu blinken. Die Grundeinstellung der Vakuum-/Druckerzeugungsvorrichtung, wie der Schneidhülse findet, wie beschrieben, erneut statt. Nach Abschluss des Vorgangs erlischt die Resetdiode 91 und die Probeentnahmediode (grün) und die Spanndiode (gelb) leuchten auf. Es liegt nun wieder am Bediener, ob er lediglich eine weitere Gewebeprobe aus dem gleichen Einschussloch herausschneiden möchte- in diesem Fall drückt er die Programmtaste 89 - oder ob er einen weiteren Einschuß durch Spannen der Biopsienadel wünscht - in diesem Fall drückt er die Spanntaste 90. Je nach Wahl laufen die weiteren Prozessschritte in der bereits beschriebenen Reihenfolge ab. Der Vorgang kann beliebig oft wiederholt werden. Der Bediener muss nach Auswurf der Probe lediglich entscheiden, ob er eine weitere Probe entnehmen will oder die Probeentnahme abgeschlossen ist und der Gehäusedeckel geöffnet wird.

Sollte es erforderlich sein, dass die Probe an einer Stelle der Geschwulst entnommen wird, die nach dem Einschuss nicht direkt über oder am Probeentnahmeraum liegt, also z.B. seitlich davon, so kann die Lage des Probeentnahmeraumes 71 über die Rändelschraube 80 verdreht werden. Damit der Bediener diese radiale Stellung des Probeentnahmeraums erkennen kann, ist auf der Rändelscheibe eine Markierung in Form einer Kerbe 119 angebracht, die nach oben zeigt, wenn die Öffnung des Probeentnahmeraums nach oben zeigt. In der jeweils eingestellten Position wird die Biopsienadel durch die Flächen des Vielkants 50 und die elastischen Kräfte im Trägerteil fixiert. Der Vorgang der Probeentnahme ist der gleiche wie bereits beschrieben.

Nach Beendigung der Biopsie wird nach Entrastung des Deckels das austauschbare Element 20 (Vakuum-/Druckvorrichtung, Biopsienadel/Schneidvorrichtung mit allen daran angeordneten Elementen) nach oben entnommen. Um ein Öffnen des Gehäuses bei gespanntem Spannschlitten unmöglich zu machen, ist an den Biopsienadelträger ein Sicherungsflügel 84 angeordnet, der im gespannten Zustand an der linken Stirnfläche 85 der Verschlusseinrichtung anliegt. Die in der X-Achse verschiebbare Verschlusseinrichtung lässt sich dadurch nicht mehr nach links in Öffnungsstellung bewegen und damit kann die Nase 12 nicht mehr aus der Ausnehmung 45 heraus genommen werden. Umgekehrt lässt sich auch der Gehäusedeckel nicht schließen, sofern das herausnehmbare Element in den bereits vorgespannten Spannschlitten eingelegt wurde, da der Sicherungsflügel verhindert, dass der Riegel in den dafür vorgesehenen Raum eingefügt werden kann. Die Fläche 85 des Riegels stößt am Sicherungsflügel an. Die Batterieladediode 96 ist abgeschaltet, sobald der Gehäusedeckel geöffnet ist. Bei geschlossenem Deckel und eingelegtem Einlegeelement 20 zeigt die Batterieladediode an, ob ausreichend Energie vorhanden ist.

Grundsätzlich ist es denkbar, dass alle Schritte für die Entnahme einer Probe sowie das Spannen des Schlittens usw. durch Aktivierung und Deaktivierung der beiden Getriebemotoren einzeln von Hand gesteuert werden. Es ist aber zweckmäßig, dass einzelne Schritte des Ablaufvorgangs zusammengefasst werden und automatisch ablaufen und nur die Einleitung des Folgeschritts durch Schalterbetätigung in Gang gesetzt wird. Diese halbautomatische Methode, wie vorher beschrieben, hat sich als besonders vorteilhaft erwiesen.

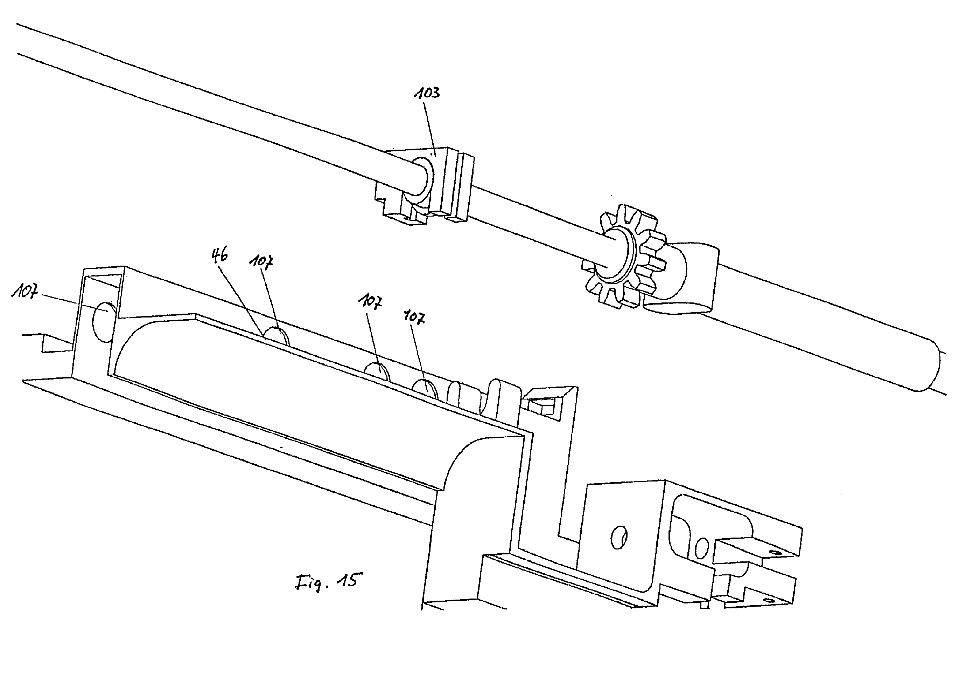

Grundsätzlich sind zwei Methoden zur Erfassung der Ist-Werte für den Vergleich mit den Soll-Werten denkbar. Die eine Methode beruht auf dem Messen der Längenverschiebung der Gewindespindel beim Herausziehen, bzw. Hineindrücken sowie dem Messen der axialen Verschiebung der Schneidhülse, bzw. des Biopsienadelträgers. Um diese Veränderungen zu erfassen sind Fotozellen oder Mikroschalter im Gehäuseinneren, insbesondere auf der Verlängerung des Basisblocks 8, angeordnet. Auf der Schneidhülse ist bei der Messung der Veränderung in Verbindung mit einer Fotozelle zusätzlich ein Positionierungsfinger 103 aufgesetzt, während bei der Gewindespindel der Vakuum-/Druckerzeugungsvorrichtung das aus der Kolbeneinheit herausragende freie Ende 61 als Messpunkt herangezogen werden kann. Sofern die vordere Kante des Biopsienadelträgers als Messpunkt mit einer Fotozelle verwendet wird, bedarf es keines zusätzlichen Positionierungsfingers. Die eingelassenen Fotozellen werden wegen einer eventuellen Verschmutzung mit geeignetem, durchsichtigen Material abgedeckt. Der Positionierungsfinger 103 durchgreift einen Schlitz im Biopsienadelhalter. An entsprechenden Stellen, an der Verlängerung 46 des Basisblocks 8 sind Ausnehmungen 107 vorgesehen, in die Fotozellen, bzw. Mikroschalter eingebaut sind, die entweder mit dem freien Ende 61 der Kolbenspindel, mit dem Positionsfinger oder der Biopsienadelträgerkante 120 zusammenwirken (Fig. 15). Diese Signale (Ist-Wert) werden in der Elektronik verarbeitet und bilden die Steuersignale.

Das andere System beruht auf der Messung der Umdrehungszahl der Gleichstromgetriebemotoren, die in Längeneinheiten umgerechnet wurden, was insbesondere dann zweckmäßig ist, wenn die Änderungen mittels der Getriebemotoren durchgeführt werden. Hierbei wird auf die Welle des Gleichstrommotors ein Geber aufgesetzt, der mit einer auf dem Gehäuse des Gleichstrommotors aufgesetzten Fotozelle zusammenwirkt. Dieser Geber besteht (vgl. Fig. 3) aus einem zweiarmigen Flügelrad 131 und einer mit dem Motor verbundenen Fotozelle. Diese Geber auf den beiden Antriebsmotoren liefern die Zählimpulse für die Fotozellen, die sie an den programmierbaren Mikroprozessor weiterleitet, der diese Erfassungsdaten mit den vorher abgespeicherten Vorgaben abgleicht und dadurch die Steuerimpulse auslöst. Da die Gleichstrommotoren lastabhängig mit einer Drehzahl von ca. 10.000 - 12.000 U/min. arbeiten und andererseits das nachgeschaltete, abtriebsseitig angeordnete Planetengetriebe, das mit dem Spindelantrieb zusammenwirkt, die Umdrehungszahl erheblich reduziert, ist auf diese Weise eine genaue Längssteuerung möglich. Die Längsverschiebung durch die Spindelantriebe ist proportional zur Anzahl der Abtriebsumdrehungen ein stets gleicher Betrag und die Anzahl der Umdrehungen ist daher als Steuersignal für die Genauigkeit der Längsverschiebung ausreichend. Um die Position der Schneidhülse 3 sowie des Kolbens 54 bei Beginn, also nach dem Einlegen des herausnehmbaren Elements und Schließen des Gehäusedeckels 10 genau zu bestimmen, dreht der Gleichstromgetriebemotor 58 den Kolben 54 auf Anschlag auf den Spritzenboden und der Gleichstromgetriebemotor 21 bringt den Schneidhülsenantrieb auf Null-Stellung, indem er das Zahnrad 74 an der Gewindespindelmutter 75 zum Anschlag bringt (Die Gewindespindelmutter 75 läuft auf das Zahnrad 74 auf). Von dieser Null-Position aus wird dann über den Vorgabe-Ist-Vergleich die Steuerung der einzelnen Schritte durchgeführt. Die erforderlichen Kabel vom Messgeber zur Elektronik sind im Gehäuse untergebracht, ebenso die Platine mit den elektronischen Bauteilen (Raum 39). Auch die Kombination der beiden beschriebenen Steuersysteme ist bei Bedarf möglich.

### Bezugszeichenliste

- 1: Handstück
- 2: Biopsienadel
- 3: Schneidhülse
- 4: Verbindungselement
- 5: Vakuum-/Druckerzeugungsvorrichtung
- 6: Gehäuseenddeckel (links)
- 7: Gehäuseenddeckel (rechts)
- 8: Basisblock
- 9: Gehäuseunterteil
- 10: Gehäusedeckel
- 11: Verschlussriegel
- 12: Nase
- 13: Durchführung
- 14: Bohrung
- 15: Durchführung
- 16: Durchführung
- 17: Zapfen
- 18: Mikroschalter
- 19: Schaltstift
- 20: herausnehmbares Element
- 21: Gleichstromgetriebebemotor
- 22: Wand
- 23: Zahnwalze
- 24: U-förmiger Raum
- 25: Wand
- 26: Block
- 27: Nut
- 28: Spannschlitten
- 29: Gewindebohrung
- 30: Bolzen
- 31: Spiralfeder
- 32: Endstück
- 33: Doppelhebel
- 34: Druckfeder
- 35: Achse
- 36: Halterung
- 37: Biopsienadelträger
- 38: Bohrungen
- 39: Schenkel
- 40: Laschen
- 41: Fläche Spannschlitten
- 42: Verlängerung der Fläche
- 43: Gleitfläche
- 44: Fläche des Blocks 26
- 45: Ausnehmung
- 46: Abdeckung
- 47: Kunststoffteil
- 48: Gewindespindelmutter
- 49: Lagerelement
- 50: Vielkant
- 51: Spritzenboden
- 52: Spritzenkörper
- 53: Gewindespindel
- 54: Kolben
- 55: Zahnrad (Zahnkranz)
- 56: Antriebsritzel
- 57: Bedienungspanel
- 58: Gleichstromgetriebemotor
- 59: Querplatte
- 60: Flächen
- 61: freies Ende
- 62: Einlegeelement
- 63: Stutzen
- 64: Ausflussstutzen
- 65: Aussparung
- 66: Anphasung
- 67: Belüftungsbohrung
- 68: Schneide (Längskanten)
- 69: Kolben-/Zylindereinheit
- 70: Nadelspitze
- 71: Probeentnahmeraum
- 72: Schneide
- 73: Gewindespindelhülse
- 74: Zahnrad
- 75: Gewindespindelmutter
- 76: Dichtelement
- 77: Ausnehmungen
- 78: Kunststoffscheibe
- 79: Stopfen
- 80: Rändelscheibe
- 81: Führungsrolle
- 82: Ausnehmung
- 83: Metallteil
- 84: Sicherungsflügel
- 85: Stirnfläche
- 86:
- 87: Mittelrippe
- 88: Betätigungstaste
- 89: Programmtaste
- 90: Spanntaste
- 91: Resetdiode
- 92: Probeentnahmediode
- 93: Auswurfdiode
- 94: Spanndiode
- 95: Verriegelungsdiode
- 96: Batterieladediode
- 97: Durchführung
- 98: Durchführung
- 99: Arm des doppelarmigen Hebels
- 100: Teil des Hebels
- 101: Flanken der Führungsrolle links
- 102: Flanken der Führungsrolle rechts
- 103: Positionsfinger
- 104: Achse
- 105: Antriebsvorrichtung (Vakuum)
- 106: Antriebsvorrichtung (Biopsienadel, Spanneinrichtung)
- 107: Ausnehmungen
- 108: Laschen
- 109: Einlegehilfe
- 110: Stift
- 111: Aku
- 112: Kunststoffteil
- 113: Fläche
- 114: Trennplatte
- 115: Führungsbohrung
- 116: Holm
- 117: Haltestücke
- 118: N.N
- 119: Kerbe
- 120: N.N.
- 121: Beilagscheibe
- 122: Lippe
- 123: Bund
- 124: kurze Spiralfeder
- 125: Beilagscheibe
- 126: Rohrstück
- 127: Bund
- 128: distale Bohrung im Schlitten
- 129: proximalseitige Bohrung im Block 26
- 130: Fotozelle
- 131: Flügelrad
- 0: deformierte Stelle
- S: Schlüsselweite
- A: lichter Abstand
- X: Verschiebung proximal
- X: Verschiebung distal Drehwinkel rechts Drehwinkel links
- H: Höhe des Probeentnahmeraums
- F: Höhe der Lffnung
- T: Teilstück
- r: Außendurchmesser
- r: Innendurchmesser

## Patentansprüche

1. Biopsievorrichtung zur Gewebeentnahme in Art eines Handstückes mit zumindest einer federkraftbeaufschlagbaren Spann- und Abschussvorrichtung in Form eines Spannschlittens (28) für eine Biopsienadeleinheit, die eine äußere Hohlnadel (3) mit einer distalseits angeschärften Schneidklinge sowie eine im Inneren der äußeren Hohlnadel (3) gelagerte, hohle Biopsienadel (2) mit einem an ihrem distalen Endbereich vorgesehenen Gewebeprobeentnahmeraum (71) aufweist, die in einem Biopsienadelträger (37) gelagert ist, der zumindest mit dem Spannschlitten (28) in Wirkverbindung bringbar ist, wobei die distalen Nadelbereiche der äußeren Hohlnadel (3) und der hohlen Biopsienadel (2) zur Gewebeentnahme aus dem Gehäuse hinaus ragen, und, wobei das Handstück ein Gehäuse mit einem Gehäusedeckel (10) aufweist, wobei bei offener Stellung Komponenten in das Gehäuseinnere fest integrierbar sind,
**dadurch gekennzeichnet, dass**
eine Druckquelle (5) vorgesehen ist, die mit der hohlen Biopsienadel (2) verbindbar ist, und dass in das Gehäuseinnere
- wenigstens eine erste und zweite Antriebseinheit (21,58) integriert sind, wobei der
- der Spannschlitten (28) mit der ersten Antriebseinheit (21) derart in Wirkverbindung bringbar ist, dass der Spannschlitten (28) in einen gespannten Zustand überführbar und in diesem verriegelbar ist, und wobei ein an der äußeren Hohlnadel angebrachtes Antriebsmittel (74) mit der ersten Antriebseinheit (21) in Wirkverbindung bringbar ist, durch die die äußere Hohlnadel (3) in Rotation um die Nadellängsrichtung versetzbar ist, wodurch sich die äußere Hohlnadel (3) relativ zur Nadellängsachse der hohlen Biopsienadel (2) verschiebt, und
- die Druckquelle (5), die im proximalen Bereich der hohlen Biopsienadel (2) mit dieser über zumindest eine Verbindungsleitung (4) gasdicht verbunden ist und mit der zweiten Antriebseinheit (58) zur Erzeugung eines Druckniveaus in Wirkverbindung bringbar ist, wobei die Verbindungsleitung (4) wenigstens teilweise innerhalb des Gehäuses verläuft.

2. Biopsievorrichtung nach Anspruch 1, **dadurch gekennzeichnet, dass** die Biopsienadeleinheit in den Biopsienadelträger (37) lösbar fest integriert ist, wobei die innere Biopsienadel (2)proximalseits mit einem Anschlussstück (47) verbunden ist, über das die Verbindungsleitung (4) gasdicht mit der inneren Biopsienadel (2) verbindbar ist und das eine Befestigungskontur (49) aufweist, die in eine innerhalb des Biopsienadelträgers (37) vorgesehene Gegenkontur derart einsetzbar ist, so dass die innere Biopsienadel (2) zumindest in Nadellängsrichtung im Biopsienadelträger festsitzt, und die äußere Hohlnadel (3) in wenigstens einem Teilabschnitt längs ihres Außenumfangs eine Gewindekontur (73) vorsieht, die mit einer zumindest lösbar fest mit dem Biopsienadelträger (37) verbundenen Gegengewindekontur (75) derart in Eingriff steht, dass die äußere Hohlnadel (3) in Nadellängsrichtung relativ zur inneren Biopsienadel (2) verschiebbar ist.

3. Biopsievorrichtung nach Anspruch 2, **dadurch gekennzeichnet, dass** das Anschlussstück (47) ein Rastelement (50) vorsieht, das mit Wirkflächen des Biopsienadelträgers (37) derart in Kontakt steht, dass die innere Biopsienadel (2) um ihre Nadellängsrichtung in vorgebbare Positionen festsitzend verrastbar ist.

4. Biopsievorrichtung nach Anspruch 2, **dadurch gekennzeichnet, dass** zwischen der äußeren Hohlnadel (3) und der hohlen Biopsienadel (2) ein die Reibung zwischen beiden Nadeln erhöhende Mittel vorgesehen ist.

5. Biopsievorrichtung nach Anspruch 4, **dadurch gekennzeichnet, dass** das Anschlussstück (47) ein Rastelement (50) vorsieht, das mit Wirkflächen des Biopsienadelträgers (37) derart in Kontakt steht, dass die innere Biopsienadel (2) um ihre Nadellängsrichtung in vorgebbare Positionen derart verrastbar ist, dass zwischen den Wirkflächen und dem Rastelement ein Spiel vorhanden ist, durch das eine winkelbegrenzte Rotation der hohlen Biopsienadel (2) um ihre Längsachse in beide Rotationsrichtungen möglich ist.

6. Biopsievorrichtung nach Anspruch 1 oder 5, **dadurch gekennzeichnet, dass** durch wechselseitige Rotation der äußeren Hohlnadel (3), angetrieben über das mit der ersten Antriebseinheit (21) in Eingriff stehende Antriebsmittel (74), die hohle Biopsienadel (2) im Wege der zwischen beiden Nadeln (2, 3) herrschenden erhöhten Reibung mitrotiert, wobei die Rotationsbewegung der hohlen Biopsienadel (2) in beiden Rotationsrichtungen durch das spielbehaftete Rastelement (50) begrenzt ist.

7. Biopsievorrichtung nach Anspruch 1, **dadurch gekennzeichnet, dass** an der äußeren Hohlnadel (3) und der hohlen Biopsienadel (2) ein elastisches Dichtelement (76) vorgesehen ist, das beide Nadeln (2, 3) gasdicht zueinander abdichtet.

8. Biopsievorrichtung nach Anspruch 2, **dadurch gekennzeichnet, dass** der Biopsienadelträger (37) eine Kupplungsstruktur (77) aufweist, die in eine an dem Spannschlitten (28) vorgesehene Gegenkupplungsstruktur (40) einsetzbar ist.

9. Biopsievorrichtung nach Anspruch 1, **dadurch gekennzeichnet, dass** die Druckquelle (5) eine Kolben-Zylindereinheit (69) aufweist, durch die in Abhängigkeit der Kolbenbewegung innerhalb der Zylindereinheit ein als Unter- oder Überdruck herrschendes Druckniveau erzeugbar ist.

10. Biopsievorrichtung nach Anspruch 9, **dadurch gekennzeichnet, dass** die Zylindereinheit (52) in Art eines Spritzenkörpers ausgebildet ist und einen Zylinderboden (51) mit einem Anschlussstutzen (63) für eine gasdichte Verbindung zu der Verbindungsleitung (4) sowie eine dem Zylinderboden (51) gegenüberliegende Zylinderöffnung aufweist, dass die Kolbeneinheit eine Gewindespindel (53) vorsieht, an deren einen Ende der Kolben (54) angebracht ist und deren anderes Ende durch eine an der Zylinderöffnung der Zylindereinheit (52) vorgesehene Gewindespindelmutter (48) hindurchragt, die eine zahnradartig ausgebildete Umfangskontur (55) aufweist, die mit der zweiten Antriebseinheit in Eingriff bringbar ist, und dass bei Rotation der Gewindespindelmutter (48) die Gewindespindel (53) samt Kolben (54) relativ zur Zylindereinheit (52) axialverschiebbar ist.

11. Biopsievorrichtung nach Anspruch 9 oder 10, **dadurch gekennzeichnet, dass** die Zylindereinheit (52) im Bereich der Zylinderöffnung innerhalb der Zylinderwand wenigstens eine Belüftungsöffnung (67) vorsieht, so dass in einer Kolbenstellung nahe der Zylinderöffnung der zwischen dem Kolben (54) und dem Zylinderboden (51) eingeschlossene Zylinderraum entlüftbar ist.

12. Biopsievorrichtung nach Anspruch 9, **dadurch gekennzeichnet, dass** die Kolben-Zylindereinheit (69) eine Zylinderlängsachse aufweist, dass die Kolben-Zylindereinheit (69) derart neben der Biopsienadeleinheit im Gehäuseinneren angeordnet ist, dass die Nadellängsachse der Biopsienadeleinheit und die Zylinderachse der Kolben-Zylindereinheit (69) koparallel verlaufen, und dass die Verbindungsleitung (4) einen U-förmige Leitungsverlauf aufweist.

13. Biopsievorrichtung nach einem der Ansprüche 1 bis 12, **dadurch gekennzeichnet, dass** die Verbindungsleitung (4) eine flexible, optisch weitgehend transparente Schlauchleitung ist, die eine Relativbewegung zwischen Druckquelle (5) und hohle Biopsienadel (2) ermöglicht.

14. Biopsievorrichtung nach einem der Ansprüche 1 bis 13, **dadurch gekennzeichnet, dass** die in einen Biopsienadelträger (37) integrierte Biopsienadeleinheit und die über die Verbindungsleitung (4) mit der hohlen Biopsienadel (2) gasdicht verbundene Druckquelle (5) als ein einheitliches in das Gehäuse einsetzbares sowie aus dem Gehäuse wieder entnehmbares Modul (29) ausgebildet sind.

15. Biopsievorrichtung nach Anspruch 14, **dadurch gekennzeichnet, dass** das aus Biopsienadelträger (37), in den die Biopsienadeleinheit integriert ist, Verbindungsleitung (4) sowie Druckquelle (5) bestehende Modul (29) in einer räumlich fest vorgebbaren Anordnung in eine Einlegehilfe (109) lösbar fest einsetzbar ist, vermittels der das Modul (29) in das Gehäuseinnere implementierbar ist.

16. Biopsievorrichtung nach Anspruch 1, **dadurch gekennzeichnet, dass** der Spannschlitten (28) vermittels des Antriebsmittels (74), das mit einer Abtriebswelle der ersten Antriebseinheit (21) in Verbindung steht, sowie der dadurch in Rotation versetzbaren Gewindekontur (73) und die dadurch bewirkbare Axialverschiebung der äußeren Hohlnadel (3) in den gespannten Zustand überführbar ist, wobei sich das Antriebsmittel (74) gegen eine als mechanisches Gegenlager dienende Halterung (36), die Gehäusefest vorgesehen ist, einseitig abstützt, und dass bei Erreichen des gespannten Zustandes der Spannschlitten (28) mechanisch verriegelbar ist.

17. Biopsievorrichtung nach Anspruch 1 oder 16, **dadurch gekennzeichnet, dass** der im gespannten Zustand verriegelte Spannschlitten (28) durch einen Entriegelungsmechanismus auslösbar ist, wodurch der Spannschlitten (28) und mit diesem verbunden der Biopsienadelträger (37) schlagartig in den entspannten Zustand überführbar ist, wobei die Biopsienadeleinheit, d. h. die äußere Hohlnadel (3) und die hohle Biopsienadel (2) gemeinsam distalseits bewegt werden.

18. Biopsievorrichtung nach Anspruch 1 oder 16, **dadurch gekennzeichnet, dass** die erste Antriebseinheit (21) als Abtriebswelle eine Zahnwalze (23) vorsieht, in die das als Zahnrad ausgebildete Antriebsmittel (74) eingreift.

19. Biopsievorrichtung nach Anspruch 1, **dadurch gekennzeichnet, dass** die erste und zweite Antriebseinheit (21,58) als Elektro- Gleichstrommotor mit nachgeschaltetem Planetengetriebe ausgebildet ist.

20. Biopsievorrichtung nach Anspruch 10 oder 19, **dadurch gekennzeichnet, dass** die zweite Antriebseinheit (58) eine Abtriebswelle mit einem Antriebsritzel (56) vorsieht, das in Eingriff mit der zahnradartig ausgebildeten Umfangskontur (55) der Gewindespindelmutter (48) der Kolben-Zylindereinheit (69) bringbar ist.

21. Biopsievorrichtung nach Anspruch 1, **dadurch gekennzeichnet, dass** lösbar fest mit dem Gehäuseinneren verbunden ein Basisblock (8) vorgesehen ist, der Ausnehmungen zur Befestigung der ersten und zweiten Antriebseinheit (21,58) derart vorsieht, so dass deren Abtriebswellen beabstandet voneinander und parallel zueinander verlaufen, dass am Basisblock (8) ein Anschlagelement (26) vorgesehen ist, von dem ein Führungsbolzen (30) ausgeht, längs dem der Spannschlitten (28) federkraftbeaufschlagt führbar ist, und dass der Basisblock (8) Ausnehmungen für das Einlegen der in dem Biopsienadelträger (37) gefassten Biopsienadeleinheit vorsieht sowie ein Positionieren der Druckquelle (5) ermöglicht.

22. Biopsievorrichtung nach 21, **dadurch gekennzeichnet, dass** der Spannschlitten (28) längs des Führungsbolzens (30) beidseitig federkraftbeaufschlagt ist, so dass der Spannschlitten (28) im entspannten Zustand aus einer Ruheposition zu beiden Seiten längs des Führungsbolzen (30) auslenkbar ist.

23. Biopsievorrichtung nach Anspruch 1, **dadurch gekennzeichnet, dass** im Gehäuseinneren eine Energieversorgungseinheit (11) in Form einer Batterie oder eines wiederaufladbaren Akkus vorgesehen ist.

24. Biopsievorrichtung nach Anspruch 23, **dadurch gekennzeichnet, dass** die Energieversorgungseinheit durch eine Trennplatte (114) von dem Bereich des Spannschlittens (28) sowie der Druckquelle (5) getrennt ist.

25. Biopsievorrichtung nach Anspruch 1, **dadurch gekennzeichnet, dass** im Gehäuse eine Steuerungseinheit zur Ansteuerung der ersten und zweiten Antriebseinheit (21,58) vorgesehen ist, und dass die Steuerungseinheit sowie ein Lösen der Verriegelung des Spannschlittens (28) über ein an einer Gehäuseaußenwand angebrachtes Bedienpanel (57) betätigbar ist.

26. Biopsievorrichtung nach Anspruch 25, **dadurch gekennzeichnet, dass** die Steuerungseinheit zur Ansteuerung der als Elektro-Gleichstrommotoren ausgebildeten Antriebseinheiten jeweils eine Drehzahlmesseinheit umfasst.

27. Biopsievorrichtung nach Anspruch 26, **dadurch gekennzeichnet, dass** die Drehzahlmesseinheit jeweils optisch mittels einer Fotozelle und eines unmittelbar auf der Motorwelle aufsitzenden Gebers erfolgt.

28. Biopsievorrichtung nach Anspruch 25, **dadurch gekennzeichnet, dass** die Steuerungseinheit wenigstens einen programmierbaren Mikroprozessor vorsieht.

29. Biopsievorrichtung nach Anspruch 25, **dadurch gekennzeichnet, dass** das Bedienpanel wenigstens folgende Bedientastenfelder (88,89, 90) umfasst, deren Bedienmöglichkeit sowie Bedienfunktion jeweils durch Lichtsignale (91,92, 93,94, 95)markierbar sind : ein mit drei Funktionen belegtes "Biopsietastenfeld" (89), dessen unterschiedliche Funktionen jeweils durch unterschiedliche Lichtsignale (91,92, 93) markierbar sind und folgende Funktionen umfasst:
- Resetfunktion (91), d. h. Überführen aller im Gehäuse integrierten Komponenten in eine Soll-Position,
- Gewebeentnahmefunktion (92), d. h. Aufbau eines Unterdruckes innerhalb der hohlen Biopsienadel vermittels der Druckquelle, Öffnen des Gewebeprobeentnahmeraums (71) durch Zurückschieben der äußeren Hohlnadel (3) gegenüber der hohlen Biopsienadel (2), Schneidevorgang durch axiale periodische Vor- und Rückbewegungen der hohlen Biopsienadel (2) sowie Gewebeabtrennvorgang durch Vorschieben der äußeren Hohlnadel (3) über den Gewebeprobeentnahmeraum (71) gegenüber der hohlen Biopsienadel (2) hinaus, Belüften der Druckquelle (5) zum Abbau eines Unterdruckes,
- Gewebeauswurffunktion (93), d. h. teilweises Öffnen des Gewebeprobeentnahmeraums (71) durch nicht vollständiges Zurückziehen der äußeren Hohlnadel (3), Aufbau eines Überdruckes innerhalb der Druckquelle, durch den abgetrenntes Gewebematerial aus dem Gewebeprobeentnahmeraum (71) gelöst wird sowie vollständiges Zurückschieben der äußeren Hohlnadel (3) und dadurch vollständiges Öffnen des Gewebeprobeentnahmeraums (71),
- "Spanntastenfeld" (90), durch dessen Betätigung der Spannschlitten (28) in den gespannten Zustand überführbar ist und
- "Abschusstastenfeld" (88), durch dessen Betätigung der Spannschlitten (28) samt der Biopsienadeleinheit distalseits um eine bestimmte Strecke distalwärts geschossen wird.

30. Biopsievorrichtung nach Anspruch 22 oder 29, **dadurch gekennzeichnet, dass** die Steuereinheit zur Ausführung der Gewebeentnahmefunktion während des Schneidevorganges die erste Antriebseinheit in periodischer kurzzeitiger Abfolge in jeweils gegenläufige Rotationsbewegungen versetzt, so dass die äußere Hohlnadel (3) und die hohle Biopsienadel (2) Vor- und Rückbewegungen längs ihrer Nadellängsachse ausführt.

31. Biopsievorrichtung nach Anspruch 29, **dadurch gekennzeichnet, dass** die Steuereinheit ein Sicherheitsdelay vorsieht, durch das zumindest die Funktion des Spanntastenfeld (90) sowie die Gewebeauswurffunktion bei Bedienung zeitverzögert im Vergleich zum Bedienverhalten der übrigen Bedientastendelder erfolgt.

32. Biopsievorrichtung nach Anspruch 1, **dadurch gekennzeichnet, dass** das Gehäuse die Form eines Quaders besitzt, mit einem am Gehäuse angelenkten aufklappbaren Gehäusedeckel (10) sowie einer ersten und zweiten Stirnseite (6,7), wobei die erste Stirnseite (6) wenigstens eine Öffnung aufweist, durch die die distalen Nadelbereiche der äußeren Hohlnadel (3) und der hohlen Biopsienadel (2) zur Gewebeentnahme außerhalb des Gehäuses hinaus ragen, und die zweite Stirnseite (7) wenigstens zwei Ausnehmungen (15,16) vorsieht, durch die zumindest die Verbindungsleitung (4) geführt ist.

33. Biopsievorrichtung nach Anspruch 32, **dadurch gekennzeichnet, dass** in der Ausnehmung (16) der zweiten Stirnseite (7) ein Mikroschalter (19) vorgesehen ist, der vermittels der Druckquelle (5) bei geschlossenem Gehäuse betätigbar ist, wodurch eine Energieversorgung für die Antriebseinheiten (21, 58) freigebbar ist.

34. Biopsienadelmodul zur Implementierung in eine Biopsievorrichtung nach einem der Ansprüche 1 bis 33, das folgende Komponenten aufweist :
- eine in einem Biopsienadelträger (37) integrierte Biopsienadeleinheit, die eine äußere Hohlnadel (3) mit einer distalseits angeschärften Schneidklinge sowie eine im Inneren der Hohlnadel (3) gelagerte, hohle Biopsienadel (2) mit einem an ihrem distalen Endbereich vorgesehenen Gewebeprobeentnahmeraum (71) umfasst, wobei die äußere Hohlnadel durch Drehung relativ zur inneren Hohlnadel (2) gleitet,
- eine Verbindungsleitung (4) sowie
- eine Druckquelle (5), wobei die Verbindungsleitung (4) die hohle Biopsienadel (2) mit der Druckquelle (5) gasdicht verbindet.

35. Biopsienadelmodul nach Anspruch 34, **dadurch gekennzeichnet, dass** die Biopsienadeleinheit in einen Biopsienadelträger (37) lösbar fest integriert ist, wobei die innere Biopsienadel (2)proximalseits mit einem Anschlussstück (47) verbunden ist, über das die Verbindungsleitung (4) gasdicht mit der inneren Biopsienadel (2) verbindbar ist und das eine Befestigungskontur (49) aufweist, die in eine innerhalb des Biopsienadelträgers (37) vorgesehene Gegenkontur derart einsetzbar ist, so dass die innere Biopsienadel (2) zumindest in Nadellängsrichtung im Biopsienadelträger festsitzt, und die äußere Hohlnadel (3) in wenigstens einem Teilabschnitt längs ihres Außenumfangs eine Gewindekontur (73) vorsieht, die mit einer zumindest lösbar fest mit dem Biopsienadelträger (37) verbundenen Gegengewindekontur (75) derart in Eingriff steht, dass die äußere HohLnadel (3) relativ zur inneren Biopsienadel (2) in Nadellängsrichtung verschiebbar ist.

36. Biopsienadelmodul nach Anspruch 34, **dadurch gekennzeichnet, dass** am Außenumfang der äußeren Hohlnadel (3) ein Antriebsmittel (74) angebracht ist, durch das die äußere Hohlnadel (3) in Rotation um die Nadellängsrichtung versetzbar ist, wodurch die äußere Hohlnadel (3) relativ zum Biopsienadelträger (37) und somit zur hohlen Biopsienadel (2) axialverschiebbar ist..

37. Biopsienadelmodul nach Anspruch 34, **dadurch gekennzeichnet, dass** das Anschlussstück (47) ein Rastelement (50) vorsieht, das mit Wirkflächen des Biopsienadelträgers (37) derart in Kontakt steht, dass die innere Biopsienadel (2) um ihre Nadellängsrichtung in vorgebbare Positionen festsitzend verrastbar ist.

38. Biopsienadelmodul nach Anspruch 34, **dadurch gekennzeichnet, dass** zwischen der äußeren Hohlnadel (3) und der hohlen Biopsienadel (2) ein die Reibung zwischen beiden Nadeln erhöhendes Mittel vorgesehen ist, das in Art eines Dichtelementes (76) ausgebildet ist.

39. Biopsienadelmodul nach Anspruch 38, **dadurch gekennzeichnet, dass** das Anschlussstück (47) ein Rastelement (50) vorsieht, das mit Wirkflächen des Biopsienadelträgers (37) derart in Kontakt steht, dass die innere Biopsienadel (2) um ihre Nadellängsrichtung in vorgebbare Positionen derart verrastbar ist, dass zwischen den Wirkflächen und dem Rastelement ein Spiel vorhanden ist, durch das eine winkelbegrenzte Rotation der hohlen Biopsienadel (2) um ihre Längsachse in beide Rotationsrichtungen möglich ist.

40. Biopsienadelmodul nach Anspruch 34, **dadurch gekennzeichnet, dass** der Biopsienadelträger (37) eine Kupplungsstruktur (77) aufweist, die in eine an einem Spannschlitten (28) vorgesehene Gegenkupplungsstruktur (40) einsetzbar ist.

41. Biopsienadelmodul nach Anspruch 34, **dadurch gekennzeichnet, dass** die Druckquelle (5) eine Kolben-Zylindereinheit (69) aufweist, durch die in Abhängigkeit der Kolbenbewegung innerhalb der Zylindereinheit ein als Unter- oder Überdruck herschendes Druckniveau erzeugbar ist.

42. Biopsienadelmodul nach Anspruch 41, **dadurch gekennzeichnet, dass** die Zylindereinheit (52) in Art eines Spritzenkörpers ausgebildet ist und einen Zylinderboden (51) mit einem Anschlussstutzen (63) für eine gasdichte Verbindung zu der Verbindungsleitung (4) sowie eine dem Zylinderboden (51) gegenüberliegende Zylinderöffnung aufweist, dass die Kolbeneinheit eine Gewindespindel (53) vorsieht, an deren einen Ende der Kolben (54) angebracht ist und deren anderes Ende durch eine an der Zylinderöffnung der Zylindereinheit (52) vorgesehene Gewindespindelmutter (48) hindurchragt, die eine zahnradartig ausgebildete Umfangskontur (55) aufweist, die mit einer Antriebseinheit in Eingriff bringbar ist, und dass bei Rotation der Gewindespindelmutter (48) die Gewindespindel (53) samt Kolben (54) relativ zur Zylindereinheit (52) axialverschiebbar ist.

43. Biopsienadelmodul nach Anspruch 42, **dadurch gekennzeichnet, dass** die Zylindereinheit (52) im Bereich der Zylinderöffnung innerhalb der Zylinderwand wenigstens eine Belüftungsöffnung (67) vorsieht, so dass in einer Kolbenstellung nahe der Zylinderöffnung der zwischen dem Kolben (54) und dem Zylinderboden (51) eingeschlossene Zylinderraum entlüftbar ist.

44. Biopsienadelmodul nach Anspruch 41, **dadurch gekennzeichnet, dass** die Kolben-Zylindereinheit (69) eine Zylinderlängsachse aufweist, dass die Kolben-Zylindereinheit (69) derart neben der Biopsienadeleinheit angeordnet ist, dass die Nadellängsachse der Biopsienadeleinheit und die Zylinderlängsachse koparallel verlaufen, und dass die Verbindungsleitung (4) einen U-förmige Leitungsverlauf aufweist.

45. Biopsienadelmodul nach Anspruch 34, **dadurch gekennzeichnet, dass** die Verbindungsleitung (4) eine flexible, optisch weitgehend transparente Schlauchleitung ist, die eine Relativbewegung zwischen Druckquelle (5) und hohler Biopsienadel (2) ermöglicht.

46. Biopsienadelmodul nach Anspruch 34, **dadurch gekennzeichnet, dass** das aus Biopsienadelträger (37), in den die Biopsienadeleinheit integriert ist, Verbindungsleitung (4) sowie Druckquelle (5) bestehende Biopsienadelmodul in einer räumlich fest vorgebbaren Anordnung in eine Einlegehilfe (109) lösbar fest einsetzbar ist.

47. Biopsienadelmodul nach Anspruch 34 oder 35, **dadurch gekennzeichnet, dass** der Gewebeprobeentnahmeraum (71) axial von zwei Längsseitenkanten begrenzt ist, die als Schneidkanten (68) ausgebildet sind.

48. Biopsienadelmodul nach Anspruch 47, **dadurch gekennzeichnet, dass** die hohle Biopsienadel (2) zumindest im Bereich des Gewebeprobeentnahmeraum (71) in Art eines geraden Hohlzylinders ausgebildet ist, der eine lokale axiale Ausnehmung vorsieht, deren radiale Ausnehmungstiefe weniger als der halbe Innendurchmesser des Hohlzylinders beträgt und deren die Ausnehmung axial beidseitig begrenzende Längsseitenkanten derart als Schneidkanten ausgebildet sind, dass sich im Bereich der Längsseitenkanten der den Hohlzylinder beschreibende Innenradius kontinuierlich an den Außenradius angleicht.

49. Biopsienadelmodul nach Anspruch 34, **dadurch gekennzeichnet, dass** die hohle Biopsienadel (2) am proximalen Ende des Gewebeprobeentnahmeraums (71) eine den Querschnitts des von der Biopsienadel eingeschlossenen Hohlkanals verkleinernde Einengung vorsieht, die eine Hohlkanaldurchtrittsöffnung in den Gewebeprobeentnahmeraum (71) im unteren Bereich des Gewebeprobeentnahmeraum (71) offen lässt.

50. Biopsienadelmodul nach Anspruch 49, **dadurch gekennzeichnet, dass** die Einengung etwa 60-70 % des Hohlkanalquerschnittes abdeckt, und die Einengung in Art eines in den Hohlkanal ragende Stopfens oder als ein in die Querschnittsfläche des Hohlkanals ragendes Flächenelement ausgebildet ist.

51. Biopspienadelmodul nach Anspruch 35, **dadurch gekennzeichnet, dass** distalseits zum Biopsienadelträger (37) eine Führungsrolle (81) gleitend auf dem Außenumfang der äußeren Hohlnadel (3) aufsitzend vorgesehen ist, die eine weitgehend passgenau an den Außenumfang der äußeren Hohlnadel (3) dimensionierte Durchtrittsöffnung aufweist, durch die die äußere Hohlnadel (3) hindurchtritt, und die in eine Durchführung (13) im distalen Gehäuseenddeckel (6) einsetzbar ist.

## Claims

1. Biopsy device for tissue collection in the nature of a handpiece with at least one clamping and closing device that can be acted on by a spring force and is in the form of a clamping carriage (28) for a biopsy needle unit, which has an outer hollow needle (3), with a distally bevelled cutting blade, and a hollow biopsy needle (2) mounted in the inside of the outer hollow needle (3) and having, provided at its distal end area, a tissue sample collection space (71) mounted in a biopsy needle carrier (37) which can be brought into operative connection at least with the clamping carriage (28), wherein the distal needle areas of the outer hollow needle (3) and of the hollow biopsy needle (2) for tissue collection protrude from the housing, and wherein the handpiece has a housing with a housing cover (10), wherein components can be securely integrated into the housing interior in the open position, **characterized in that** a pressure source (5) is provided which can be connected to the hollow biopsy needle (2), and **in that**
- at least a first and second drive unit (21, 58) are integrated into the housing interior, wherein
- the clamping carriage (28) can be brought into operative connection with the first drive unit (21) in such a way that the clamping carriage (28) can be transferred into a clamped state and can be locked in this state, and wherein a drive means (74) mounted on the outer hollow needle can be brought into operative connection with the first drive unit (21), by which the outer hollow needle (3) can be set in rotation about the longitudinal direction of the needle, as a result of which the outer hollow needle (3) shifts relative to the longitudinal axis of the hollow biopsy needle (2), and
- the pressure source (5), which in the proximal area of the hollow biopsy needle (2) is connected to the latter in a gas-tight manner via at least one connecting line (4) and can be brought into operative connection with the second drive unit (58) in order to generate a pressure level, wherein the connecting line (4) runs at least partially inside the housing.

2. Biopsy device according to Claim 1, **characterized in that** the biopsy needle unit is securely integrated releasably into the biopsy needle carrier (37), wherein the inner biopsy needle (2) is connected proximally to an attachment piece (47) via which the connecting line (4) can be connected in a gas-tight manner to the inner biopsy needle (2), and which has a securing contour (49) which can be inserted into a mating contour provided inside the biopsy needle carrier (37) in such a way that the inner biopsy needle (2) sits firmly in the biopsy needle carrier at least in the longitudinal direction of the needle, and the outer hollow needle (3), in at least a partial section along the outer circumference thereof, has a thread contour (73), which engages with a mating thread contour (75), connected at least releasably to the biopsy needle carrier (37), in such a way that the outer hollow needle (3) is displaceable in the longitudinal direction of the needle relative to the inner biopsy needle (2).

3. Biopsy device according to Claim 2, **characterized in that** the attachment piece (47) has a locking element (50), which is in contact with active surfaces of the biopsy needle carrier (37) in such a way that the inner biopsy needle (2) can be firmly locked in predefinable positions about its longitudinal direction.

4. Biopsy device according to Claim 2, **characterized in that**, between the outer hollow needle (3) and the hollow biopsy needle (2), a means is provided that increases the friction between both needles.

5. Biopsy device according to Claim 4, **characterized in that** the attachment piece (47) has a locking element (50), which is in contact with active surfaces of the biopsy needle carrier (37) in such a way that the inner biopsy needle (2) can be locked in predefinable positions about its longitudinal direction, such that, between the active surfaces and the locking element, a clearance is present by which an angle-limited rotation of the hollow biopsy needle (2) about its longitudinal axis is possible in both directions of rotation.

6. Biopsy device according to Claim 1 or 5, **characterized in that**, by alternate rotation of the outer hollow needle (3), driven via the drive means (74) engaging with the first drive unit (21), the hollow biopsy needle (2) co-rotates in the path of the increased friction prevailing between both needles (2, 3), wherein the rotation movement of the hollow biopsy needle (2) is limited in both directions of rotation by the clearance-associated locking element (50).

7. Biopsy device according to Claim 1, **characterized in that** an elastic sealing element (76) that seals both needles (2, 3) in a gas-tight manner is provided on the outer hollow needle (3) and the hollow biopsy needle (2).

8. Biopsy device according to Claim 2, **characterized in that** the biopsy needle carrier (37) has a coupling structure (77), which can be inserted into a mating coupling structure (40) provided on the clamping carriage (28).

9. Biopsy device according to Claim 1, **characterized in that** the pressure source (5) has a piston/cylinder unit (69), by means of which a pressure level prevailing as underpressure or overpressure can be generated as a function of the piston movement inside the cylinder unit.

10. Biopsy device according to Claim 9, **characterized in that** the cylinder unit (52) is designed in the manner of a syringe body and has a cylinder base (51), with a connector (63) for gas-tight connection to the connecting line (4), and a cylinder opening located opposite the cylinder base (51), **in that** the piston unit has a threaded spindle (53), at one end of which the piston (54) is mounted, and the other end of which protrudes through a threaded spindle nut (48) which is provided on the cylinder opening of the cylinder unit (52) and which has a toothed-wheel-like circumferential contour (55) that can be brought into engagement with the second drive unit, and **in that**, upon rotation of the threaded spindle nut (48), the threaded spindle (53) together with the piston (54) is axially displaceable relative to the cylinder unit (52).

11. Biopsy device according to Claim 9 or 10, **characterized in that** the cylinder unit (52) has, inside the cylinder wall in the area of the cylinder opening, at least one vent opening (67), such that, in a piston position near the cylinder opening, the cylinder space enclosed between the piston (54) and the cylinder base (51) can be vented.

12. Biopsy device according to Claim 9, **characterized in that** the piston/cylinder unit (69) has a cylinder longitudinal axis, **in that** the piston/ cylinder unit (69) is arranged alongside the biopsy needle unit in the housing interior in such a way that the longitudinal axis of the needle of the biopsy needle unit and the cylinder axis of the piston/cylinder unit (69) run co-parallel, and **in that** the connecting line (4) has a U-shaped line profile.

13. Biopsy device according to one of Claims 1 to 12, **characterized in that** the connecting line (4) is a flexible, optically substantially transparent hose line, which permits a relative movement between pressure source (5) and hollow biopsy needle (2).

14. Biopsy device according to one of Claims 1 to 13, **characterized in that** the biopsy needle unit, integrated into a biopsy needle carrier (37), and the pressure source (5) connected in a gas-tight manner to the hollow biopsy needle (2) via the connecting line (4) are designed as a uniform module (29) that can be inserted into the housing and can be removed again from the housing.

15. Biopsy device according to Claim 14, **characterized in that** the module (29), consisting of biopsy needle carrier (37), into which the biopsy needle unit is integrated, of connecting line (4) and of pressure source (5), can be inserted releasably in a spatially predefinable arrangement into an insert aid (109), by means of which the module (29) can be introduced into the housing interior.

16. Biopsy device according to Claim 1, **characterized in that**, by means of the drive means (74), which is connected to an output shaft of the first drive unit (21), and by means of the thread contour (73) thus moved in rotation and of the resulting axial displacement of the outer hollow needle (3), the clamping carriage (28) can be transferred into the clamped state, wherein the drive means (74) is supported at one end against a holder (36) which serves as a mechanical counter-bearing and is fixed with respect to the housing, and **in that** the clamping carriage (28) can be mechanically locked when the clamped state is reached.

17. Biopsy device according to Claim 1 or 16, **characterized in that** the clamping carriage (28) locked in the clamped state can be released by an unlocking mechanism, as a result of which the clamping carriage (28) and, connected thereto, the biopsy needle carrier (37) can be transferred abruptly into the unclamped state, wherein the biopsy needle unit, i.e. the outer hollow needle (3) and the hollow biopsy needle (2) are jointly moved distally.

18. Biopsy device according to Claim 1 or 16, **characterized in that** the first drive unit (21), as output shaft, has a toothed roller (23) into which the drive means (74) designed as a toothed wheel engages.

19. Biopsy device according to Claim 1, **characterized in that** the first and second drive units (21, 58) are designed as an electric direct-current motor with downstream planetary gear.

20. Biopsy device according to Claim 10 or 19, **characterized in that** the second drive unit (58) has an output shaft with a driving pinion (56) which can be brought into engagement with the toothed-wheel-like circumferential contour (55) of the threaded spindle nut (48) of the piston/ cylinder unit (69).

21. Biopsy device according to Claim 1, **characterized in that** a base block (8) is provided, which is connected releasably to the housing interior and which has recesses for securing the first and second drive units (21, 58) in such a way that the output shafts thereof run at a distance from each other and parallel to each other, **in that** an abutment element (26) is provided on the base block (8), from which abutment element (26) a guide pin (30) emerges along which the clamping carriage (28) can be guided under the effect of a spring force, and **in that** the base block (8) has recesses for the insertion of the biopsy needle unit held in the biopsy needle carrier (37) and permits a positioning of the pressure source (5).

22. Biopsy device according to Claim 21, **characterized in that** the clamping carriage (28) is acted on by a spring force on both sides along the guide pin (30), such that the clamping carriage (28) in the unclamped state can be deflected from a rest position to both sides along the guide pin (30).

23. Biopsy device according to Claim 1, **characterized in that** a power supply unit (11) in the form of a battery or of a rechargeable accumulator is provided in the housing interior.

24. Biopsy device according to Claim 23, **characterized in that** the power supply unit is separated by a separating plate (114) from the area of the clamping carriage (28) and of the pressure source (5).

25. Biopsy device according to Claim 1, **characterized in that** a control unit for controlling the first and second drive units (21, 58) is provided in the housing, and **in that** the control unit and a release of the locking of the clamping carriage (28) can be actuated via an operating panel (57) mounted on an outer wall of the housing.

26. Biopsy device according to Claim 25, **characterized in that** the control unit for controlling the drive units, designed as electric direct-current motors, comprises in each case a revolution counter.

27. Biopsy device according to Claim 26, **characterized in that** the revolution counter functions in each case optically by means of a photocell and of a sensor sitting directly on the motor shaft.

28. Biopsy device according to Claim 25, **characterized in that** the control unit has at least one programmable microprocessor.

29. Biopsy device according to Claim 25, **characterized in that** the operating panel comprises at least the following operating fields (88, 89, 90), of which the operating possibility and operating function can in each case be marked by light signals (91, 92, 93, 94, 95): a "biopsy field" (89) with three functions, of which the different functions can each be marked by different light signals (91, 92, 93) and comprises the following functions:
- reset function (91), i.e. transferring all the components integrated in the housing to a desired position,
- tissue collection function (92), i.e. build-up of an underpressure inside the hollow biopsy needle by means of the pressure source, opening of the tissue sample collection space (71) by sliding back of the outer hollow needle (3) relative to the hollow biopsy needle (2), cutting procedure by axial periodic forward and backward movements of the hollow biopsy needle (2), and tissue separation procedure by advancing the outer hollow needle (3) past the tissue sample collection space (71) in relation to the hollow biopsy needle (2), venting the pressure source (5) to reduce an underpressure,
- tissue ejection function (93), i.e. partial opening of the tissue sample collection space (71) by incomplete retraction of the outer hollow needle (3), build-up of an overpressure inside the pressure source, the separated tissue material is released from the tissue sample collection space (71), and complete sliding-back of the outer hollow needle (3) and, as a result, complete opening of the tissue sample collection space (71),
- "clamping field" (90), the actuation of which allows the clamping carriage (28) to be transferred to the clamped state, and
- "launch field" (88), the actuation of which allows the clamping carriage (28) together with the biopsy needle unit to be shot a certain distance in the distal direction.

30. Biopsy device according to Claim 22 or 29, **characterized in that** the control unit, for executing the tissue collection function during the cutting procedure, moves the first drive unit in periodic short sequence in each case in opposite rotation movements such that the outer hollow needle (3) and the hollow biopsy needle (2) execute forward and backward movements along the longitudinal axis of the needle.

31. Biopsy device according to Claim 29, **characterized in that** the control unit has a safety delay, by which at least the function of the clamping field (90) and the tissue ejection function are delayed in comparison to the operating behaviour of the other operating panel fields.

32. Biopsy device according to Claim 1, **characterized in that** the housing has the shape of a cuboid, with a fold-open housing cover (10) articulated on the housing and with a first and second end face (6, 7), wherein the first end face (6) has at least one opening through which the distal needle areas of the outer hollow needle (3) and of the hollow biopsy needle (2) protrude from the housing for tissue collection, and the second end face (7) has at least two recesses (15, 16) through which at least the connecting line (4) is guided.

33. Biopsy device according to Claim 32, **characterized in that**, in the recess (16) of the second end face (7), a micro-switch (19) is provided which can be actuated by means of the pressure source (5) when the housing is closed, as a result of which a power supply for the drive units (21, 58) can be released.

34. Biopsy needle module for implementation in a biopsy device according to one of Claims 1 to 33, having the following components:
- a biopsy needle unit, which is integrated in a biopsy needle carrier (37) and which comprises an outer hollow needle (3), with a distally bevelled cutting blade, and a hollow biopsy needle (2) mounted in the inside of the hollow needle (3), with a tissue sample collection space (71) provided at the distal end area thereof, wherein the outer hollow needle slides relative to the inner hollow needle (2) by rotation,
- a connecting line (4), and
- a pressure source (5), wherein the connecting line (4) connects the hollow biopsy needle (2) to the pressure source (5) in a gas-tight manner.

35. Biopsy needle module according to Claim 34, **characterized in that** the biopsy needle unit is securely integrated releasably into a biopsy needle carrier (37), wherein the inner biopsy needle (2) is connected proximally to an attachment piece (47) via which the connecting line (4) can be connected in a gas-tight manner to the inner biopsy needle (2), and which has a securing contour (49) which can be inserted into a mating contour provided inside the biopsy needle carrier (37) in such a way that the inner biopsy needle (2) sits firmly in the biopsy needle carrier at least in the longitudinal direction of the needle, and the outer hollow needle (3), in at least a partial section along the outer circumference thereof, has a thread contour (73), which engages with a mating thread contour (75), connected at least releasably to the biopsy needle carrier (37), in such a way that the outer hollow needle (3) is displaceable in the longitudinal direction of the needle relative to the inner biopsy needle (2).

36. Biopsy needle module according to Claim 34, **characterized in that** a drive means (74) is mounted on the outer circumference of the outer hollow needle (3), via which drive means (74) the outer hollow needle (3) can be set in rotation about the longitudinal direction of the needle, as a result of which the outer hollow needle (3) is axially displaceable relative to the biopsy needle carrier (37) and thus to the hollow biopsy needle (2).

37. Biopsy needle module according to Claim 34, **characterized in that** the attachment piece (47) has a locking element (50), which is in contact with active surfaces of the biopsy needle carrier (37) in such a way that the inner biopsy needle (2) can be firmly locked in predefinable positions about its longitudinal direction.

38. Biopsy needle module according to Claim 34, **characterized in that**, between the outer hollow needle (3) and the hollow biopsy needle (2), a means is provided which increases the friction between both needles and which is designed in the manner of a sealing element (76).

39. Biopsy needle module according to Claim 38, **characterized in that** the attachment piece (47) has a locking element (50), which is in contact with active surfaces of the biopsy needle carrier (37) in such a way that the inner biopsy needle (2) can be locked in predefinable positions about its longitudinal direction, such that, between the active surfaces and the locking element, a clearance is present by which an angle-limited rotation of the hollow biopsy needle (2) about its longitudinal axis is possible in both directions of rotation.

40. Biopsy needle module according to Claim 34, **characterized in that** the biopsy needle carrier (37) has a coupling structure (77), which can be inserted into a mating coupling structure (40) provided on a clamping carriage (28).

41. Biopsy needle module according to Claim 34, **characterized in that** the pressure source (5) has a piston/cylinder unit (69), by means of which a pressure level prevailing as underpressure or overpressure can be generated as a function of the piston movement inside the cylinder unit.

42. Biopsy needle module according to Claim 41, **characterized in that** the cylinder unit (52) is designed in the manner of a syringe body and has a cylinder base (51), with a connector (63) for gas-tight connection to the connecting line (4), and a cylinder opening located opposite the cylinder base (51), **in that** the piston unit has a threaded spindle (53), at one end of which the piston (54) is mounted, and the other end of which protrudes through a threaded spindle nut (48) which is provided on the cylinder opening of the cylinder unit (52) and which has a toothed-wheel-like circumferential contour (55) that can be brought into engagement with a second drive unit, and **in that**, upon rotation of the threaded spindle nut (48), the threaded spindle (53) together with the piston (54) is axially displaceable relative to the cylinder unit (52).

43. Biopsy needle module according to Claim 42, **characterized in that** the cylinder unit (52) has, inside the cylinder wall in the area of the cylinder opening, at least one vent opening (67), such that, in a piston position near the cylinder opening, the cylinder space enclosed between the piston (54) and the cylinder base (51) can be vented.

44. Biopsy needle module according to Claim 41, **characterized in that** the piston/cylinder unit (69) has a cylinder longitudinal axis, **in that** the piston/cylinder unit (69) is arranged alongside the biopsy needle unit in such a way that the longitudinal axis of the needle of the biopsy needle unit and the cylinder longitudinal axis run co-parallel, and **in that** the connecting line (4) has a U-shaped line profile.

45. Biopsy needle module according to Claim 34, **characterized in that** the connecting line (4) is a flexible, optically substantially transparent hose line, which permits a relative movement between pressure source (5) and hollow biopsy needle (2).

46. Biopsy needle module according to Claim 34, **characterized in that** the biopsy needle module, consisting of biopsy needle carrier (37), into which the biopsy needle unit is integrated, of connecting line (4) and of pressure source (5), can be inserted releasably in a spatially predefinable arrangement into an insert aid (109).

47. Biopsy needle module according to Claim 34 or 35, **characterized in that** the tissue sample collection space (71) is axially delimited by two longitudinal side edges, which are designed as cutting edges (68).

48. Biopsy needle module according to Claim 47, **characterized in that** the hollow biopsy needle (2), at least in the area of the tissue sample collection space (71), is designed in the manner of a straight hollow cylinder, which has a local axial recess, of which the radial recess depth is less than half the internal diameter of the hollow cylinder, and of which the longitudinal side edges axially delimiting the recess on both sides are designed as cutting edges in such a way that, in the area of the longitudinal side edges, the inner radius describing the hollow cylinder continuously matches the outer radius.

49. Biopsy needle module according to Claim 34, **characterized in that** the hollow biopsy needle (2), at the proximal end of the tissue sample collection space (71), has a constriction reducing the cross section of the hollow channel enclosed by the biopsy needle, which constriction leaves open a hollow channel passage into the tissue sample collection space (71) in the lower area of the tissue sample collection space (71).

50. Biopsy needle module according to Claim 49, **characterized in that** the constriction covers about 60-70% of the cross section of the hollow channel, and the constriction is designed in the manner of a stopper protruding into the hollow channel or as a surface element protruding into the cross-sectional surface area of the hollow channel.

51. Biopsy needle module according to Claim 35, **characterized in that**, distally with respect to the biopsy needle carrier (37), a guide roller (81) is provided sitting and sliding on the outer circumference of the outer hollow needle (3), which guide roller (81) has a through-opening which is dimensioned substantially to match the outer circumference of the outer hollow needle (3) and through which the outer hollow needle (3) passes, and which can be inserted into a passage (13) in the distal housing cover (6).

## Revendications

1. Dispositif de biopsie pour le prélèvement de tissu du type d'un instrument manuel avec au moins un dispositif de serrage et de propulsion actionnable par la force d'un ressort sous la forme d'un coulisseau de serrage (28) pour une unité d'aiguille de biopsie, qui présente une aiguille creuse extérieure (3) avec une lame de coupe affûtée côté distal ainsi qu'une aiguille de biopsie creuse (2) logée à l'intérieur de l'aiguille creuse extérieure (3), avec une chambre de prélèvement d'échantillon de tissu (71) prévue dans sa région d'extrémité distale, qui est montée dans un support d'aiguille de biopsie (37), qui peut être mis en liaison active au moins avec le coulisseau de serrage (28), dans lequel les régions d'aiguille distales de l'aiguille creuse extérieure (3) et de l'aiguille de biopsie creuse (2) sortent du boîtier pour le prélèvement de tissu, et dans lequel l'instrument manuel présente un boîtier avec un couvercle de boîtier (10), dans lequel, en position ouverte, des composants peuvent être intégrés à l'intérieur du boîtier, **caractérisé en ce que**
- il est prévu une source de pression (5), qui peut être raccordée à l'aiguille de biopsie creuse (2), et
- dans le boîtier:
- au moins une première et une deuxième unités d'entraînement (21, 58) sont intégrées, dans lequel
- le coulisseau de serrage (28) peut être mis en liaison active avec la première unité d'entraînement (21), de telle manière que le coulisseau de serrage (28) puisse être amené dans un état serré et puisse être verrouillé dans celui-ci, et dans lequel un moyen d'entraînement (74) placé sur l'aiguille creuse extérieure peut être mis en liaison active avec la première unité d'entraînement (21), par laquelle l'aiguille creuse extérieure (3) peut être mise en rotation autour de la direction longitudinale de l'aiguille, ce qui provoque le déplacement de l'aiguille creuse extérieure (3) par rapport à l'axe longitudinal d'aiguille de l'aiguille de biopsie creuse (2), et
- la source de pression (5), qui est raccordée de façon étanche au gaz à l'aiguille de biopsie creuse (2) dans la région proximale de celle-ci au moyen d'au moins une conduite de raccordement (4) et qui peut être mise en liaison active avec la deuxième unité d'entraînement (58) pour produire un niveau de pression, dans lequel la conduite de raccordement (4) s'étend au moins en partie à l'intérieur du boîtier.

2. Dispositif de biopsie selon la revendication 1, **caractérisé en ce que** l'unité d'aiguille de biopsie est fermement intégrée de façon détachable dans le support d'aiguille de biopsie (37), dans lequel l'aiguille de biopsie intérieure (2) est raccordée côté proximal à une pièce de raccordement (47), par laquelle la conduite de raccordement (4) peut être raccordée de façon étanche au gaz à l'aiguille de biopsie intérieure (2) et qui présente un contour de fixation (49), qui peut être inséré dans un contour opposé prévu à l'intérieur du support d'aiguille de biopsie (37), de telle manière que l'aiguille de biopsie intérieure (2) soit calée dans le support d'aiguille de biopsie au moins en direction longitudinale de l'aiguille, et l'aiguille creuse extérieure (3) prévoit, sur au moins une section partielle le long de son périmètre extérieur, un contour fileté (73), qui est en prise avec un contour fileté opposé (75) au moins fermement assemblé de façon détachable au support d'aiguille de biopsie (37) de telle manière que l'aiguille creuse extérieure (3) soit déplaçable en direction longitudinale par rapport à l'aiguille de biopsie intérieure (2).

3. Dispositif de biopsie selon la revendication 2, **caractérisé en ce que** la pièce de raccordement (47) prévoit un élément d'encliquetage (50), qui est en contact avec des faces actives du support d'aiguille de biopsie (37), de telle manière que l'aiguille de biopsie intérieure (2) puisse être calée par encliquetage dans des positions prédéterminables autour de sa direction longitudinale d'aiguille.

4. Dispositif de biopsie selon la revendication 2, **caractérisé en ce qu'**il est prévu entre l'aiguille creuse extérieure (3) et l'aiguille de biopsie intérieure (2) un moyen augmentant le frottement entre les deux aiguilles.

5. Dispositif de biopsie selon la revendication 4, **caractérisé en ce que** la pièce de raccordement (47) prévoit un élément d'encliquetage (50), qui est en contact avec des faces actives du support d'aiguille de biopsie (37), de telle manière que l'aiguille de biopsie intérieure (2) puisse être encliquetée dans des positions prédéterminables autour de sa direction longitudinale d'aiguille, de telle manière qu'il existe entre les faces actives et l'élément d'encliquetage un jeu par lequel une rotation angulaire limitée de l'aiguille de biopsie creuse (2) autour de son axe longitudinal est possible dans les deux sens de rotation.

6. Dispositif de biopsie selon la revendication 1 ou 5, **caractérisé en ce que**, par une rotation alternée de l'aiguille creuse extérieure (3), entraînée par le moyen d'entraînement (74) se trouvant en prise avec la première unité d'entraînement (21), l'aiguille de biopsie creuse (2) est entraînée en rotation à cause du frottement accru régnant entre les deux aiguilles (2, 3), dans lequel le mouvement de rotation de l'aiguille de biopsie creuse (2) est limité dans les deux sens de rotation par l'élément d'encliquetage (50) présentant du jeu.

7. Dispositif de biopsie selon la revendication 1, **caractérisé en ce qu'**il est prévu sur l'aiguille creuse extérieure (3) et sur l'aiguille de biopsie creuse (2) un élément d'étanchéité élastique (76), qui assure l'étanchéité au gaz des deux aiguilles (2, 3) l'une par rapport à l'autre.

8. Dispositif de biopsie selon la revendication 2, **caractérisé en ce que** le support d'aiguille de biopsie (37) présente une structure de couplage (77), qui peut être insérée dans une structure de couplage opposée (40) prévue sur le coulisseau de serrage (28).

9. Dispositif de biopsie selon la revendication 1, **caractérisé en ce que** la source de pression (5) présente une unité à piston-cylindre (69), par laquelle on peut produire un niveau de pression régnant, en dépression ou en surpression, à l'intérieur de l'unité de cylindre, en fonction du mouvement du piston.

10. Dispositif de biopsie selon la revendication 9, **caractérisé en ce que** l'unité de cylindre (52) est réalisée sous la forme d'un corps de seringue et présente un fond de cylindre (51) avec un embout de raccordement (63) pour un raccordement étanche au gaz de la conduite de raccordement (4) ainsi qu'une ouverture de cylindre opposée au fond de cylindre (51), **en ce que** l'unité de piston prévoit une broche filetée (53), sur une extrémité de laquelle le piston (54) est monté et dont l'autre extrémité traverse un écrou de broche filetée (48) prévu sur l'ouverture de cylindre de l'unité de cylindre (52), qui présente un contour périphérique en forme de roue dentée (55), qui peut être mis en prise avec la deuxième unité d'entraînement, et **en ce que**, lors de la rotation de l'écrou de broche filetée (48), la broche filetée (53) avec le piston (54) est déplaçable en direction axiale par rapport à l'unité de cylindre (52).

11. Dispositif de biopsie selon la revendication 9 ou 10, **caractérisé en ce que** l'unité de cylindre (52) prévoit, dans la région de l'ouverture de cylindre à l'intérieur de la paroi du cylindre au moins une ouverture de ventilation (67), de telle manière que, dans une position du piston proche de l'ouverture de cylindre, la chambre de cylindre formée entre le piston (54) et le fond de cylindre (51) puisse être purgée.

12. Dispositif de biopsie selon la revendication 9, **caractérisé en ce que** l'unité à piston-cylindre (69) présente un axe longitudinal de cylindre, **en ce que** l'unité à piston-cylindre (69) est disposée à l'intérieur du boîtier à côté de l'unité d'aiguille de biopsie, de telle manière que l'axe longitudinal d'aiguille de l'unité d'aiguille de biopsie et l'axe de cylindre de l'unité à piston-cylindre (69) soient co-parallèles, et **en ce que** la conduite de raccordement (4) présente un parcours de conduite en forme de U.

13. Dispositif de biopsie selon l'une quelconque des revendications 1 à 12, **caractérisé en ce que** la conduite de raccordement (4) est formée par un tuyau flexible, largement transparent optiquement, qui permet un mouvement relatif entre la source de pression (5) et une aiguille de biopsie creuse (2).

14. Dispositif de biopsie selon l'une quelconque des revendications 1 à 13, **caractérisé en ce que** l'unité d'aiguille de biopsie intégrée dans un support d'aiguille de biopsie (37) et la source de pression (5) raccordée de façon étanche au gaz à l'aiguille de biopsie creuse (2) par la conduite de raccordement (4) se présentent sous la forme d'un module (29) pouvant être inséré comme un tout dans le boîtier et de nouveau retiré hors du boîtier.

15. Dispositif de biopsie selon la revendication 14, **caractérisé en ce que** le module (29) composé du support d'aiguille de biopsie (37), dans lequel l'unité d'aiguille de biopsie est intégrée, de la conduite de raccordement (4) ainsi que de la source de pression (5) peut être fermement inséré de façon détachable dans un accessoire d'insertion (109) dans un agencement fermement prédéterminable spatialement, au moyen duquel le module (29) peut être mis en oeuvre à l'intérieur du boîtier.

16. Dispositif de biopsie selon la revendication 1, **caractérisé en ce que** le coulisseau de serrage (28) peut être amené à l'état serré au moyen du moyen d'entraînement (74), qui est en liaison avec un arbre de sortie de la première unité d'entraînement (21), ainsi que du contour fileté (73) pouvant ainsi être mis en rotation et du déplacement axial de l'aiguille creuse extérieure (3) réalisable de cette manière, dans lequel le moyen d'entraînement (74) prend appui d'un côté contre un support (36) servant de contre-appui mécanique, qui est prévu solidairement au boîtier, et **en ce que** le coulisseau de serrage (28) peut être verrouillé mécaniquement lorsqu'il atteint l'état serré.

17. Dispositif de biopsie selon la revendication 1 ou 16, **caractérisé en ce que** le coulisseau de serrage (28) verrouillé dans l'état serré peut être libéré par un mécanisme de déverrouillage, le coulisseau de serrage (28) et le support d'aiguille de biopsie (37) assemblé à celui-ci peut être amené brusquement à l'état desserré, dans lequel l'unité d'aiguille de biopsie, c'est-à-dire l'aiguille creuse extérieure (3) et l'aiguille de biopsie creuse (2) sont déplacées ensemble côté distal.

18. Dispositif de biopsie selon la revendication 1 ou 16, **caractérisé en ce que** la première unité d'entraînement (21) prévoit comme arbre de sortie un rouleau denté (23), dans lequel engrène le moyen d'entraînement (74) formé par une roue dentée.

19. Dispositif de biopsie selon la revendication 1, **caractérisé en ce que** la première et la deuxième unités d'entraînement (21, 58) sont constituées par un moteur électrique à courant continu suivi d'un engrenage planétaire.

20. Dispositif de biopsie selon la revendication 10 ou 19, **caractérisé en ce que** la deuxième unité d'entraînement (58) prévoit un arbre de sortie avec un pignon d'entraînement (56), qui peut être mis en prise avec le contour périphérique (55) réalisé en forme de roue dentée de l'écrou de broche filetée (48) de l'unité à piston-cylindre (69).

21. Dispositif de biopsie selon la revendication 1, **caractérisé en ce qu'**il est prévu, fermement assemblé de façon détachable à l'intérieur du boîtier, un bloc de base (8), qui prévoit des évidements pour la fixation de la première et de la deuxième unités d'entraînement (21, 58), de telle manière que leurs arbres de sortie soient espacés l'un de l'autre et soient parallèles l'un à l'autre, **en ce qu'**il est prévu sur le bloc de base (8) un élément de butée (26), duquel sort un boulon de guidage (30) le long duquel le coulisseau de serrage (28) peut être guidé sous l'action de la force d'un ressort, et **en ce que** le bloc de base (8) prévoit des évidements pour la pose de l'unité d'aiguille de biopsie saisie dans le support d'aiguille de biopsie (37) et permet un positionnement de la source de pression (5).

22. Dispositif de biopsie selon la revendication 21, **caractérisé en ce que** le coulisseau de serrage (28) est sollicité par une force de ressort de part et d'autre le long du boulon de guidage (30), de telle manière que le coulisseau de serrage (28) à l'état détendu puisse être déplacé à partir d'une position de repos vers les deux côtés le long du boulon de guidage (30).

23. Dispositif de biopsie selon la revendication 1, **caractérisé en ce qu'**il est prévu à l'intérieur du boîtier une unité de fourniture d'énergie (11) sous la forme d'une batterie ou d'un accumulateur rechargeable.

24. Dispositif de biopsie selon la revendication 23, **caractérisé en ce que** l'unité de fourniture d'énergie est séparée de la région du coulisseau de serrage (28) ainsi que de la source de pression (5) au moyen d'une plaque de séparation (114).

25. Dispositif de biopsie selon la revendication 1, **caractérisé en ce qu'**il est prévu dans le boîtier une unité de commande destinée à commander la première et la deuxième unités d'entraînement (21, 58), et **en ce que** l'unité de commande ainsi qu'une libération du verrouillage du coulisseau de serrage (28) est actionnable au moyen d'un tableau de commande (57) installé sur une paroi extérieure du boîtier.

26. Dispositif de biopsie selon la revendication 25, **caractérisé en ce que** l'unité de commande destinée à commander les unités d'entraînement en forme de moteurs électriques à courant continu comprend chaque fois une unité de mesure de la vitesse de rotation.

27. Dispositif de biopsie selon la revendication 26, **caractérisé en ce que** l'unité de mesure de la vitesse de rotation opère chaque fois optiquement au moyen d'une cellule photoélectrique et d'un capteur installé directement sur l'arbre du moteur.

28. Dispositif de biopsie selon la revendication 25, **caractérisé en ce que** l'unité de commande prévoit au moins un microprocesseur programmable.

29. Dispositif de biopsie selon la revendication 25, **caractérisé en ce que** le tableau de commande comprend au moins les touches de commande suivantes (88, 89, 90), dont la possibilité de commande ainsi que la fonction de commande peuvent être marquées respectivement par des signaux lumineux (91, 92, 93, 94, 95): une "touche de commande de biopsie" (89) revêtue de trois fonctions, dont les différentes fonctions peuvent être marquées respectivement par des signaux lumineux différents (91, 92, 93) et qui comprend les fonctions suivantes:
- fonction de remise à zéro (91), c'est-à-dire retour de tous les composants intégrés dans le boîtier en position de consigne,
- fonction de prélèvement de tissu (92), c'est-à-dire création d'une dépression à l'intérieur de l'aiguille de biopsie creuse au moyen de la source de pression, ouverture de la chambre de prélèvement d'échantillon de tissu (71) par recul de l'aiguille creuse extérieure (3) par rapport à l'aiguille de biopsie creuse (2), opération de coupe par mouvement avant et arrière périodique axial de l'aiguille de biopsie creuse (2) ainsi qu'opération de séparation de tissu par mouvement avant de l'aiguille creuse extérieure (3) au-dessus de la chambre de prélèvement d'échantillon de tissu (71) par rapport à l'aiguille de biopsie creuse (2), ventilation de la source de pression (5) pour supprimer une dépression,
- fonction d'éjection de tissu (93), c'est-à-dire ouverture partielle de la chambre de prélèvement d'échantillon de tissu (71) par un recul incomplet de l'aiguille creuse extérieure (3), production d'une surpression à l'intérieur de la source de pression, par laquelle la matière de tissu séparée est expulsée hors de la chambre de prélèvement d'échantillon de tissu (71) ainsi que recul complet de l'aiguille creuse extérieure (3) et de ce fait ouverture complète de la chambre de prélèvement d'échantillon de tissu (71),
- "touche de serrage" (90) dont l'actionnement permet d'amener le coulisseau de serrage (28) dans l'état serré, et
- "touche de propulsion" (88), dont l'actionnement provoque la propulsion du coulisseau de serrage (28) avec l'unité d'aiguille de biopsie côté distal sur une course déterminée en direction distale.

30. Dispositif de biopsie selon la revendication 22 ou 29, **caractérisé en ce que** l'unité de commande déplace la première unité d'entraînement selon une succession périodique brève de mouvements de rotation respectivement opposés pour exécuter la fonction de prélèvement d'échantillon pendant l'opération de coupe, de telle manière que l'aiguille creuse extérieure (3) et l'aiguille de biopsie creuse (2) exécutent des mouvements avant et arrière le long de leur axe d'aguille longitudinal.

31. Dispositif de biopsie selon la revendication 29, **caractérisé en ce que** l'unité de commande prévoit un délai de sécurité, par lequel au moins la fonction de la touche de serrage (90) ainsi que la fonction d'éjection d'échantillon est retardée à la commande par comparaison avec le comportement de commande des autres touches de commande.

32. Dispositif de biopsie selon la revendication 1, **caractérisé en ce que** le boîtier présente la forme d'un parallélépipède, avec un couvercle de boîtier rabattable (10) articulé sur le boîtier ainsi qu'avec une première et une deuxième faces frontales (6, 7), dans lequel la première face frontale (6) présente au moins une ouverture, à travers laquelle les régions d'aiguille distales de l'aiguille creuse extérieure (3) et de l'aiguille de biopsie creuse (2) sortent à l'extérieur du boîtier pour le prélèvement de tissu, et la deuxième face d'extrémité (7) prévoit au moins deux évidements (15, 16), à travers lesquels ladite au moins une conduite de raccordement (4) est menée.

33. Dispositif de biopsie selon la revendication 32, **caractérisé en ce qu'**il est prévu, dans l'évidement (16) de la deuxième face d'extrémité (7), un micro-interrupteur (19) qui peut être actionné au moyen de la source de pression (5) lorsque le boîtier est fermé, ce qui permet de libérer une alimentation en énergie pour les unités d'entraînement (21, 58).

34. Module d'aiguille de biopsie à mettre en oeuvre dans un dispositif de biopsie selon l'une quelconque des revendications 1 à 33, qui présente les composants suivants:
- une unité d'aiguille de biopsie intégrée dans un support d'aiguille de biopsie (37), qui comprend une aiguille creuse extérieure (3) avec une lame de coupe affûtée côté distal ainsi qu'une aiguille de biopsie creuse (2) logée à l'intérieur de l'aiguille creuse (3) avec une chambre de prélèvement d'échantillon de tissu (71) prévue dans sa région d'extrémité distale, dans lequel l'aiguille creuse extérieure glisse par rotation par rapport à l'aiguille creuse intérieure (2),
- une conduite de raccordement (4), ainsi que
- une source de pression (5), dans lequel la conduite de raccordement (4) raccorde de façon étanche au gaz l'aiguille de biopsie creuse (2) à la source de pression (5).

35. Module d'aiguille de biopsie selon la revendication 34, **caractérisé en ce que** l'unité d'aiguille de biopsie est fermement intégrée de façon détachable dans un support d'aiguille de biopsie (37), dans lequel l'aiguille de biopsie intérieure (2) est raccordée côté proximal à une pièce de raccordement (47), par laquelle la conduite de raccordement (4) peut être raccordée de façon étanche au gaz à l'aiguille de biopsie intérieure (2) et qui présente un contour de fixation (49), qui peut être inséré dans un contour opposé prévu à l'intérieur du support d'aiguille de biopsie (37), de telle manière que l'aiguille de biopsie intérieure (2) soit calée dans le support d'aiguille de biopsie au moins en direction longitudinale de l'aiguille, et l'aiguille creuse extérieure (3) prévoit, sur au moins une section partielle le long de son périmètre extérieur, un contour fileté (73), qui est en prise avec un contour fileté opposé (75) au moins fermement assemblé de façon détachable au support d'aiguille de biopsie (37) de telle manière que l'aiguille creuse extérieure (3) soit déplaçable en direction longitudinale par rapport à l'aiguille de biopsie intérieure (2).

36. Module d'aiguille de biopsie selon la revendication 34, **caractérisé en ce qu'**un moyen d'entraînement (74) est installé sur la périphérie extérieure de l'aiguille creuse extérieure (3), par lequel l'aiguille creuse extérieure (3) peut être mise en rotation autour de la direction longitudinale d'aguille, ce qui permet de déplacer l'aiguille creuse extérieure (3) en direction axiale par rapport au support d'aiguille de biopsie (37) et de ce fait par rapport à l'aiguille de biopsie creuse (2).

37. Module d'aiguille de biopsie selon la revendication 34, **caractérisé en ce que** la pièce de raccordement (47) prévoit un élément d'encliquetage (50), qui est en contact avec des faces actives du support d'aiguille de biopsie (37), de telle manière que l'aiguille de biopsie intérieure (2) puisse être calée par encliquetage dans des positions prédéterminables autour de sa direction longitudinale d'aiguille.

38. Module d'aiguille de biopsie selon la revendication 34, **caractérisé en ce qu'**il est prévu entre l'aiguille creuse extérieure (3) et l'aiguille de biopsie intérieure (2) un moyen augmentant le frottement entre les deux aiguilles, qui se présente sous la forme d'un élément d'étanchéité (76).

39. Module d'aiguille de biopsie selon la revendication 38, **caractérisé en ce que** la pièce de raccordement (47) prévoit un élément d'encliquetage (50), qui est en contact avec des faces actives du support d'aiguille de biopsie (37), de telle manière que l'aiguille de biopsie intérieure (2) puisse être encliquetée dans des positions prédéterminables autour de sa direction longitudinale d'aiguille, de telle manière qu'il existe entre les faces actives et l'élément d'encliquetage un jeu par lequel une rotation angulaire limitée de l'aiguille de biopsie creuse (2) autour de son axe longitudinal est possible dans les deux sens de rotation.

40. Module d'aiguille de biopsie selon la revendication 34, **caractérisé en ce que** le support d'aiguille de biopsie (37) présente une structure de couplage (77), qui peut être insérée dans une structure de couplage opposée (40) prévue sur un coulisseau de serrage (28).

41. Module d'aiguille de biopsie selon la revendication 34, **caractérisé en ce que** la source de pression (5) présente une unité à piston-cylindre (69), par laquelle on peut produire un niveau de pression régnant, en dépression ou en surpression, à l'intérieur de l'unité de cylindre, en fonction du mouvement du piston.

42. Module d'aiguille de biopsie selon la revendication 41, **caractérisé en ce que** l'unité de cylindre (52) est réalisée sous la forme d'un corps de seringue et présente un fond de cylindre (51) avec un embout de raccordement (63) pour un raccordement étanche au gaz de la conduite de raccordement (4) ainsi qu'une ouverture de cylindre opposée au fond de cylindre (51), **en ce que** l'unité de piston prévoit une broche filetée (53), sur une extrémité de laquelle le piston (54) est monté et dont l'autre extrémité traverse un écrou de broche filetée (48) prévu sur l'ouverture de cylindre de l'unité de cylindre (52), qui présente un contour périphérique en forme de roue dentée (55), qui peut être mis en prise avec une unité d'entraînement, et **en ce que**, lors de la rotation de l'écrou de broche filetée (48), la broche filetée (53) avec le piston (54) est déplaçable en direction axiale par rapport à l'unité de cylindre (52).

43. Module d'aiguille de biopsie selon la revendication 42, **caractérisé en ce que** l'unité de cylindre (52) prévoit, dans la région de l'ouverture de cylindre à l'intérieur de la paroi du cylindre au moins une ouverture de ventilation (67), de telle manière que, dans une position du piston proche de l'ouverture de cylindre, la chambre de cylindre formée entre le piston (54) et le fond de cylindre (51) puisse être purgée.

44. Module d'aiguille de biopsie selon la revendication 41, **caractérisé en ce que** l'unité à piston-cylindre (69) présente un axe longitudinal de cylindre, **en ce que** l'unité à piston-cylindre (69) est disposée à côté de l'unité d'aiguille de biopsie, de telle manière que l'axe longitudinal d'aiguille de l'unité d'aiguille de biopsie et l'axe longitudinal de cylindre soient co-parallèles, et **en ce que** la conduite de raccordement (4) présente un parcours de conduite en forme de U.

45. Module d'aiguille de biopsie selon la revendication 34, **caractérisé en ce que** la conduite de raccordement (4) est formée par un tuyau flexible, largement transparent optiquement, qui permet un mouvement relatif entre la source de pression (5) et une aiguille de biopsie creuse (2).

46. Module d'aiguille de biopsie selon la revendication 34, **caractérisé en ce que** le module d'aiguille de biopsie composé du support d'aiguille de biopsie (37), dans lequel l'unité d'aiguille de biopsie est intégrée, de la conduite de raccordement (4) ainsi que de la source de pression (5) peut être fermement inséré de façon détachable dans un accessoire d'insertion (109) dans un agencement fermement prédéterminable spatialement.

47. Module d'aiguille de biopsie selon la revendication 34 ou 35, **caractérisé en ce que** la chambre de prélèvement d'échantillon de tissu (71) est limitée axialement par deux arêtes latérales longitudinales, qui se présentent sous la forme d'arêtes de coupe (68).

48. Module d'aiguille de biopsie selon la revendication 47, **caractérisé en ce que** l'aiguille de biopsie creuse (2) est réalisée, au moins dans la région de la chambre de prélèvement d'échantillon de tissu (71), à la manière d'un cylindre creux droit, qui prévoit un évidement axial local, dont la profondeur d'évidement radiale est inférieure à la moitié du diamètre intérieur du cylindre creux et dont les arêtes latérales longitudinales limitant axialement l'évidement sur les deux côtés se présentent sous la forme d'arêtes de coupe, de telle manière que, dans la région des arêtes latérales longitudinales, le rayon intérieur décrivant le cylindre creux s'assimile en continu au rayon extérieur.

49. Module d'aiguille de biopsie selon la revendication 34, **caractérisé en ce que** l'aiguille de biopsie creuse (2) prévoit à l'extrémité proximale de la chambre de prélèvement d'échantillon de tissu (71) un étranglement réduisant la section transversale du canal creux formé par l'aiguille de biopsie, et qui laisse ouverte une ouverture de passage du canal creux dans la chambre de prélèvement d'échantillon de tissu (71) dans la région inférieure de la chambre de prélèvement d'échantillon de tissu (71).

50. Module d'aiguille de biopsie selon la revendication 49, **caractérisé en ce que** l'étranglement couvre environ 60-70 % de la section transversale du canal creux, et l'étranglement est réalisé à la manière d'un bouchon s'engageant dans le canal creux ou sous la forme d'un élément plat s'engageant dans la surface de section transversale du canal creux.

51. Module d'aiguille de biopsie selon la revendication 35, **caractérisé en ce qu'**il est prévu, côté distal du support d'aiguille de biopsie (37), un rouleau de guidage (81) glissant en appui sur la périphérie extérieure de l'aiguille creuse extérieure (3), qui présente une ouverture de passage dimensionnée en ajustement largement serré avec la périphérie extérieure de l'aiguille creuse extérieure (3), à travers laquelle l'aiguille creuse extérieure (3) passe, et qui peut être introduit dans une traversée (13) dans le couvercle (6) de l'extrémité distale du boîtier.
